(19) Europäisches Patentamt
European Patent Office
Office européen des brevets

(11) EP 4 741 395 A1

(12) EUROPEAN PATENT APPLICATION
published in accordance with Art. 153(4) EPC

(43) Date of publication:
**13.05.2026 Bulletin 2026/20**

(21) Application number: **24836369.9**

(22) Date of filing: **05.07.2024**

(51) International Patent Classification (IPC):
***C07D 487/04*** (2006.01) ***A61K 31/519*** (2006.01)
***A61K 47/55*** (2017.01) ***A61P 29/00*** (2006.01)
***A61P 25/00*** (2006.01) ***A61P 9/00*** (2006.01)
***A61P 35/00*** (2006.01) ***A61P 3/04*** (2006.01)
***A61P 39/06*** (2006.01)

(52) Cooperative Patent Classification (CPC):
**A61K 31/519; A61K 47/55; A61P 3/04; A61P 9/00; A61P 25/00; A61P 29/00; A61P 35/00; A61P 39/06; C07D 487/04**

(86) International application number:
**PCT/KR2024/009549**

(87) International publication number:
**WO 2025/009922 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.07.2023 KR 20230086949**
**04.07.2024 KR 20240088043**

(71) Applicant: **Omniamed Co. Ltd**
**Pohang-si, Gyeongsangbuk-do 37673 (KR)**

(72) Inventors:
- **KIM, Won Jong**
  **Pohang-si, Gyeongsangbuk-do 37673 (KR)**
- **YOON, Joo Byoung**
  **Hwaseong-si, Gyeonggi-do 18479 (KR)**
- **KWON, Bo Sung**
  **Suwon-si, Gyeonggi-do 16543 (KR)**
- **JU, Jae Eun**
  **Pohang-si, Gyeongsangbuk-do 37699 (KR)**
- **KANG, Byung Yoon**
  **Seoul 05575 (KR)**
- **LEE, Ji Youn**
  **Seoul 08790 (KR)**
- **JEON, So Yeon**
  **Pohang-si Gyeongsangbuk-do 37691 (KR)**

(74) Representative: **Vossius & Partner**
**Patentanwälte Rechtsanwälte mbB**
**Siebertstraße 3**
**81675 München (DE)**

# (54) NOVEL COMPOUND SENSITIVE TO NITROGEN MONOXIDE AND PHARMACEUTICAL COMPOSITION COMPRISING SAME

(57) The present disclosure relates to a compound in which a nitrogen monoxide scavenger is conjugated to a low-molecular-weight compound having pharmaceutical activity, and a pharmaceutical composition sensitive to nitrogen monoxide including the same. The compound is designed such that a nitrogen monoxide scavenger is separated while removing nitrogen monoxide only in a diseased area such as an inflammatory area with high concentration of nitrogen monoxide, so that a low-molecular-weight compound in an inactivated state exhibits pharmaceutical activity. The compound is activated only in a diseased area, thereby minimizing side effects of existing medicines and simultaneously securing the effect of pharmaceutical activity of the low-molecular-weight compound and the effect of removing nitrogen monoxide, and can be used in preventing or treating diseases associated with nitrogen monoxide generation.

[FIG. 1]

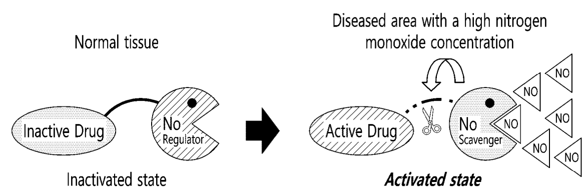


EP 4 741 395 A1

## Description

### Technical Field

This application claims priority to and the benefit of Korean Patent Application No. 10-2023-0086949 filed in the Korean Intellectual Property Office on July 5, 2023, the entire contents of which are incorporated herein by reference.

The present disclosure relates to a pharmaceutical composition sensitive to nitrogen monoxide. The pharmaceutical composition includes a nitrogen monoxide scavenger moiety that senses and removes nitrogen monoxide and a low-molecular-weight compound having separate pharmaceutical activity, and, whereas activity of the low-molecular-weight compound having pharmaceutical activity is minimized in a normal tissue with low nitrogen monoxide concentration, the nitrogen monoxide scavenger is separated from the low-molecular-weight compound as it removes nitrogen monoxide only in the inflammatory disease-affected area with a high nitrogen monoxide concentration, thereby allowing the low-molecular-weight compound to exhibit pharmaceutical activity locally and selectively only in the target area and thus minimizing side effects and maximizing efficacy. Accordingly, the pharmaceutical composition may be useful in preventing or treating all diseases associated with nitrogen monoxide.

### Background Art

Nitrogen monoxide (nitric oxide) is a highly reactive radical molecule having a short half-life of less than about 5 seconds, and is associated with various diseases such as inflammatory diseases, cancer growth and metastasis by acting as a major mediator of cell signaling.

Specifically, inflammatory-related diseases include rheumatoid arthritis, osteoarthritis, asthma, ulcerative colitis and Crohn's disease, inflammatory diseases caused by bacterial infection, inflammatory diseases caused by viral infection, inflammatory diseases caused by parasitic infection, septic shock and the like, and as neurological disorders, Alzheimer's disease, amyotrophic lateral sclerosis, Huntington's disease, Parkinson's disease and stroke are associated with nitrogen monoxide. In addition, cardiovascular diseases such as hypertension and heart failure, and diseases such as cancer, obesity and hepatic encephalopathy are also known to be associated with nitrogen monoxide.

## DISCLOSURE

### Technical Problem

The present disclosure is directed to providing a pharmaceutical composition for preventing or treating all diseases associated with nitrogen monoxide in which a pharmaceutical composition sensitive to nitrogen monoxide removes nitrogen monoxide locally and selectively only in a diseased area with a high nitrogen monoxide concentration and releases a low-molecular-weight compound having pharmaceutical activity, thereby minimizing side effects and toxicity of the low-molecular-weight compound, and maximizing its efficacy.

However, problems to be resolved by the present disclosure are not limited to the above-mentioned problem, and other problems not mentioned will be clearly understood by those skilled in the art from the following description.

### Technical Solution

All combinations of various elements disclosed in the present disclosure fall within the scope of the present disclosure. In addition, the scope of the present disclosure should not be construed as being limited by the following detailed description.

In view of the above, the present disclosure provides a pharmaceutical composition for preventing or treating all diseases associated with nitrogen monoxide, the pharmaceutical composition comprising a low-molecular-weight compound conjugated to a nitrogen monoxide scavenger as an active ingredient.

The compound according to the present disclosure is a material in which a nitrogen monoxide scavenger and a pharmaceutically active low-molecular-weight compound are conjugated, and an amine group or a hydroxyl group present in the low-molecular-weight compound and the nitrogen monoxide scavenger may be conjugated using an organic synthesis reaction.

One embodiment of the present disclosure provides a compound represented by the following Chemical Formula 1, or a solvate, a hydrate or a stereoisomer thereof, or pharmaceutically acceptable salts thereof:

[Chemical Formula 1]

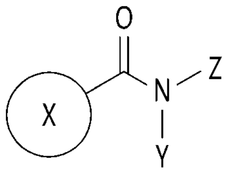

in Chemical Formula 1, X is a low-molecular-weight compound including an amine group or a hydroxyl group in the molecular structure of the compound and having pharmaceutical activity, and examples thereof may include kinase inhibitors, particularly Janus kinase inhibitors (JAK inhibitors), and in addition thereto, anticancer agents, anti-inflammatory agents, and the like.

The kinase inhibitor may include netarsudil (ROCK1/2, therapeutic agent for glaucoma), fostamatinib (Syk, therapeutic agent for thrombocytopenia), belumosudil (ROCK2, therapeutic agent for graft-versus-host disease), and the like.

The Janus kinase inhibitor may be selected from the group consisting of tofacitinib, upadacitinib, baricitinib, filgotinib, abrocitinib, delgocitinib, oclacitinib, peficitinib, ruxolitinib, and the like.

The anticancer agent may be selected from the group consisting of doxorubicin, cyclophosphamide, cisplatin, oxaliplatin, 5-fluorouracil (5-Fu), gemcitabine, paclitaxel, docetaxel, irinotecan, monomethyl auristatin E (MMAE), crizotinib, osimertinib, sorafenib, ibrutinib, ruxolitinib, vemurafenib, ceritinib, alectinib, brigatinib, lorlatinib, capmatinib, gefitinib, erlotinib, lapatinib, icotinib, afatinib, neratinib, dacomitinib, almonertinib, tucatinib, midostaurin, gilteritinib, quizartinib, pexidartinib, sunitinib, pazopanib, vandetanib, axitinib, cabozantinib, regorafenib, apatinib, lenvatinib, tivozanib, fruquintinib, nintedanib, anlotinib, erdafitinib, pemigatinib, avapritinib, ripretinib, pralsetinib, larotrectinib, entrectinib, imatinib, dasatinib, nilotinib, bosutinib, radotinib, ponatinib, acalabrutinib, zanubrutinib, fedratinib, dabrafenib, encorafenib, trametinib, cobimetinib, binimetinib, selumetinib, palbociclib, ribociclib, abemaciclib, idelalisib, copanlisib, duvelisib, alpelisib, tazemetostat, vorinostat belinostat, tucidinostat, panobinostat, enasidenib, ivosidenib, venetoclax, vismodegib, sonidegib, glasdegib, bortezomib, carfilzomib, ixazomib, olaparib, rucaparib, niraparib, talazoparib, umbralisib, trilaciclib, infigratinib, mobocertinib, asciminib, futibatinib, pacritinib, everolimus, and the like.

The anti-inflammatory agent may be selected from the group consisting of dexamethasone, methotrexate, cyclosporine, acetaminophen, etodolac, piroxicam, aceclofenac, and the like.

In Chemical Formula 1, Y may be selected from hydrogen, a $C_1$-$C_6$ alkyl group, a $C_1$-$C_6$ alkoxy group, a hydroxyl group, a nitro group, an amino group, halogen, a thiol group, a cyano group and

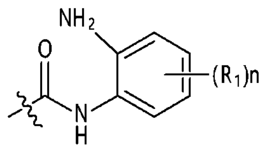

.

n is an integer of 0 to 4.

$R_1$ may be selected from a $C_1$-$C_6$ alkyl group, a $C_1$-$C_6$ alkoxy group, a hydroxyl group, a nitro group, an amino group, halogen, a thiol group, a cyano group, a $C_3$-$C_{14}$ aryl group, a $C_3$-$C_{14}$ heteroaryl group, $-NR_{11}R_{12}$, $-NR_{12}C(=O)R_{12}$, $-C(=O)R_{11}$ and $-C(=O)OR_{11}$, and when there are two or more $R_1$s, these are the same as or different from each other, and two adjacent $R_1$s may optionally bond to form a fused ring. Herein, the aryl group or the heteroaryl group may be each independently unsubstituted or substituted with at least one substituent selected from the group consisting of halogen, a hydroxyl group, a thiol group, a cyano group, a $C_1$-$C_6$ alkyl group and a $C_1$-$C_6$ alkoxy group, and $R_{11}$ and $R_{12}$ may be each independently selected from hydrogen, halogen, $CF_3$, a $C_1$-$C_6$ alkyl group, a $C_3$-$C_{14}$ aryl group and a $C_3$-$C_{14}$ heteroaryl group.

In Chemical Formula 1, Z may be selected from

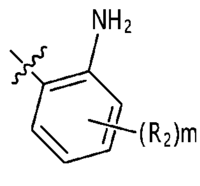

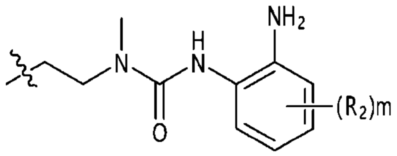
,
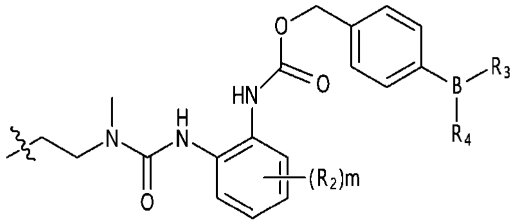
,

and

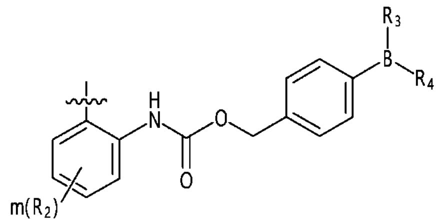
.

In Chemical Formula 1, m is an integer of 0 to 4.

$R_2$ may be selected from a $C_1$-$C_6$ alkyl group, a $C_1$-$C_6$ alkoxy group, a hydroxyl group, a nitro group, an amino group, halogen, a thiol group, a cyano group, a $C_3$-$C_{14}$ aryl group, a $C_3$-$C_{14}$ heteroaryl group, $-NR_{21}R_{22}$, $-NR_{22}C(=O)R_{22}$, $-C(=O)R_{21}$ and $-C(=O)OR_{21}$, and when there are two or more $R_2$s, these are the same as or different from each other, and two adjacent $R_2$s may optionally bond to form a fused ring. Herein, the aryl group or the heteroaryl group may be each independently unsubstituted or substituted with at least one substituent selected from the group consisting of halogen, a hydroxyl group, a thiol group, a cyano group, a $C_1$-$C_6$ alkyl group and a $C_1$-$C_6$ alkoxy group, and $R_{21}$ and $R_{22}$ may be each independently selected from hydrogen, halogen, $CF_3$, a $C_1$-$C_6$ alkyl group, a $C_3$-$C_{14}$ aryl group and a $C_3$-$C_{14}$ heteroaryl group.

$R_2$ may have the same definition as $R_1$. $R_{21}$ and $R_{22}$ may respectively have the same definitions as $R_{11}$ and $R_{12}$.

$R_3$ and $R_4$ may be each independently selected from hydrogen, halogen, a hydroxyl group, a thiol group, a cyano group, a $C_1$-$C_6$ alkyl group and a $C_1$-$C_6$ alkoxy group, or $R_3$ and $R_4$ may bond to form a fused ring.

Another embodiment of the present disclosure provides a compound represented by the following Chemical Formula 2, or a solvate, a hydrate or a stereoisomer thereof, or pharmaceutically acceptable salts thereof:

[Chemical Formula 2]

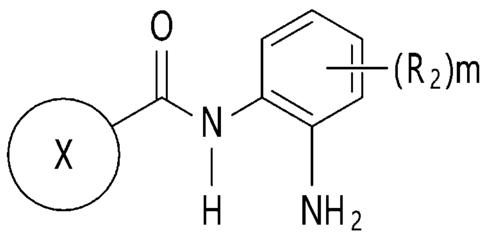

in Chemical Formula 2, $R_2$ and m have the same definitions as in Chemical Formula 1.

In the compound represented by Chemical Formula 2, X may be tofacitinib, and the compound may include compounds described in the following Table 1, or solvates, hydrates or stereoisomers thereof, or pharmaceutically acceptable salts thereof.

[Table 1]

| Compound (1) | Compound (2) |
|---|---|

(continued)

| | |
|---|---|
| 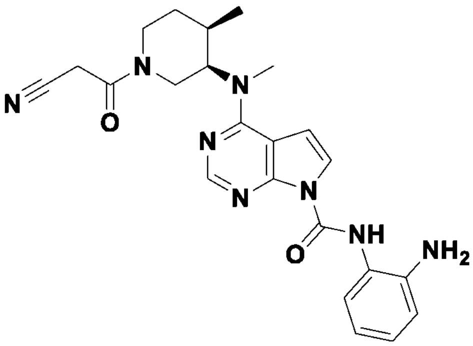 | 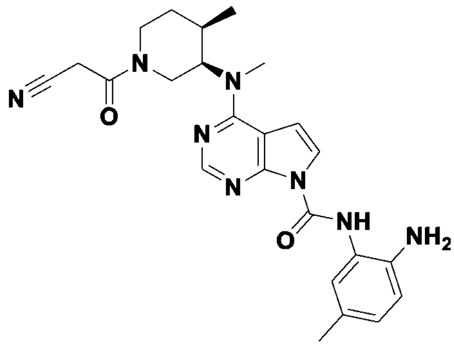 |
| Compound (3) | Compound (4) |
| 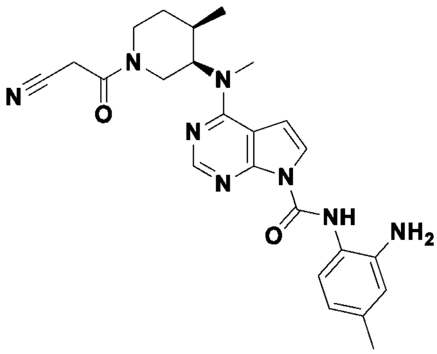 | 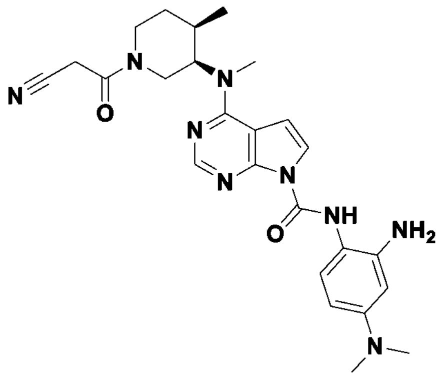 |
| Compound (5) | Compound (6) |
| 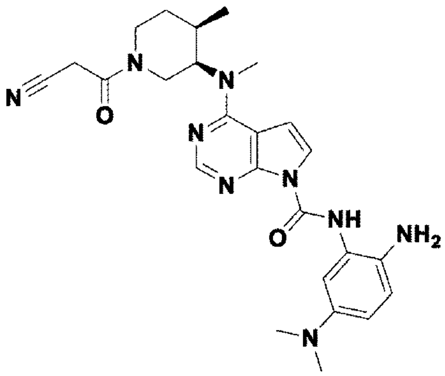 | 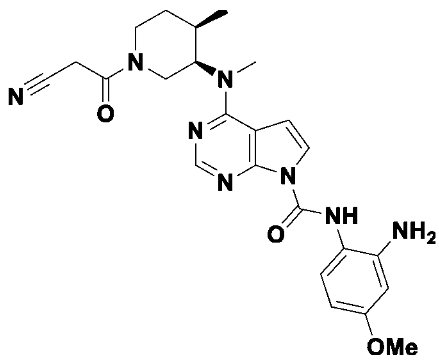 |
| Compound (7) | Compound (8) |
| 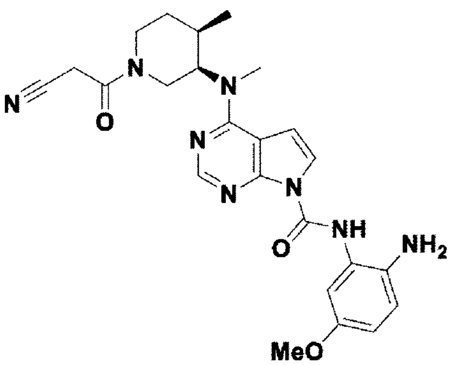 | 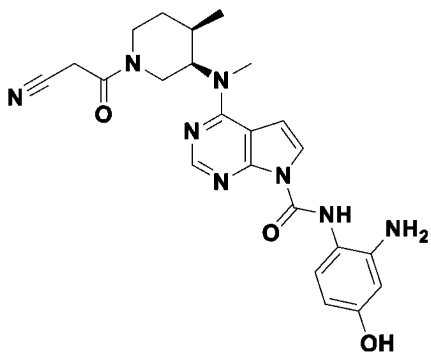 |
| Compound (9) | Compound (10) |

(continued)

| | |
|---|---|
| 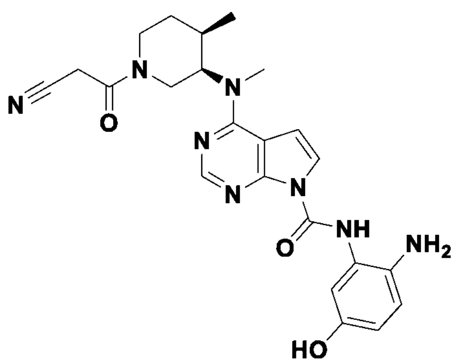 | 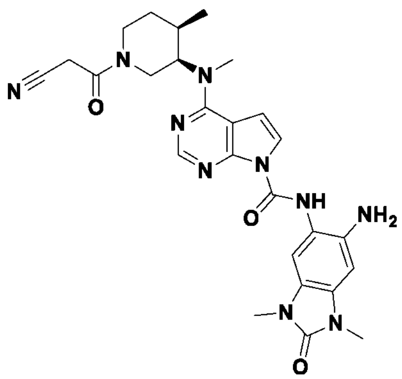 |

In the compound represented by Chemical Formula 2, X may be upadacitinib, and the compound may include compounds described in the following Table 2, or solvates, hydrates or stereoisomers thereof, or pharmaceutically acceptable salts thereof.

[Table 2]

| Compound (29) | Compound (30) |
|---|---|
| 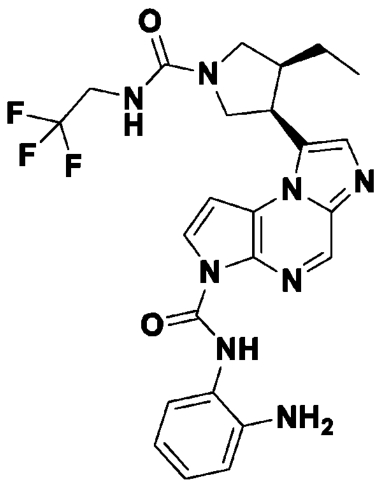 | 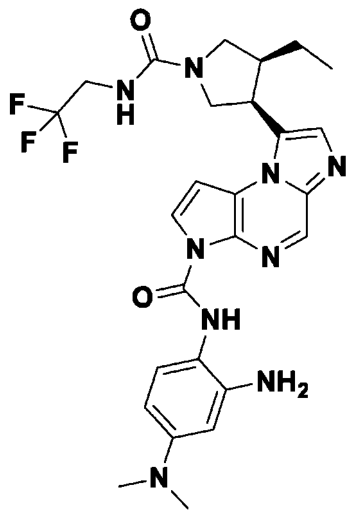 |
| Compound (31) | |

(continued)

| | |
|---|---|
| 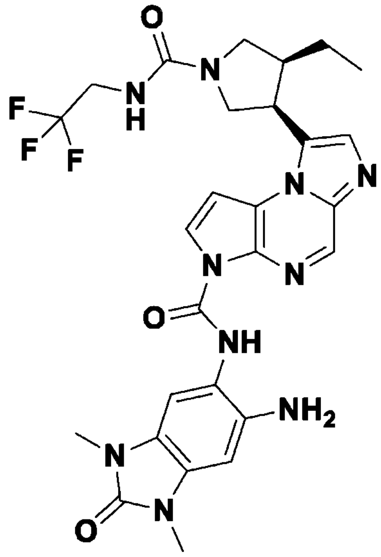 | |

In the compound represented by Chemical Formula 2, X may be dexamethasone, and the compound may include compounds described in the following Table 3, or solvates, hydrates or stereoisomers thereof, or pharmaceutically acceptable salts thereof.

[Table 3]

| Compound (33) | Compound (34) |
|---|---|
| 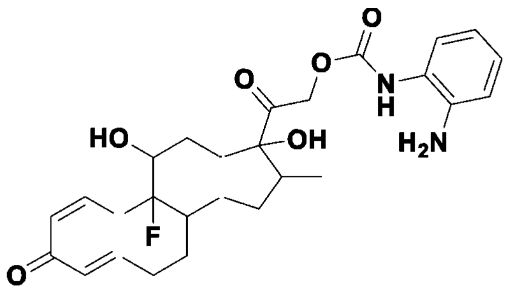 | 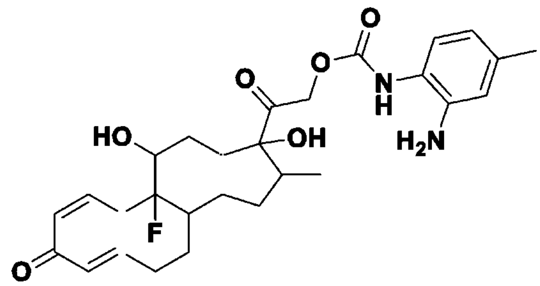 |

In the compound represented by Chemical Formula 2, X may be cyclosporine, and the compound may include a compound described in the following Table 4, or a solvate, a hydrate or a stereoisomer thereof, or pharmaceutically acceptable salts thereof.

[Table 4]

| Compound (35) |
|---|

(continued)

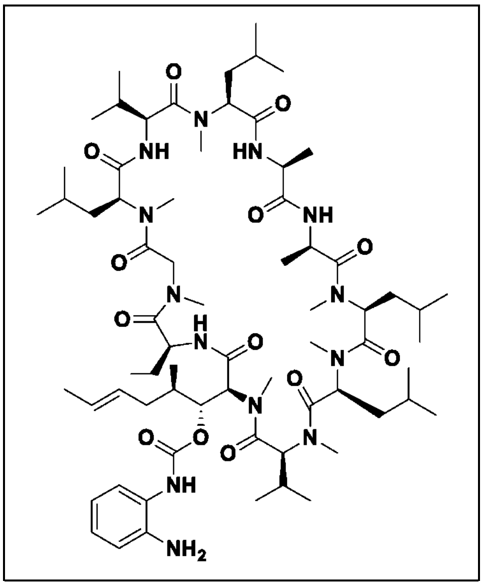

Another embodiment of the present disclosure provides a compound represented by the following Chemical Formula 3, or a solvate, a hydrate or a stereoisomer thereof, or pharmaceutically acceptable salts thereof:

[Chemical Formula 3]

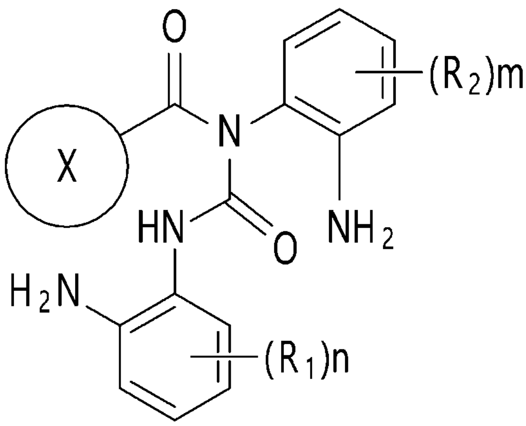

in Chemical Formula 3, $R_1$, $R_2$, n and m have the same definitions as in Chemical Formula 1.

In the compound represented by Chemical Formula 3, X may be tofacitinib, and the compound may include compounds described in the following Table 5, or solvates, hydrates or stereoisomers thereof, or pharmaceutically acceptable salts thereof.

[Table 5]

| Compound (11) | Compound (12) |
| --- | --- |

(continued)

| 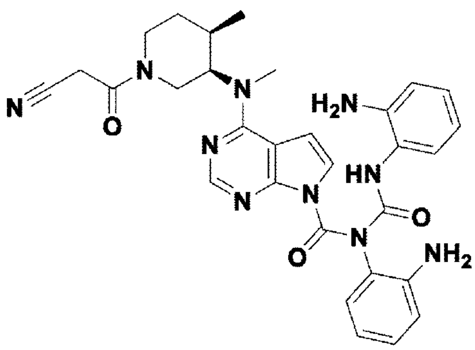 | 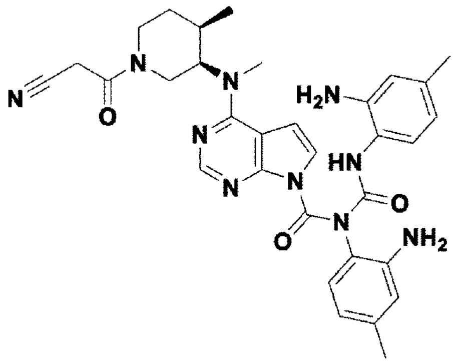 |
|---|---|
| Compound (13) | |
| 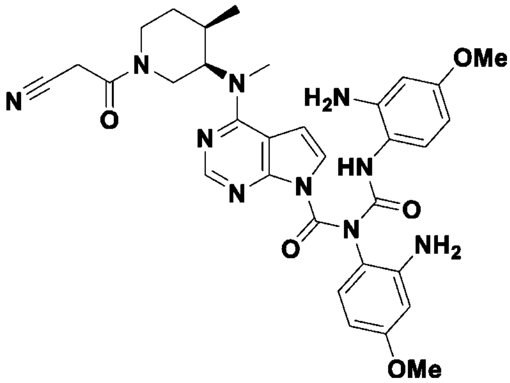 | |

Another embodiment of the present disclosure provides a compound represented by the following Chemical Formula 4, or a solvate, a hydrate or a stereoisomer thereof, or pharmaceutically acceptable salts thereof:

[Chemical Formula 4]

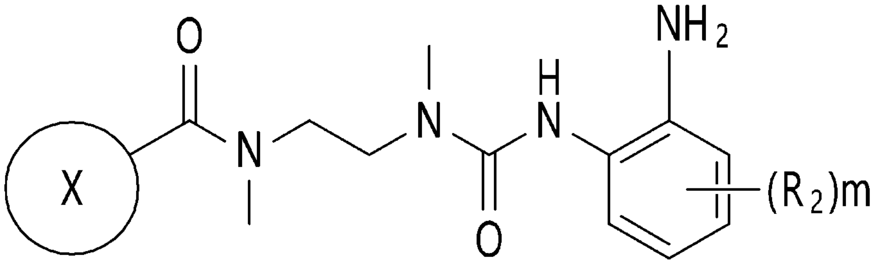

in Chemical Formula 4, $R_2$ and m have the same definitions as in Chemical Formula 1.

In the compound represented by Chemical Formula 4, X may be tofacitinib, and the compound may include compounds described in the following Table 6, or solvates, hydrates or stereoisomers thereof, or pharmaceutically acceptable salts thereof.

[Table 6]

| Compound (14) | Compound (15) |
|---|---|

(continued)

| | |
|---|---|
| 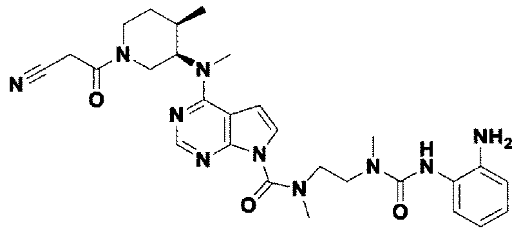 | 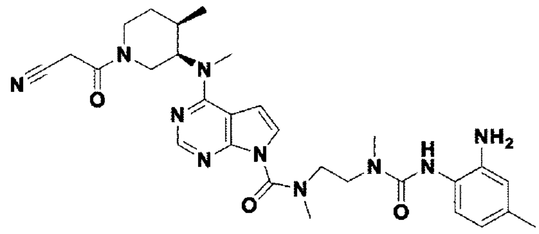 |
| Compound (16) | Compound (17) |
| 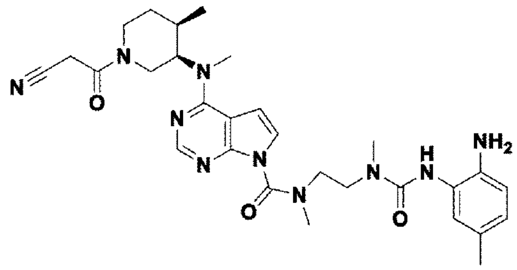 | 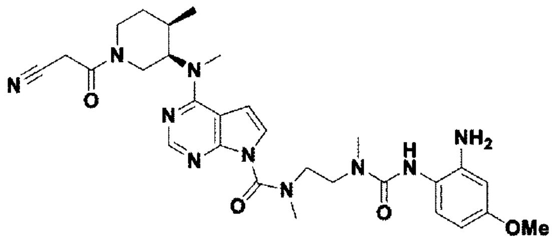 |
| Compound (18) | Compound (19) |
| 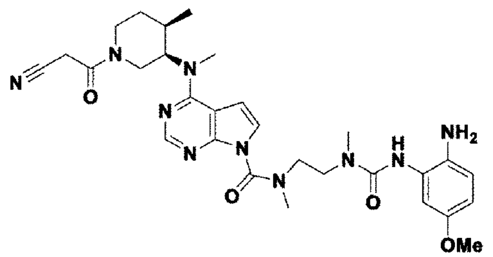 | 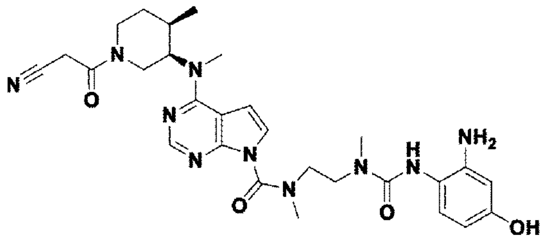 |
| Compound (20) | Compound (21) |
| 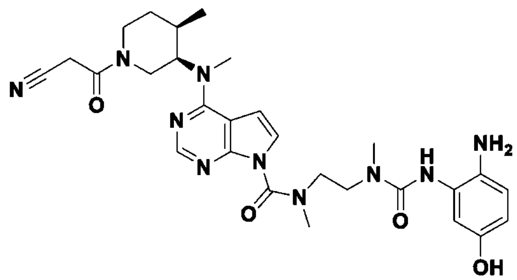 | 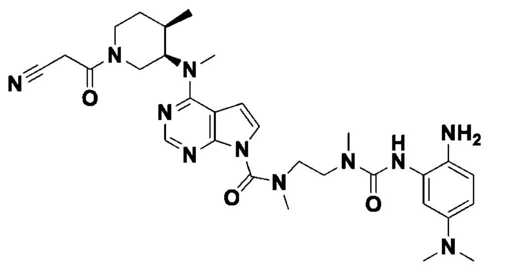 |
| Compound (22) | |
| 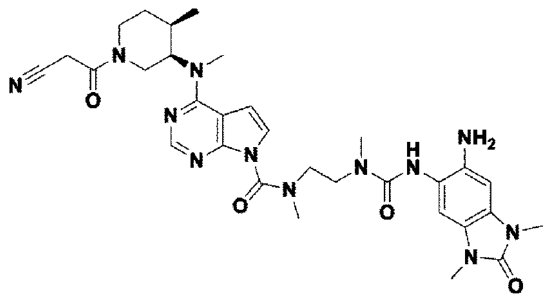 | |

Another embodiment of the present disclosure provides a compound represented by the following Chemical Formula 5, or a solvate, a hydrate or a stereoisomer thereof, or pharmaceutically acceptable salts thereof:

[Chemical Formula 5]

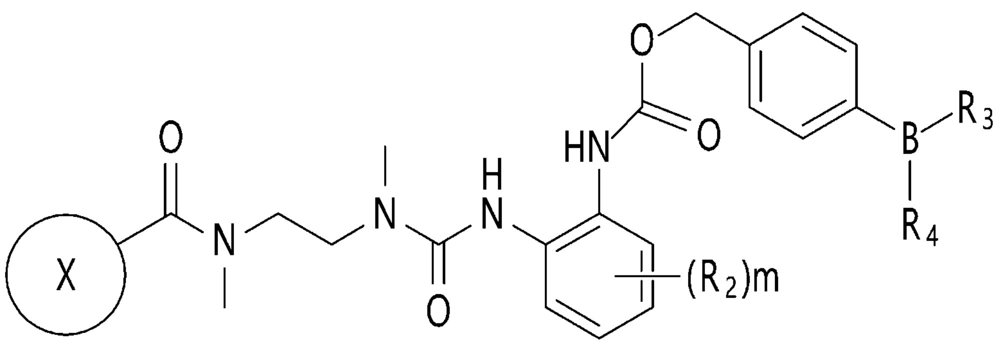

in Chemical Formula 5, $R_2$, $R_3$, $R_4$ and m have the same definitions as in Chemical Formula 1.

In the compound represented by Chemical Formula 5, X may be tofacitinib, and the compound may include compounds described in the following Table 7, or solvates, hydrates or stereoisomers thereof, or pharmaceutically acceptable salts thereof.

[Table 7]

| Compound (23) | Compound (24) |
|---|---|
| 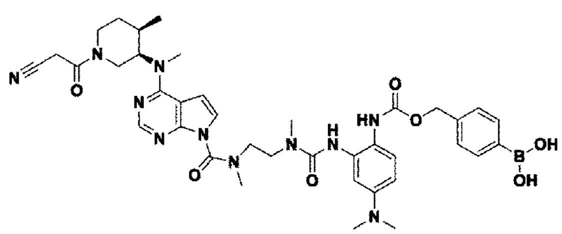 | 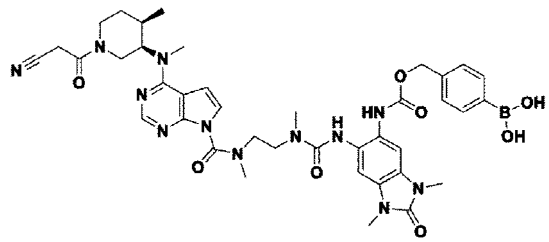 |

Another embodiment of the present disclosure provides a compound represented by the following Chemical Formula 6, or a solvate, a hydrate or a stereoisomer thereof, or pharmaceutically acceptable salts thereof:

[Chemical Formula 6]

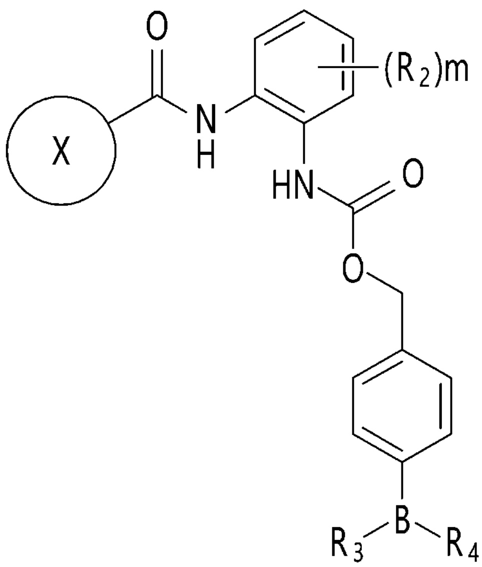

in Chemical Formula 6, $R_2$, $R_3$, $R_4$ and m have the same definitions as in Chemical Formula 1.

In the compound represented by Chemical Formula 6, X may be tofacitinib, and the compound may include compounds described in the following Table 8, or solvates, hydrates or stereoisomers thereof, or pharmaceutically acceptable salts thereof.

[Table 8]

| Compound (25) | Compound (26) |
|---|---|
| 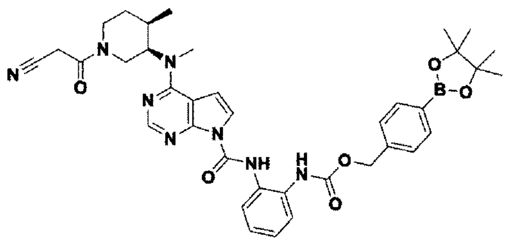 | 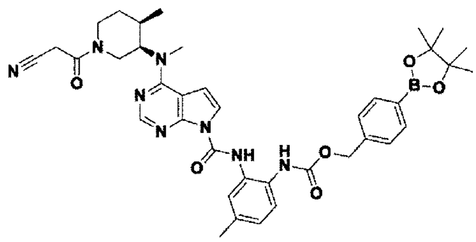 |
| Compound (27) | Compound (28) |
| 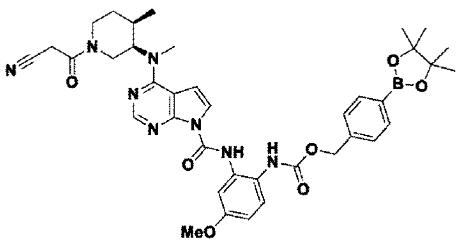 | 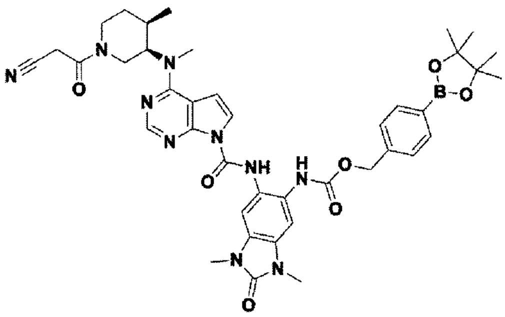 |

In the compound represented by Chemical Formula 6, X may be upadacitinib, and the compound may include a compound described in the following Table 9, or a solvate, a hydrate or a stereoisomer thereof, or pharmaceutically acceptable salts thereof.

[Table 9]

| Compound (32) |
|---|
| 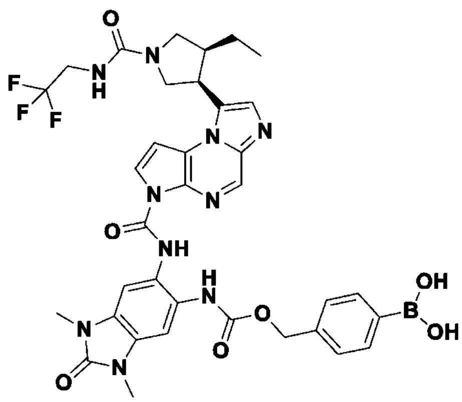 |

Another embodiment of the present disclosure provides a pharmaceutical composition for preventing and treating diseases associated with nitrogen monoxide generation, the pharmaceutical composition including the compound represented by Chemical Formula 1, or a solvate, a hydrate or a stereoisomer of the compound, or pharmaceutically acceptable salts thereof. Specifically, the pharmaceutical composition for preventing and treating diseases associated with nitrogen monoxide generation may include at least one of the compounds represented by Chemical Formulae 1 to 6, or a solvate, a hydrate or a stereoisomer thereof, or pharmaceutically acceptable salts thereof. The pharmaceutical composition may be a pharmaceutical composition sensitive to nitrogen monoxide. The pharmaceutical composition sensitive to nitrogen monoxide may remove at least a portion of nitrogen monoxide from a diseased area associated with

nitrogen monoxide generation, and release a compound having pharmaceutical activity to the diseased area.

The disease associated with nitrogen monoxide generation may be selected from the group consisting of inflammatory diseases, neurological disorders, cardiovascular diseases, autoimmune diseases, allergic diseases, cancer, obesity, myelofibrosis and hepatic encephalopathy.

The inflammatory disease may be selected from the group consisting of inflammatory diseases caused by infection (inflammatory diseases caused by viral infection, inflammatory diseases caused by bacterial infection, sepsis and the like), degenerative inflammatory diseases (osteoarthritis), inflammatory diseases caused by injury, autoimmune diseases (rheumatoid arthritis, ulcerative colitis, Crohn's disease, systemic lupus erythematosus, psoriasis, multiple sclerosis, juvenile idiopathic arthritis), allergic diseases (asthma, rhinitis, atopic dermatitis), cancer (lung cancer, breast cancer, colorectal cancer, gastric cancer, liver cancer, brain cancer, pancreatic cancer, thyroid cancer, skin cancer, bone marrow cancer, lymphoma, uterine cancer, cervical cancer, ovarian cancer, renal cancer, melanoma and the like) and other diseases caused by abnormal hyperactivation of immune cells such as myelofibrosis, that are all inflammatory diseases caused by an increase in nitrogen monoxide concentration.

The neurological disorder may be selected from the group consisting of Alzheimer's disease, amyotrophic lateral sclerosis, Huntington's disease, Parkinson's disease and stroke.

The cardiovascular disease may be hypertension or heart failure.

The pharmaceutical composition may be administered orally or parenterally. When the pharmaceutical composition is administered parenterally, it may be administered intraarticularly, intravenously, subcutaneously, intramuscularly, intraperitoneally, intranasally, intrapulmonarily or rectally.

The pharmaceutical composition may be administered orally (for example, form of tablet or capsule), parenterally (for example, form of injection solution or suspension), topically (for example, form of lotion, gel, ointment or cream), or in a nasal or suppository form as a pharmaceutical composition by any traditional route. The pharmaceutical composition including the compound of the present disclosure in a free form or in a pharmaceutically acceptable salt form, together with at least one pharmaceutically acceptable carrier or diluent, may be prepared using an existing method such as mixing, granulating or coating. For example, the composition for oral administration may be in the form of a tablet, granule or capsule containing an excipient, disintegrant, binder, lubricant or the like, active ingredients, and the like. In addition, the composition for injection may be in the form of a solution or suspension, may be sterilized, and may also include a preservative, a stabilizer, a buffer or the like.

In order to obtain target therapeutic effects using the pharmaceutical composition in actual treatment, the dose of the active ingredient, that is, the compound represented by Chemical Formula 1 of the present disclosure, or a solvate, a hydrate or a stereoisomer thereof, or pharmaceutically acceptable salts thereof may be determined depending on age, sex and body weight of a patient, the type of disease associated with nitrogen monoxide generation, the extent of treatment, and the like. For example, in the case of oral administration, the compound may be administered to an adult (having body weight of 60 kg) at a daily dose generally ranging from 0.001 mg/kg to 3,000 mg/kg, either as a single dose or in multiple divided doses, and the composition may be administered orally or parenterally once or multiple times every two days, weekly or monthly. It will be appreciated that the dose may be determined depending on the type of the low-molecular-weight compound represented by X. The dose for a particular individual or patient needs to be determined in view of various relevant factors such as body weight, age, sex, health status and diet of a patient, time and method of administration, and severity of disease, and may be properly adjusted by a skilled practitioner. The dose mentioned above is not intended to limit the scope of the present disclosure in any aspect.

The compound of the present disclosure may be used in combination with other therapeutic agents. The compound may be administered together with a therapeutic agent selected from the group consisting of cytotoxic drugs, radiotherapy and immunotherapy.

Another embodiment of the present disclosure provides a method for treating a disease associated with nitrogen monoxide generation, the method including administering a pharmaceutical composition including the compound represented by Chemical Formula 1, or a solvate, a hydrate or a stereoisomer of the compound, or pharmaceutically acceptable salts thereof to a subject.

**Advantageous Effects**

A pharmaceutical composition for preventing or treating diseases associated with nitrogen monoxide generation of the present disclosure selectively releases a drug only to a diseased area with a high nitrogen monoxide concentration in diseases associated with nitrogen monoxide, and therefore, can be used as a preventive or therapeutic agent for the diseases.

**Brief Description of Figures**

FIG. 1 is a schematic diagram illustrating an operating principle of a pharmaceutical composition sensitive to nitrogen monoxide according to one embodiment of the present disclosure.

FIG. 2 shows HPLC results of Compound (4) prepared in Example 4 in the presence or absence of nitrogen monoxide. FIG. 2A shows the HPLC result of Compound (4) in an environment without nitrogen monoxide. FIG. 2B shows the HPLC result of Compound (4) reacting in an environment with excess nitrogen monoxide. FIG. 2C shows an HPLC result for tofacitinib.

FIG. 3 shows NMR data of Compound (35) prepared in Example 35.

FIG. 4 is a graph showing efficacy evaluation of Compound (1) prepared in Example 1 on ulcerative colitis.

FIG. 5 is a graph showing efficacy evaluation of Compound (4) prepared in Example 4 on ulcerative colitis.

FIG. 6 is a graph showing efficacy evaluation of Compound (1) prepared in Example 1 on rheumatoid arthritis.

FIG. 7 is a graph showing efficacy evaluation of Compound (11) prepared in Example 11 on rheumatoid arthritis.

**Mode For Invention**

The present disclosure may be more readily understood with reference to the following detailed description of preferred embodiments and examples of the present disclosure included herein. It should be understood that terms used herein are for the purpose of describing specific embodiments only and are not intended to limit the present disclosure. In addition, unless specifically defined herein, it should be understood that terms used herein are to be understood as having meanings commonly in the related art.

As used herein, singular forms include plural forms unless otherwise indicated. For example, a substituent includes one or more substituents.

In the present specification, the term "halo" or "halogen" refers to fluorine (F), chlorine (Cl), bromine (Br) or iodine (I) unless otherwise mentioned.

In the present specification, the term "alkyl" refers to, unless otherwise mentioned, a saturated monovalent aliphatic hydrocarbon radical, including linear and branched forms, having a specific number of carbon atoms. An alkyl group typically contains 1 to 20 carbon atoms ("$C_1$-$C_{20}$ alkyl"), preferably 1 to 12 carbon atoms ("$C_1$-$C_{12}$ alkyl"), and more preferably 1 to 8 carbon atoms ("$C_1$-$C_6$ alkyl"), 1 to 6 carbon atoms ("$C_1$-$C_6$ alkyl") or 1 to 4 carbon atoms ("$C_1$-$C_4$ alkyl"). Examples of the alkyl group include methyl, ethyl, n-propyl, isopropyl, n-butyl, iso-butyl, tert-butyl, n-pentyl, isopentyl, neopentyl, n-hexyl, n-heptyl, n-octyl, and the like.

In the present specification, the term "alkoxy" refers to, unless otherwise stated, a monovalent -O-alkyl group in which the alkyl moiety has a specific number of carbon atoms. An alkoxy group typically has 1 to 8 carbon atoms ("$C_1$-$C_8$ alkoxy"), 1 to 6 carbon atoms ("$C_1$-$C_6$ alkoxy") or 1 to 4 carbon atoms ("$C_1$-$C_4$ alkoxy"). For example, the $C_1$-$C_4$ alkoxy includes methoxy ($-OCH_3$), ethoxy ($-OCH_2CH_3$), isopropoxy ($-OCH(CH_3)_2$), tert-butyloxy ($-OC(CH_3)_3$), or the like. The alkoxy group is substituted or unsubstituted on the alkyl moiety by the same group described herein to be suitable for alkyl. Particularly, the alkoxy group may be optionally substituted with one or more halo atoms, particularly, one or more fluoro atoms, up to the total number of hydrogen atoms present on the alkyl moiety. Such a group is referred to as "haloalkoxy" having a specific number of carbon atoms and substituted with one or more halo substituents, and more specifically a "fluoroalkoxy" group when, for example, fluorinated. Such a group typically contains 1 to 6 carbon atoms, preferably 1 to 4 carbon atoms, often 1 or 2 carbon atoms, and 1, 2 or 3 halo atoms (that is, "$C_1$-$C_6$ haloalkoxy", "$C_1$-$C_4$ haloalkoxy" or "$C_1$-$C_2$ haloalkoxy"). More specifically, the fluorinated alkyl group may be typically referred to as a fluoroalkoxy group substituted with 1, 2 or 3 fluoro atoms, specifically, for example, a $C_1$-$C_6$, $C_1$-$C_4$ or $C_1$-$C_2$ fluoroalkoxy group. Accordingly, the $C_1$-$C_4$ fluoroalkoxy includes trifluoromethyloxy ($-OCF_3$), difluoromethyloxy ($-OCF_2H$), fluoromethyloxy ($-OCFH_2$), difluoroethyloxy ($-OCH_2CF_2H$) or the like.

In the present specification, the term "aryl" or "aromatic" refers to, unless otherwise stated, an optionally substituted or unsubstituted monocyclic or fused bicyclic or polycyclic ring system having well-known characteristics of aromaticity, and herein, one or more rings contain a completely conjugated pi-electron system. An aryl group typically has 3 to 20 carbon atoms ("$C_3$-$C_{20}$ aryl"), preferably 6 to 14 carbon atoms ("$C_6$-$C_{14}$ aryl") and more preferably 6 to 12 carbon atoms ("$C_6$-$C_{12}$ aryl") as a ring member. A fused aryl group may include an aryl ring (for example, phenyl ring) fused with another aryl or heteroaryl ring, or fused with a saturated or partially unsaturated carbocyclic or heterocyclic ring, and the point of attachment for the base molecule in such a fused ring system is an atom of the aromatic moiety in the ring system. For example, the aryl group includes phenyl, biphenyl, naphthyl, toluyl, anthracenyl, phenanthrenyl, indanyl, indenyl or tetrahydronaphthyl. The aryl group is substituted or unsubstituted as further described herein.

In the present specification, the term "heteroaryl" or "heteroaromatic" refers to, unless otherwise stated, a monocyclic or fused bicyclic or polycyclic ring system having well-known characteristics of aromaticity, including a specific number of cyclic atoms as a ring member in the aromatic ring and including one or more heteroatoms selected from B, N, O, S, P and Si. The inclusion of heteroatoms allows aromaticity in a 5-membered ring and a 6-membered ring. A heteroaryl group typically has 5 to 20 cyclic atoms ("5- to 20-membered heteroaryl"), preferably 5 to 14 cyclic atoms ("5- to 14-membered heteroaryl") and more preferably 5 to 12 cyclic atoms ("5- to 12-membered heteroaryl"). The heteroaryl ring is

attached to the base molecule through a cyclic atom of the heteroaromatic ring to main aromaticity. Accordingly, a 6-membered heteroaryl ring may be attached to the base molecule through a cyclic C atom, whereas a 5-membered heteroaryl ring may be attached to the base molecule through a cyclic C or N atom. The heteroaryl group may also be fused with another aryl or heteroaryl ring, or fused with saturated or partially unsaturated carbocyclic or heterocyclic ring, and the point of attachment for the base molecule in such a fused ring system is an atom of the heteroaromatic moiety in the ring system. For example, the unsubstituted heteroaryl group includes pyrrole, furan, thiophene, pyrazole, imidazole, isoxazole, oxazole, isothiazole, thiazole, triazole, oxadiazole, thiadiazole, tetrazole, pyridine, pyridazine, pyrimidine, pyrazine, benzofuran, benzothiophene, indole, benzimidazole, indazole, quinoline, isoquinoline, purine, triazine, naphthyridine or carbazole. In various embodiments, the 5- or 6-membered heteroaryl group is selected from the group consisting of pyrrolyl, furanyl, thiophenyl, pyrazolyl, imidazolyl, isoxazolyl, oxazolyl, isothiazolyl, thiazolyl, triazolyl, pyridinyl, pyrimidinyl, pyrazinyl and pyridazinyl rings. The heteroaryl group is substituted or unsubstituted as further described herein.

However, the present disclosure will be described in detail with reference to examples and experimental examples. However, the following examples and experimental examples are for illustrative purposes only, and the present disclosure is not limited thereto.

Example 1: Preparation of Compound (1) (N-(2-aminophenyl)-4-(((3R,4R)-1-(2-cyanoacetyl)-4-methylpiperidin-3-yl)(methyl)amino)-7H-pyrrolo[2,3-d]pyrimidine-7-carboxamide)

[Reaction Formula 1]

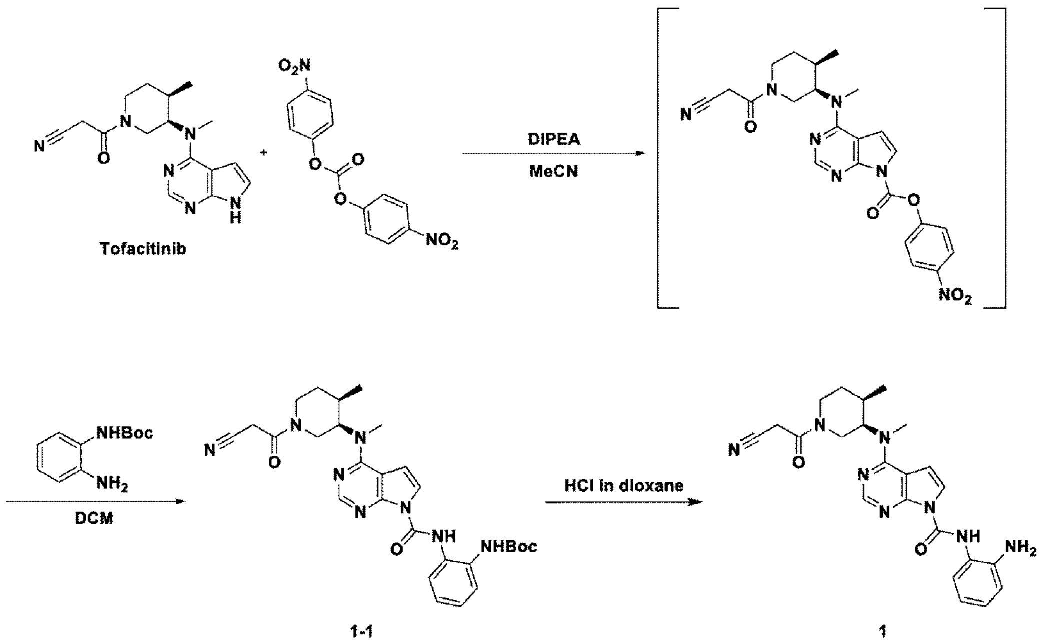


Step (1): Preparation of Compound (1-1)

300 mg of tofacitinib (0.96 mmol, 1 equiv) and 394 mg of bis(4-nitrophenyl)carbonate (1.30 mmol, 1.35 equiv) were introduced to 8 mL of acetonitrile, and the mixture solution was cooled to 0°C to 5°C. To the mixture solution, 229 μL of DIPEA (1.34 mmol, 1.4 equiv) was introduced, and then the mixture solution was stirred for 4 hours at room temperature. After cooling the mixture solution to 0°C to 5°C, 214 mg of tert-butyl (2-aminophenyl)carbamate (0.96 mmol, 1 equiv) dissolved in 6 mL of dichloromethane was added dropwise thereto, and the mixture solution was stirred for 16 hours at room temperature. After the reaction was finished, water and dichloromethane were introduced to the reaction solution for extraction. The organic layer was separated, then dried with magnesium sulfate, and filtered. The remaining filtrate was concentrated under reduced pressure. The concentrated solution was purified by silica gel column chromatography (ethyl acetate:hexane=1:1) to obtain Compound (1-1), a target compound. (Yield 19%)

Step (2): Preparation of Compound (1)

34 mg of Compound (1-1) (0.06 mmol, 1 equiv) was dissolved in 1 mL of dioxane, and then the mixture solution was cooled to 0°C to 5°C. To the mixture solution, 130 µL of hydrochloric acid (4 M dioxane solution, 0.44 mmol, 7.25 equiv) was introduced, and then the mixture solution was stirred for 16 hours at room temperature. After the reaction was finished, the reaction solution was cooled to 0°C to 5°C, and neutralized by adding a 5% aqueous $NaHCO_3$ solution dropwise thereto. Water and ethyl acetate were introduced to the mixture solution for extraction. The organic layer was separated, then dried with magnesium sulfate, and filtered. The remaining filtrate was concentrated under reduced pressure. The concentrated solution was purified by silica gel column chromatography (ethyl acetate:hexane=1:1) to obtain Compound (1), a target compound. (Yield 99%)
$^1$H-NMR (300 MHz, DMSO-$d_6$) δ 11.55 (s, 1H), 8.42 (d, 1H, J=3 Hz), 8.12 (d, 1H, J=9 Hz), 7.77 (d, 1H, J=3 Hz), 7.61 (d, 1H, J=6 Hz), 7.29-7.18 (m, 3H), 6.96-6.91 (m, 2H), 4.21-3.62 (m, 4H), 3.49-3.38 (m, 2H), 3.33 (s, 3H), 2.41 (m, 1H), 1.98-1.60 (m, 3H), 1.02 (d, 3H, J=6 Hz).

**Example 2: Preparation of Compound (2) (N-(2-amino-5-methylphenyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 2]

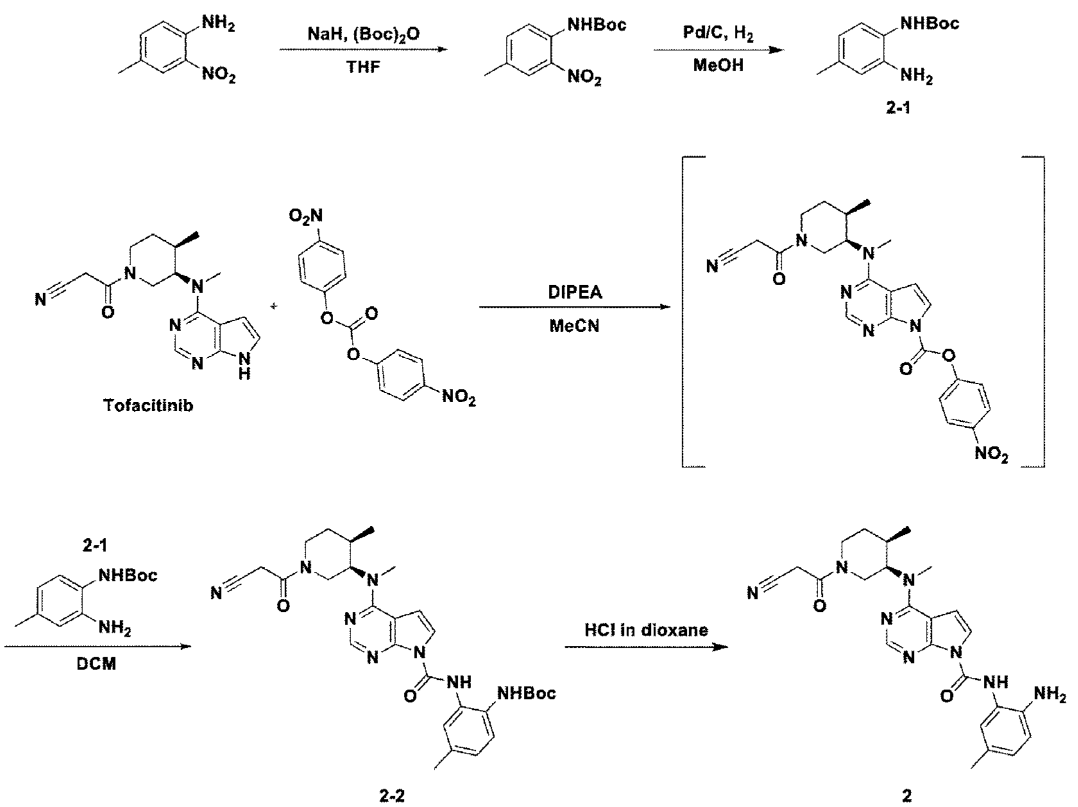

2-1
Tofacitinib
2-1
2-2
2

Step (1): Preparation of tert-butyl (4-methyl-2-nitrophenyl)carbamate

1 g of 4-methyl-2-nitroaniline (6.57 mmol, 1 equiv) was dissolved in 18 mL of anhydrous THF, and then the mixture solution was cooled to 0°C to 5°C. 578 mg of NaH (60% content) (14.46 mmol, 2.2 equiv) was introduced thereto, and then the mixture solution was stirred for 10 minutes. The mixture solution was warmed to room temperature, and stirred for 30 minutes. To the mixture solution, 1.58 g of $(Boc)_2O$ (7.23 mmol, 1.1 equiv) dissolved in 1.8 mL of anhydrous THF was introduced, and the mixture solution was stirred for 16 hours. After the reaction was finished, water and ethyl acetate were introduced to the reaction solution for extraction. The organic layer was separated, then dried with magnesium sulfate, and filtered. The remaining filtrate was concentrated under reduced pressure. The concentrated solution was purified by silica gel column chromatography (ethyl acetate:hexane=1:100) to obtain tert-butyl (4-methyl-2-nitrophenyl)carbamate, a

target compound. (Yield 92%)

Step (2): Preparation of Compound (2-1)

465 mg of tert-butyl (4-methyl-2-nitrophenyl)carbamate (1.84 mmol, 1 equiv) was dissolved in 32 mL of methanol. 47 mg of 10% Pd/C was introduced to the mixture solution at room temperature, and the mixture solution was stirred for 2 hours under hydrogen gas at atmospheric pressure. After the reaction was finished, the mixture solution was purified by a Celite filter, and washed with ethyl acetate. The filtrate was concentrated under reduced pressure to obtain Compound (2-1), a target compound. (Yield 99%)

Step (3): Preparation of Compound (2-2)

Compound (2-2), a target compound, was obtained by repeating the same procedure as in Step (1) of Example 1 using Compound (2-1) instead of tert-butyl (2-aminophenyl)carbamate. (Yield 74%)

Step (4): Preparation of Compound (2)

Compound (2), a target compound, was obtained by repeating the same procedure as in Step (2) of Example 1 using Compound (2-2) instead of Compound (1-1). (Yield 42%)
$^1$H-NMR (300 MHz, DMSO-$d_6$) δ 11.21 (s, 1H), 8.38 (d, 1H, J=3 Hz), 7.74 (d, 1H, J=3 Hz), 7.29 (s, 1H), 6.91-6.72 (m, 3H), 4.79 (s, 2H), 4.13-3.42 (m, 6H), 3.32 (s, 3H), 2.49 (m, 1H), 2.19 (s, 3H), 1.89-1.59 (m, 3H), 1.02 (d, 3H, J=6 Hz).

**Example 3: Preparation of Compound (3) (N-(2-amino-4-methylphenyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 3]

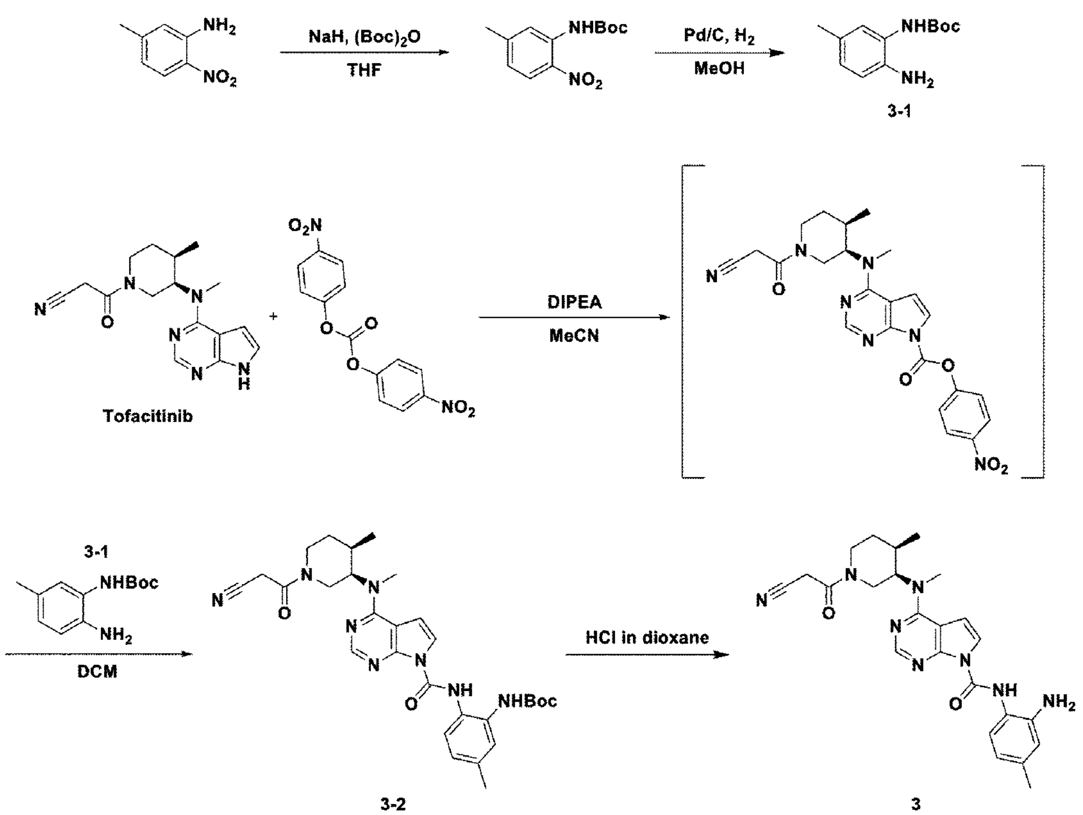


3-1

Tofacitinib

3-2

3

Step (1): Preparation of tert-butyl (5-methyl-2-nitrophenyl)carbamate

Tert-butyl (5-methyl-2-nitrophenyl)carbamate, a target compound, was obtained by repeating the same procedure as in Step (1) of Example 2 using 5-methyl-2-nitroaniline instead of 4-methyl-2-nitroaniline. (Yield 56%)

Step (2): Preparation of Compound (3-1)

Compound (3-1), a target compound, was obtained by repeating the same procedure as in Step (2) of Example 2 using tert-butyl (5-methyl-2-nitrophenyl)carbamate instead of tert-butyl (4-methyl-2-nitrophenyl)carbamate. (Yield 97%)

Step (3): Preparation of Compound (3-2)

Compound (3-2), a target compound, was obtained by repeating the same procedure as in Step (1) of Example 1 using Compound (3-1) instead of tert-butyl (2-aminophenyl)carbamate. (Yield 64%)

Step (4): Preparation of Compound (3)

Compound (3), a target compound, was obtained by repeating the same procedure as in Step (2) of Example 1 using Compound (3-2) instead of Compound (1). (Yield 67%)
$^1$H-NMR (300 MHz, DMSO-$d_6$) δ 11.09 (s, 1H), 8.38 (d, 1H, J=3 Hz), 7.74 (d, 1H, J=3 Hz), 7.28-7.25 (m, 1H), 6.89 (d, 1H, J=6 Hz), 6.64 (s, 1H), 6.46 (d, 1H, J=9 Hz), 4.94 (s, 2H), 4.13-3.42 (m, 6H), 3.32 (s, 3H), 2.41-2.39 (m, 1H), 2.20 (s, 3H), 1.86-1.59 (m, 3H), 1.01 (d, 3H, J=6 Hz)

**Example 4: Preparation of Compound (4) (N-(2-amino-4-(dimethylamino)phenyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 4]

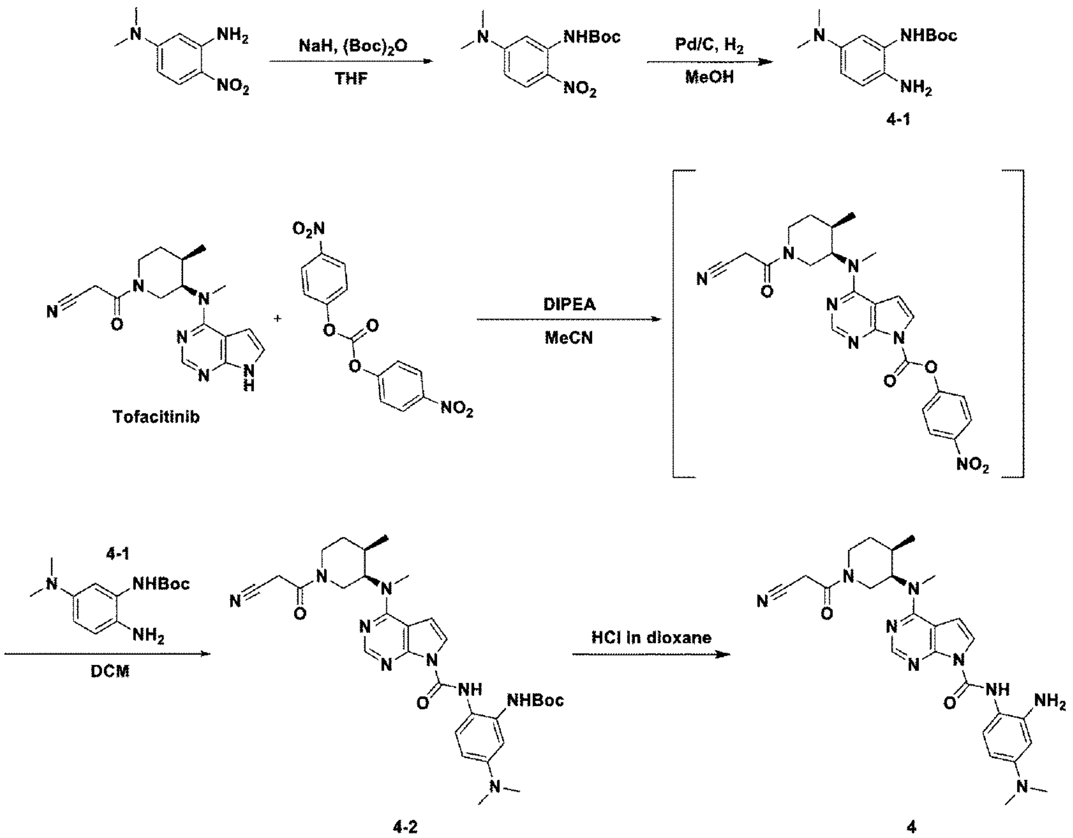

4-1
Tofacitinib
4-2
4

Step (1): Preparation of tert-butyl (5-(dimethylamino)-2-nitrophenyl)carbamate

Tert-butyl (5-(dimethylamino)-2-nitrophenyl) carbamate, a target compound, was obtained by repeating the same procedure as in Step (1) of Example 2 using N1,N1-dimethyl-4-nitrobenzene-1,3-diamine instead of 4-methyl-2-nitroaniline. (Yield 52%)

Step (2): Preparation of Compound (4-1)

Compound (4-1), a target compound, was obtained by repeating the same procedure as in Step (2) of Example 2 using tert-butyl (5-(dimethylamino)-2-nitrophenyl)carbamate instead of tert-butyl (4-methyl-2-nitrophenyl)carbamate. (Yield 91%)

Step (3): Preparation of Compound (4-2)

Compound (4-2), a target compound, was obtained by repeating the same procedure as in Step (1) of Example 1 using Compound (4-1) instead of tert-butyl (2-aminophenyl)carbamate. (Yield 59%)

Step (4): Preparation of Compound (4)

Compound (4), a target compound, was obtained by repeating the same procedure as in Step (2) of Example 1 using Compound (4-2) instead of Compound (1). (Yield 77%)
$^1$H-NMR (300 MHz, DMSO-$d_6$) δ 10.87 (s, 1H), 8.37 (d, 1H, J=3 Hz), 7.73 (d, 1H, J=3 Hz), 7.10 (d, 1H, J=9 Hz), 6.88 (d, 1H, J=3 Hz), 6.19 (d, 1H, J=3 Hz), 6.06 (dd, 1H, 12, 3 Hz), 4.84 (s, 2H), 4.20-3.41 (m, 6H), 3.32 (s, 3H), 2.84 (s, 6H), 2.41 (m, 1H), 2.20 (s, 3H), 1.86-1.59 (m, 3H), 1.02 (d, 3H, J=9 Hz).

**Example** 5: **Preparation of Compound (5) (N-(2-amino-5-(dimethylamino)phenyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 5]

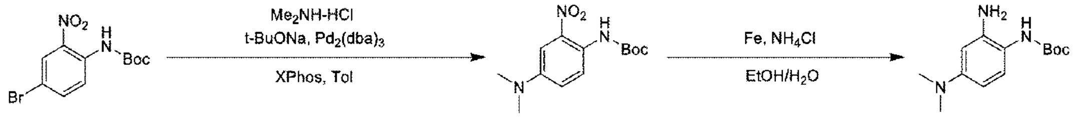


5-1 5-2 5-3

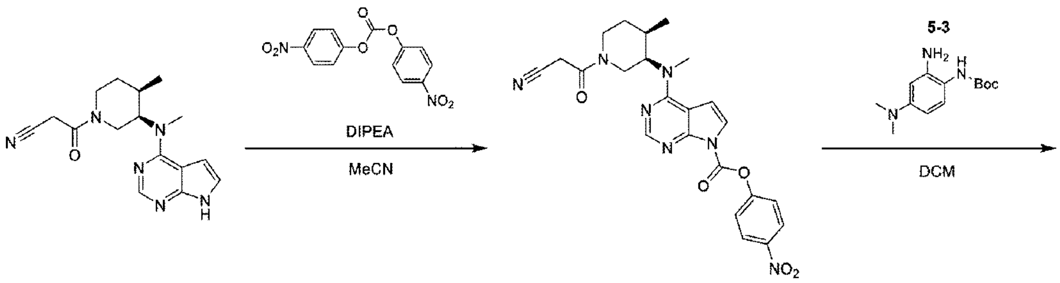


Tofacitinib

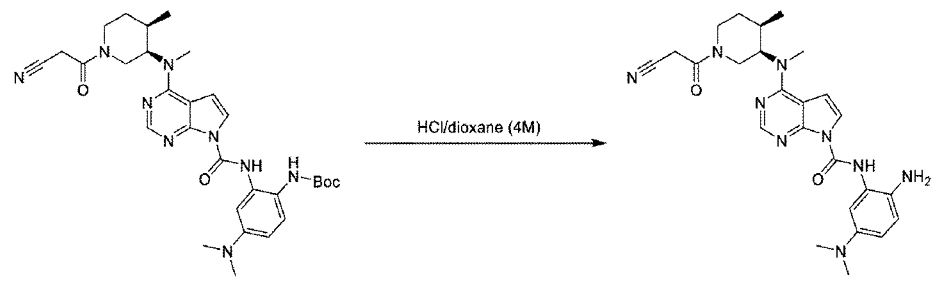


5-4 5

Step (1): Preparation of Compound (5-2)

To a solution in which 1 g of Compound (5-1) (3.15 mmol, 1 equiv) was dissolved in 10 mL of toluene, $Me_2NH$-HCl (1.29 g, 15.7 mmol, 5.00 equiv), XPhos (150 mg, 315 μmol, 0.1 equiv), t-BuONa (909 mg, 9.46 mmol, 3.00 equiv) and $Pd_2(dba)_3$ (144 mg, 157 μmol, 0.05 equiv) were introduced. The mixture solution was stirred for 12 hours at 90°C. After the reaction was finished, water and ethyl acetate were introduced to the reaction solution for extraction. The organic layer was separated, then dried with magnesium sulfate, and filtered. The remaining filtrate was concentrated under reduced pressure. The concentrated solution was purified by silica gel column chromatography (ethyl acetate:petroleum ether=1:100 to 1:0) to obtain Compound (5-2), a target compound. (Yield 29%)

Step (2): Preparation of Compound (5-3)

390 mg of Compound (5-2) (1.39 mmol, 1 equiv) was dissolved in 3.25 mL of ethanol and 0.65 mL of water. To the mixture solution, Fe (387 mg, 6.93 mmol, 5.00 equiv) and $NH_4Cl$ (370 mg, 6.93 mmol, 5.00 equiv) were introduced, and then the mixture solution was stirred for 12 hours at 80°C. After the reaction was finished, water and ethyl acetate were introduced to the reaction solution for extraction. The organic layer was separated, then dried with magnesium sulfate, and filtered. The remaining filtrate was concentrated under vacuum to obtain Compound (5-3), a target compound. (Yield 57%)

Step (3): Preparation of Compound (5-4)

Compound (5-4), a target compound, was obtained by repeating the same procedure as in Step (1) of Example 1 using Compound (5-3) instead of tert-butyl (2-aminophenyl)carbamate. (Yield 71%)

Step (4): Preparation of Compound (5)

Compound (5), a target compound, was obtained by repeating the same procedure as in Step (2) of Example 1 using Compound (5-4) instead of Compound (1). (Yield 96%)
$^1$H-NMR (400 MHz, DMSO-$d_6$) δ 11.37-11.33 (m, 1H), 8.42-8.36 (m, 1H), 7.76 (d, 1H, J=4.2 Hz), 7.16-7.13 (m, 1H),

6.94-6.90 (m, 1H), 6.80-6.75 (m, 1H), 6.56-6.51 (m, 1H), 5.01-4.85 (m, 1H), 4.50-4.02 (m, 5H), 3.98 (br s, 5H), 3.43 (br d, 2H, J=4.4 Hz), 2.77 (s, 6H), 1.97-1.53 (m, 2H), 1.03 (br d, 3H, J=7.0 Hz).

**Example 6: Preparation of Compound (6) (N-(2-amino-4-methoxyphenyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 6]

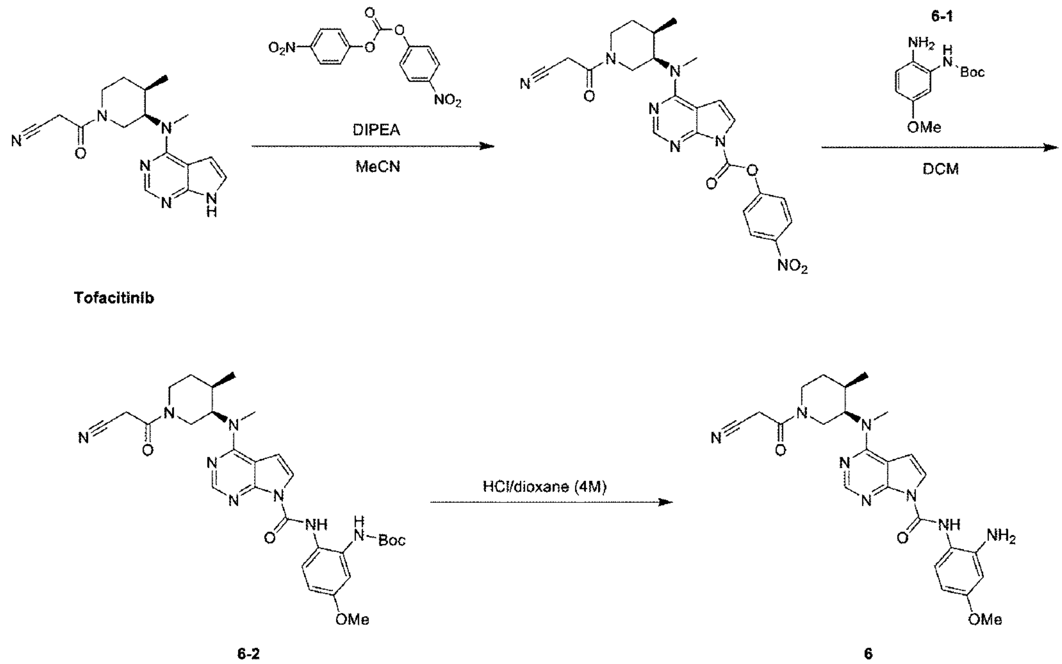


Tofacitinib

6-2

6

Step (1): Preparation of Compound (6-2)

Compound (6-2), a target compound, was obtained by repeating the same procedure as in Step (1) of Example 1 using Compound (6-1) instead of tert-butyl (2-aminophenyl)carbamate. (Yield 92%)

Step (2): Preparation of Compound (6)

Compound (6), a target compound, was obtained by repeating the same procedure as in Step (2) of Example 1 using Compound (6-2) instead of Compound (1). (Yield 6%)

$^1$H-NMR (400 MHz, DMSO-$d_6$) δ 11.07-10.86 (m, 1H), 8.49-8.01 (m, 1H), 7.74 (d, 1H, J=4.0 Hz), 7.31-7.14 (m, 1H), 6.96-6.79 (m, 1H), 6.60-6.38 (m, 1H), 6.22 (dd, 1H, J=2.4, 8.8 Hz), 5.10 (br s, 2H), 5.00-4.82 (m, 1H), 4.21-4.01 (m, 2H), 3.99-3.74 (m, 2H), 3.71 (m, 4H), 3.43 (br dd, 1H, J=3.2, 5.4 Hz), 3.33-3.32 (m, 2H), 3.32-3.26 (m, 1H), 2.46-2.37 (m, 1H), 1.93-1.69 (m, 1H), 1.68-1.53 (m, 1H), 1.03 (d, 3H, J=7.2 Hz).

**Example 7: Preparation of Compound (7) (N-(2-amino-5-methoxyphenyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 7]

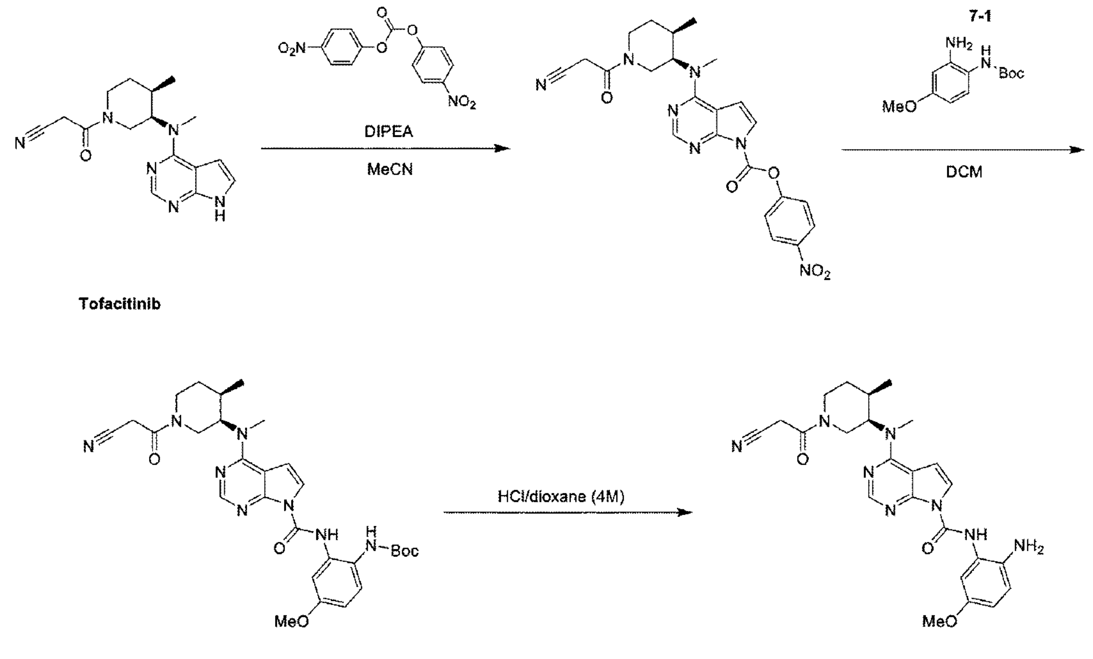


Tofacitinib

7-2

7

Step (1): Preparation of Compound (7-2)

Compound (7-2), a target compound, was obtained by repeating the same procedure as in Step (1) of Example 1 using Compound (7-1) instead of tert-butyl (2-aminophenyl) carbamate.

Step (2): Preparation of Compound (7)

Compound (7), a target compound, was obtained by repeating the same procedure as in Step (2) of Example 1 using Compound (7-2) instead of Compound (1). (Yield 15%)
$^1$H-NMR (400 MHz, DMSO-$d_6$) δ 11.50-11.31 (m, 1H), 8.43-8.35 (m, 1H), 7.76 (d, 1H, J=4.0 Hz), 7.35-7.23 (m, 1H), 6.91 (br s, 1H), 6.81 (br d, 1H, J=8.6 Hz), 6.66-6.49 (m, 1H), 4.91 (br d, 1H, J=3.2 Hz), 4.56 (br s, 2H), 4.23-4.05 (m, 2H), 3.96 (br dd, 1H, J=3.6, 12.4 Hz), 3.88-3.58 (m, 5H), 3.45-3.40 (m, 1H), 3.33 (br s, 2H), 3.28 (br d, 1H, J=4.0 Hz), 2.40 (br dd, 1H, J=5.6, 12.0 Hz), 1.89-1.52 (m, 2H), 1.03 (br d, 3H, J=7.2 Hz).

**Example 8: Preparation of Compound (8) (N-(2-amino-4-hydroxyphenyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 8]

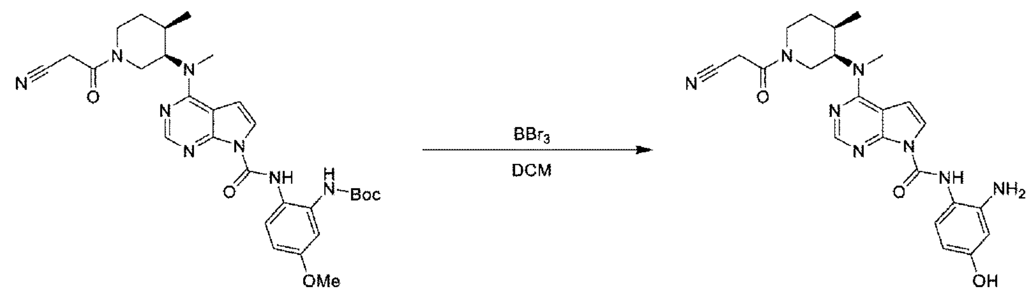


6-2

8

Step (1): Preparation of Compound (8)

To a solution in which 130 mg of Compound (6-2) (0.225 mmol, 1 equiv) was dissolved in 1.3 mL of dichloromethane, $BBr_3$ (169 mg, 0.676 mmol, 3.00 equiv) was introduced. The mixture solution was stirred for 4 hours at 0°C. After the reaction was finished, the reaction solution was concentrated under reduced pressure. The concentrated solution was purified by silica gel column chromatography to obtain Compound (8), a target compound. (Yield 30%)
$^1$H-NMR (400 MHz, DMSO-$d_6$) δ 10.91-10.85 (m, 1H), 9.11-8.97 (m, 1H), 8.38-8.35 (m, 1H), 7.72 (d, 1H, J=3.6 Hz), 7.04 (br d, 1H, J=8.4 Hz), 6.88 (br d, 1H, J=2.8 Hz), 6.28-6.21 (m, 1H), 6.04 (br d, 1H, J=8.8 Hz), 5.01-4.92 (m, 2H), 4.21-4.05 (m, 2H), 4.02-3.92 (m, 1H), 3.85-3.64 (m, 3H), 3.51-3.47 (m, 2H), 3.24-3.20 (m, 1H), 2.38 (br d, 2H, J=2.4 Hz), 1.90-1.68 (m, 1H), 1.66-1.54 (m, 1H), 1.16-0.98 (m, 3H).

**Example 9: Preparation of Compound (9) (N-(2-amino-5-hydroxyphenyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 9]

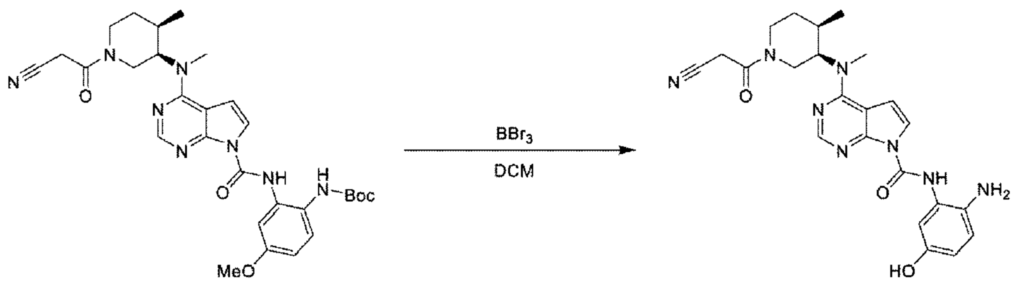


7-2 9

Step (1): Preparation of Compound (9)

Compound (9), a target compound, was obtained by repeating the same procedure as in Step (1) of Example 8 using Compound (7-2) instead of Compound (6-2). (Yield 12%)
$^1$H-NMR (400 MHz, DMSO-$d_6$) δ 11.39 (br d, 1H, J=8.0 Hz), 8.71 (s, 1H), 8.40-8.36 (m, 1H), 7.76 (d, 1H, J=4.0 Hz), 7.23-7.13 (m, 1H), 6.94-6.87 (m, 1H), 6.70 (d, 1H, J=8.4 Hz), 6.43 (dd, 1H, J=2.8, 8.4 Hz), 4.98-4.83 (m, 1H), 4.45-4.30 (m, 2H), 4.21-4.05 (m, 2H), 4.03-3.91 (m, 1H), 3.86-3.65 (m, 2H), 3.47-3.42 (m, 2H), 3.22 (br d, 1H, J=7.2 Hz), 2.42 (br d, 2H, J=8.2 Hz), 2.09-1.37 (m, 2H), 1.24 (s, 1H), 1.14-1.13 (m, 1H), 1.02 (br d, 3H, J=6.8 Hz).

**Example 10: Preparation of Compound (10) (N-(6-amino-1,3-dimethyl-2-oxo-2,3-dihydro-1H-benzo[d]imidazol-5-yl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 10]

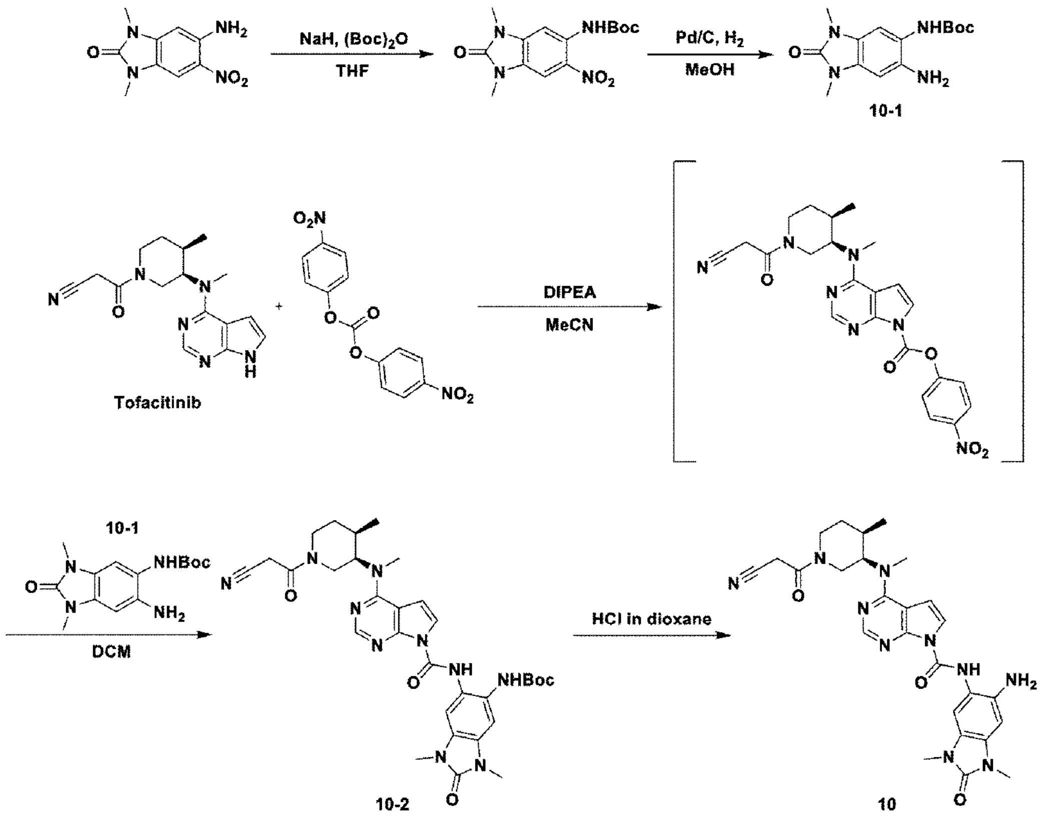


10-1

Tofacitinib

10-1

10-2

10

Step (1): Preparation of tert-butyl (1,3-dimethyl-6-nitro-2-oxo-2,3-dihydro-1H-benzo[d]imidazol-5-yl)carbamate

Tert-butyl (1,3-dimethyl-6-nitro-2-oxo-2,3-dihydro-1H-benzo[d]imidazol-5-yl)carbamate, a target compound, was obtained by repeating the same procedure as in Step (1) of Example 2 using 5-amino-1,3-dimethyl-6-nitro-1,3-dihydro-2H-benzo[d]imidazol-2-one instead of 4-methyl-2-nitroaniline.

Step (2): Preparation of Compound (10-1)

Compound (10-1), a target compound, was obtained by repeating the same procedure as in Step (2) of Example 2 using tert-butyl (1,3-dimethyl-6-nitro-2-oxo-2,3-dihydro-1H-benzo[d]imidazol-5-yl)carbamate instead of tert-butyl(4-methyl-2-nitrophenyl)carbamate. (Yield 35%)

Step (3): Preparation of Compound (10-2)

Compound (10-2), a target compound, was obtained by repeating the same procedure as in Step (1) of Example 1 using Compound (10-1) instead of tert-butyl (2-aminophenyl)carbamate. (Yield 42%)

Step (4): Preparation of Compound (10)

Compound (10), a target compound, was obtained by repeating the same procedure as in Step (2) of Example 1 using Compound (10-2) instead of Compound (1). (Yield 61%)
$^1$H-NMR (300 MHz, DMSO-$d_6$) δ 11.19-11.17 (m, 1H), 8.39-8.37 (d, 1H, J=6 Hz), 7.74 (d, 1H, J=2.1 Hz), 7.24-7.24 (m, 1H), 6.90 (s, 1H), 6.62-6.61 (d, 1H, J=2.1 Hz), 4.91 (s, 1H), 4.81-4.79 (m, 2H), 4.17-3.94 (m, 4H), 3.83-3.70 (m, 2H), 3.47-3.36 (m, 3H), 3.25-3.25 (m, 5H), 2.44-2.38 (m, 1H), 1.88-1.59 (m, 3H), 1.03-1.02 (d, 3H, J=3.6 Hz).

**Example 11: Preparation of Compound (11) (N-(2-aminophenyl)-N-((2-aminophenyl)carbamoyl)-4-(((3R,4R)-1-(2-cyanoacetyl)-4-methylpiperidin-3-yl)(methyl)amino)-7H-pyrrolo[2,3-d]pyrimidine-7-carboxamide)**

[Reaction Formula 11]

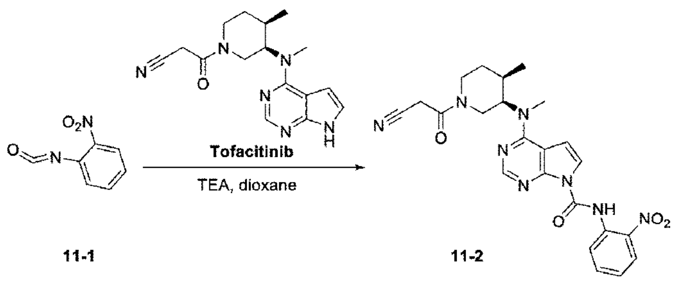


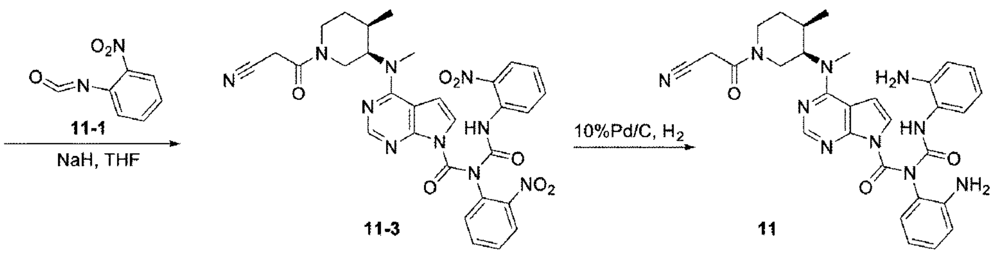


Step (1): Preparation of Compound (11-2)

To a solution in which 1 g of tofacitinib (3.20 mmol, 1 equiv) was dissolved in 30 mL of dioxane, Compound (11-1) (0.63 g, 3.84 mmol, 1.2 equiv) and triethylamine (421 mg, 4.16 mmol, 1.3 equiv) were introduced. The mixture solution was stirred for 2 days at 120°C. After the reaction was finished, the reaction solution was concentrated under reduced pressure. The concentrated solution was purified by silica gel column chromatography (methanol:dichloromethane=1:10) to obtain Compound (11-2), a target compound. (Yield 60%)

Step (2): Preparation of Compound (11-3)

After dissolving 2.9 g of Compound (11-2) (6.09 mmol, 1 equiv) in 50 mL of THF, the mixture solution was cooled to 0°C. 219 mg of NaH (60% content) (7.31 mmol, 1.2 equiv) was introduced thereto, and then the mixture solution was stirred for 30 minutes. The mixture solution was warmed to room temperature, and stirred for 18 hours. After the reaction was finished, water and ethyl acetate were introduced to the reaction solution for extraction. The organic layer was separated, then dried with magnesium sulfate, and filtered. The remaining filtrate was concentrated under reduced pressure. The concentrated solution was purified by silica gel column chromatography (methanol:dichloromethane=1:10) to obtain Compound (11-3), a target compound. (Yield 51%)

Step (3): Preparation of Compound (11)

To a solution in which 1.1 g of Compound (11-3) (1.72 mmol, 1 equiv) was dissolved in 50 mL of methanol, 100 mg of 10% Pd/C (10 wt.) was introduced, and then the mixture solution was stirred for 18 hours under hydrogen gas at atmospheric pressure. The mixture solution was warmed to room temperature, and stirred for 18 hours. After the reaction was finished, the reaction solution was filtered through Celite, and washed with 50 mL of methanol. The filtrate was concentrated under reduced pressure, and the concentrated solution was purified by silica gel column chromatography (methanol:dichloromethane=1:10) to obtain Compound (11), a target compound. (Yield 6%)
$^1$H-NMR (400 MHz, DMSO-$d_6$) δ 11.24-11.19 (m, 1H), 8.40-8.34 (m, 1H), 7.73 (dd, 1H, J=11.2, 4.0 Hz), 7.43 (dd, 1H, J=8.0, 1.2 Hz), 7.08-6.81 (m, 5H), 6.75-6.69 (m, 1H), 6.65-6.62 (m, 1H), 6.58-6.51 (m, 1H), 5.58 (d, 1H, J=8.4 Hz), 5.02 (br, 4H), 4.00-3.79 (m, 2H), 3.75-3.55 (m, 2H), 3.40-3.32 (m, 5H), 2.46-2.32 (m, 1H), 1.94-1.74 (m, 1H), 1.72-1.64 (m, 1H), 1.09-0.99 (m, 3H).

**Example 12: Preparation of Compound (12) (N-(2-amino-4-methylphenyl)-N-((2-amino-4-methylphenyl)carbamoyl)-4-(((3R,4R)-1-(2-cyanoacetyl)-4-methylpiperidin-3-yl) (methyl)amino)-7H-pyrrolo[2,3-d]pyrimidine-7-carboxamide)**

[Reaction Formula 12]

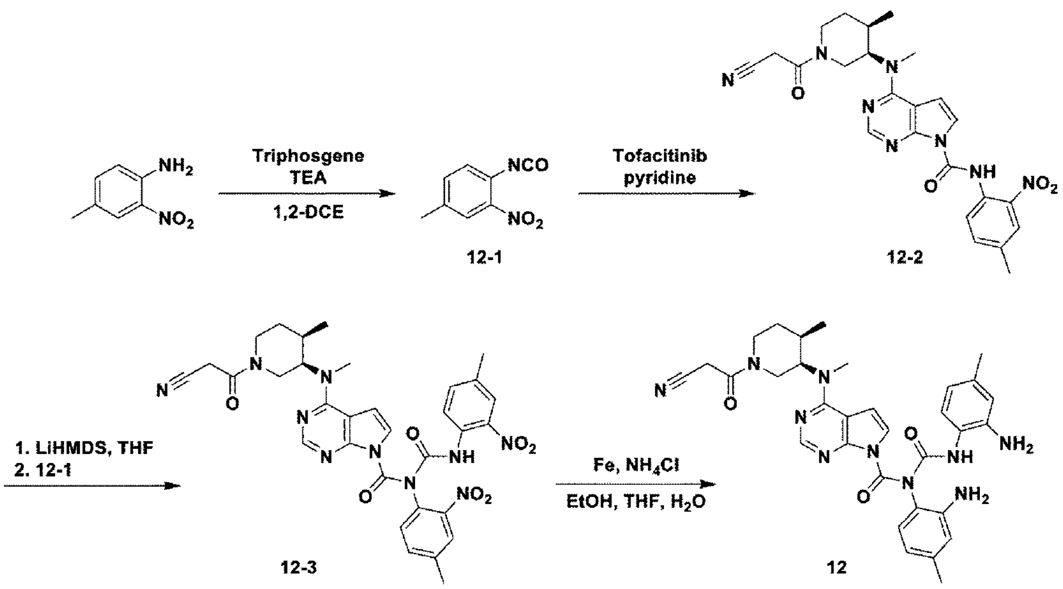


12-1 12-2 12-3 12

Step (1): Preparation of Compound (12-1)

After dissolving 500 mg of 4-methyl-2-nitroaniline (3.29 mmol, 1 equiv) in 28 mL of 1,2-dichloroethane, the mixture solution was cooled to 0°C to 5°C. 448 mg of triphosgene (1.64 mmol, 0.5 equiv) dissolved in 66 mL of 1,2-dichloroethane was introduced thereto, and the temperature was raised to room temperature. After that, 458 μL of trimethylamine (3.29 mmol, 1 equiv) was introduced thereto, and then the mixture solution was stirred under reflux for 2 to 16 hours. After the reaction was finished, the temperature was lowered, and the reaction solution was used in the next reaction without purification.

Step (2): Preparation of Compound (12-2)

After introducing 513 mg of tofacitinib (1.64 mmol, 1 equiv) and 265 μL of pyridine (3.29 mmol, 2 equiv) to Compound (12-1) (3.29 mmol, 2 equiv), the mixture solution was stirred under reflux for 1 to 2 hours. After the reaction was finished, the mixture solution was concentrated under reduced pressure. The concentrated solution was purified by silica gel column chromatography (ethyl acetate:hexane=1:1) to obtain Compound (12-2), a target compound. (Yield 85%)

Step (3): Preparation of Compound (12-3)

After dissolving 100 mg of Compound (12-2) (0.20 mmol, 1 equiv) in 2.6 mL of anhydrous THF, the mixture solution was cooled to 0°C to 5°C. 410 μL of 1M LiHMDS in THF (0.41 mmol, 2 equiv) was introduced thereto, and the temperature was raised to room temperature. After introducing Compound (12-1) thereto, the mixture solution was stirred for 1 hour. After the reaction was finished, water and ethyl acetate were introduced to the reaction solution for extraction. The organic layer was separated, then dried with magnesium sulfate, and filtered. The remaining filtrate was concentrated under reduced pressure. The concentrated solution was purified by silica gel column chromatography (dichloromethane:methanol=10:1) to obtain Compound (12-3), a target compound. (Yield 40%)

Step (4): Preparation of Compound (12)

54 mg of Compound (12-3) (0.08 mmol, 1 equiv) was dissolved in 5 mL of ethanol, 1.5 mL of THF and 1.5 mL of

water. 45 mg of Fe (0.81 mmol, 10 equiv) and 44 mg of $NH_4Cl$ (0.81 mmol, 10 equiv) were introduced to the mixture solution at room temperature, and the mixture solution was stirred under reflux for 1 hour. After the reaction was finished, ethyl acetate was introduced to the reaction solution for extraction. The organic layer was separated, then dried with magnesium sulfate, and filtered. The remaining filtrate was concentrated under reduced pressure. The concentrated solution was purified by silica gel column chromatography (dichloromethane:methanol=10:1) to obtain Compound (12), a target compound. (Yield 38%)

$^1H$-NMR (300 MHz, DMSO-$d_6$) δ 11.56-11.53, 11.12-11.09 (m, 1H), 10.46-10.42 (m, 1H), 8.16-8.13 (d, 1H, J=9 Hz), 7.28-7.25 (d, 1H, J=9 Hz), 6.93-6.87 (m, 2H), 6.80-6.71(m, 1H), 6.63-6.30 (m, 6H), 5.55-5.53 (m, 1H), 4.93 (s, 4H), 4.00-3.51 (m, 7H), 2.29-2.27 (m, 6H), 1.73-1.58 (m, 2H), 1.05-1.03 (m, 3H).

**Example 13: Preparation of Compound (13) (N-(2-amino-4-methoxyphenyl)-N-((2-amino-4-methoxyphenyl)carbamoyl)-4-(((3R,4R)-1-(2-cyanoacetyl)-4-methylpiperidin-3-yl) (methyl)amino)-7H-pyrrolo[2,3-d]pyrimidine-7-carboxamide)**

[Reaction Formula 13]

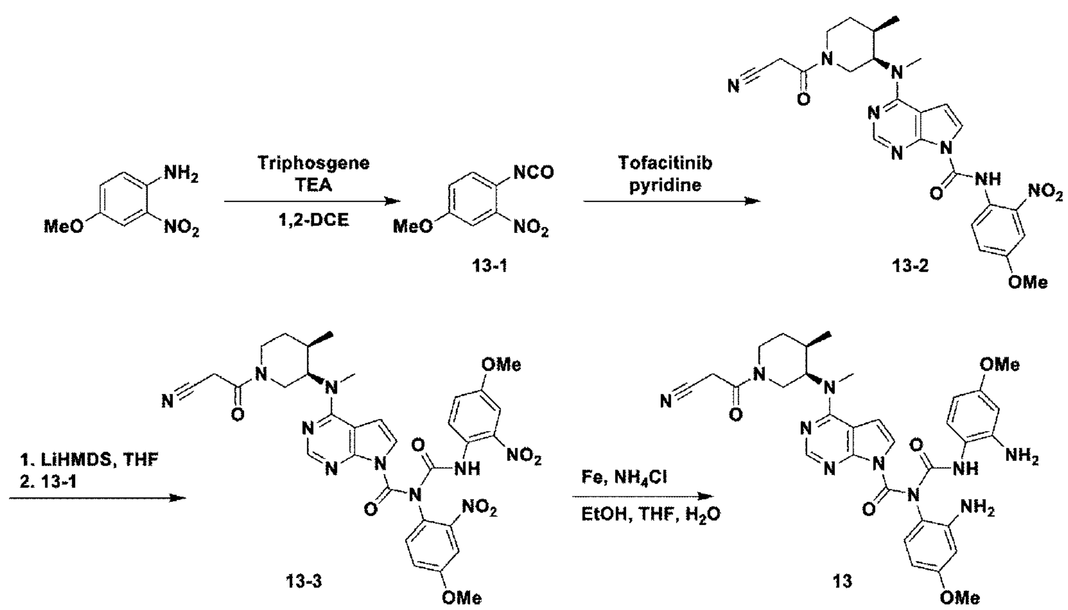


13-1 13-2 13-3 13

Step (1): Preparation of Compound (13-1)

Compound (13-1), a target compound, was obtained by repeating the same procedure as in Step (1) of Example 12 using 4-methoxy-2-nitroaniline instead of 4-methyl-2-nitroaniline.

Step (2): Preparation of Compound (13-2)

Compound (13-2), a target compound, was obtained by repeating the same procedure as in Step (2) of Example 12 using Compound (13-1) instead of Compound (12-1). (Yield 88%)

Step (3): Preparation of Compound (13-3)

Compound (13-3), a target compound, was obtained by repeating the same procedure as in Step (3) of Example 12 without purification using Compound (13-2) instead of Compound (12-2).

Step (4): Preparation of Compound (13)

Compound (13), a target compound, was obtained by repeating the same procedure as in Step (4) of Example 12 using Compound (13-3) instead of Compound (12-3). (Yield 27%)

$^1$H-NMR (300 MHz, DMSO-$d_6$) δ 10.97-10.94 (m, 1H), 10.49-10.36 (m, 1H), 8.39-8.36 (m, 1H), 7.75-7.71 (m, 1H), 7.21-7.18 (d, 1H, J=9 Hz), 6.93-6.85 (m, 1H), 6.81-6.78 (d, 1H, J=9 Hz), 6.52-6.49 (m, 2H), 6.38-6.11 (m, 4H), 5.54-5.50 (m, 1H), 5.08-5.02 (m, 4H), 3.96-3.61 (m, 13H), 1.76-1.66 (m, 2H), 1.05-1.01 (m, 3H).

**Example 14: Preparation of Compound (14) (N-(2-(3-(2-aminophenyl)-1-methylureido)ethyl)-4-(((3R,4R)-1-(2-cyanoacetyl)-4-methylpiperidin-3-yl)(methyl)amino)-N-methyl-7H-pyrrolo[2,3-d]pyrimidine-7-carboxamide)**

[Reaction Formula 14]

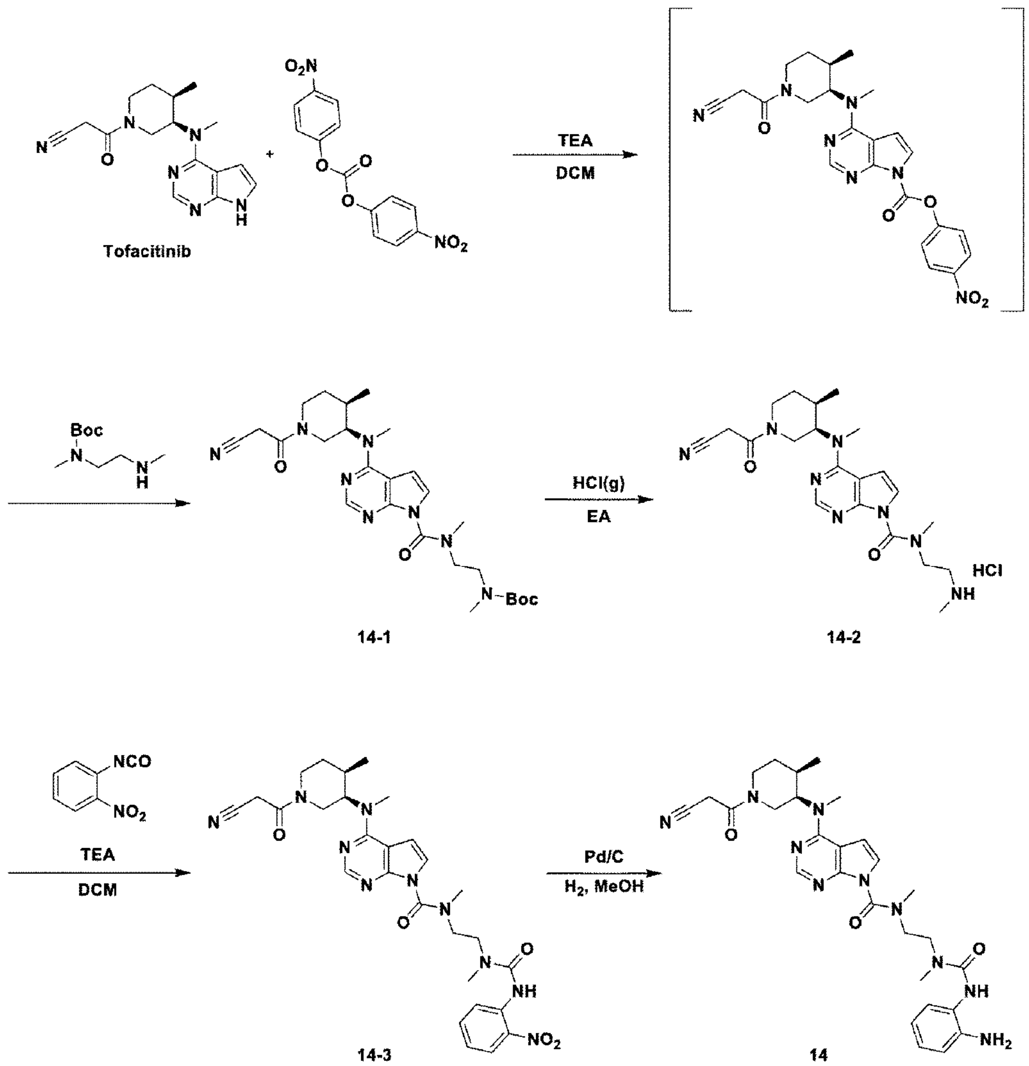


14-1

14-2

14-3

14

Step (1): Preparation of Compound (14-1)

5 g of tofacitinib (16.0 mmol, 1 equiv) and 3.24 g of TEA (32.0 mmol, 2 equiv) were dissolved in dichloromethane. 5.84 g of bis(4-nitrophenyl)carbonate (19.2 mmol, 1.2 equiv) was introduced thereto, and then the mixture solution was stirred for 2 hours at 40°C. 4.54 g of tert-butyl methyl(2-(methylamino)ethyl)carbamate (24.0 mmol, 1.5 equiv) was introduced thereto, and then the mixture solution was stirred for 16 hours at 40°C. After the reaction was finished, water and dichloromethane were introduced to the reaction solution for extraction. The organic layer was separated, then dried with magnesium sulfate, and filtered. The remaining filtrate was concentrated under reduced pressure. The concentrated solution was purified by silica gel column chromatography (dichloromethane:methanol=20:1) to obtain Compound (14-1), a target compound. (Yield 39%)

Step (2): Preparation of Compound (14-2)

After dissolving 3.3 g of Compound (14-1) (7.7 mmol, 1 equiv) in 30 mL of ethyl acetate, the mixture solution was cooled to 0°C to 5°C. To the mixture solution, hydrochloric acid gas was introduced for 2 minutes. After the reaction was finished, the reaction solution was filtered to obtain Compound (14-2), a target compound.

Step (3): Preparation of Compound (14-3)

900 mg of Compound (14-2) (2.1 mmol, 1 equiv) and 320 mg of TEA (3.2 mmol, 1.5 equiv) were dissolved in 20 mL of dichloromethane. 415 mg of 1-isocyanato-2-nitrobenzene (2.5 mmol, 1.2 equiv) was introduced thereto, and then the mixture solution was stirred for 16 hours at room temperature. After the reaction was finished, water and dichloromethane were introduced to the reaction solution for extraction. The organic layer was separated, then dried with magnesium sulfate, and filtered. The remaining filtrate was concentrated under reduced pressure. The concentrated solution was purified by silica gel column chromatography (dichloromethane:methanol=20:1) to obtain Compound (14-3), a target compound. (Yield 36%)

Step (4): Preparation of Compound (14)

445 mg of Compound (14-3) (0.75 mmol, 1 equiv) was dissolved in 10 mL of methanol. 80 mg of 10% Pd/C was introduced to the mixture solution at room temperature, and the mixture solution was stirred for 2 hours under hydrogen gas at atmospheric pressure. After the reaction was finished, the mixture solution was purified using a Celite filter, and washed with 20 mL of methanol. The filtrate was concentrated under reduced pressure, and the concentrated solution was purified by silica gel column chromatography (dichloromethane:methanol=20:1) to obtain Compound (14), a target compound. (Yield 36%)
$^1$H-NMR (400 MHz, DMSO-$d_6$) δ 8.25-8.18 (m, 1H), 7.73-7.50 (m, 1H), 7.40-7.20 (m, 1H), 7.06-6.82 (m, 2H), 6.80-6.65 (m, 2H), 6.51 (d, 1H, J=7.2 Hz), 4.95-4.65 (m, 3H), 4.20-4.00 (m, 2H), 3.99-3.35 (m, 8H), 3.27 (s, 3H), 3.16-2.98 (m, 3H), 2.96-2.62 (m, 3H), 2.46-2.31 (m, 1H), 1.89-1.68 (m, 1H), 1.63-1.52 (m, 1H), 1.01 (d, 3H, J=7.2 Hz).

**Example 15: Preparation of Compound (15) (N-(2-(3-(2-amino-4-methylphenyl)-1-methylureido) ethyl)-4-(((3R,4R)-1-(2-cyanoacetyl)-4-methylpiperidin-3-yl) (methyl)amino)-N-methyl-7H-pyrrolo[2,3-d]pyrimidine-7-carboxamide)**

[Reaction Formula 15]

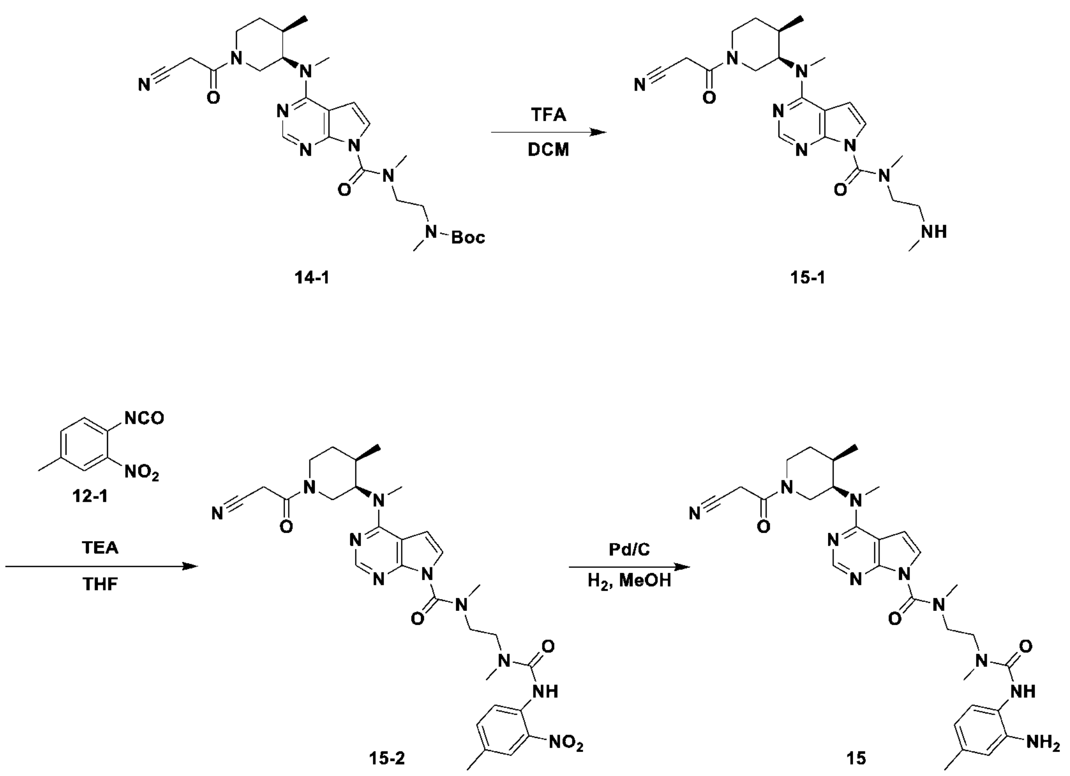


Step (1): Preparation of Compound (15-1)

250 mg of Compound (14-1) (0.47 mmol, 1 equiv) was dissolved in 20 mL of dichloromethane. 5 mL of TFA (20 V) was introduced thereto, and then the mixture solution was stirred for 2 hours at room temperature. After the reaction was finished, the mixture solution was concentrated under reduced pressure to obtain Compound (15-1). (Yield 99%)

Step (2): Preparation of Compound (15-2)

202 mg of Compound (15-1) (0.47 mmol, 1 equiv) and 84 mg of Compound (12-1) were dissolved in 40 mL of anhydrous THF. 47 mg of TEA (0.47, 1 equiv) was introduced thereto, and then the mixture solution was stirred for 16 hours at room temperature. After the reaction was finished, the concentrated solution that was concentrated under reduced pressure was purified by silica gel column chromatography (dichloromethane:methanol=20:1) to obtain Compound (15-2), a target compound. (Yield 42%)

Step (3): Preparation of Compound (15)

120 mg of Compound (15-2) (0.20 mmol, 1 equiv) was dissolved in 15 mL of methanol. 100 mg of 10% Pd/C was introduced to the mixture solution at room temperature, and the mixture solution was stirred for 1 hour under hydrogen gas at atmospheric pressure. After the reaction was finished, the mixture solution was filtered using a Celite filter, and then the filtrate was concentrated under reduced pressure. The concentrated solution was purified by reverse-phase prep-HPLC to obtain Compound (15), a target compound. (Yield 40%)
$^1$H-NMR (400 MHz, DMSO-$d_6$) δ 8.22-8.20 (m, 1H), 7.56-7.53 (m, 1H), 7.30 (s, 1H), 6.86-6.76 (m, 2H), 6.52 (s, 1H), 6.34-6.62 (d, 1H, J=7.2 Hz), 4.85 (s, 1H), 4.65 (s, 2H), 4.17-3.91 (m, 3H), 3.78-3.63 (m, 4h), 3.46-3.37 (m, 3H), 3.27 (s, 4H), 3.11-3.06 (m, 1H), 2.88 (s, 1H), 2.67-2.63 (m, 1H), 2.39-2.36 (m, 1H), 2.15 (s, 3H), 1-84-1.68 (m, 1H), 1.62-1.57 (m, 1H), 1.02-1.00 (d, 3H, J=7.2 Hz).

**Example 16: Preparation of Compound (16) (N-(2-(3-(2-amino-5-methylphenyl)-1-methylureido) ethyl)-4-(((3R,4R)-1-(2-cyanoacetyl)-4-methylpiperidin-3-yl) (methyl)amino)-N-methyl-7H-pyrrolo[2,3-d]pyrimidine-7-carboxamide)**

[Reaction Formula 16]

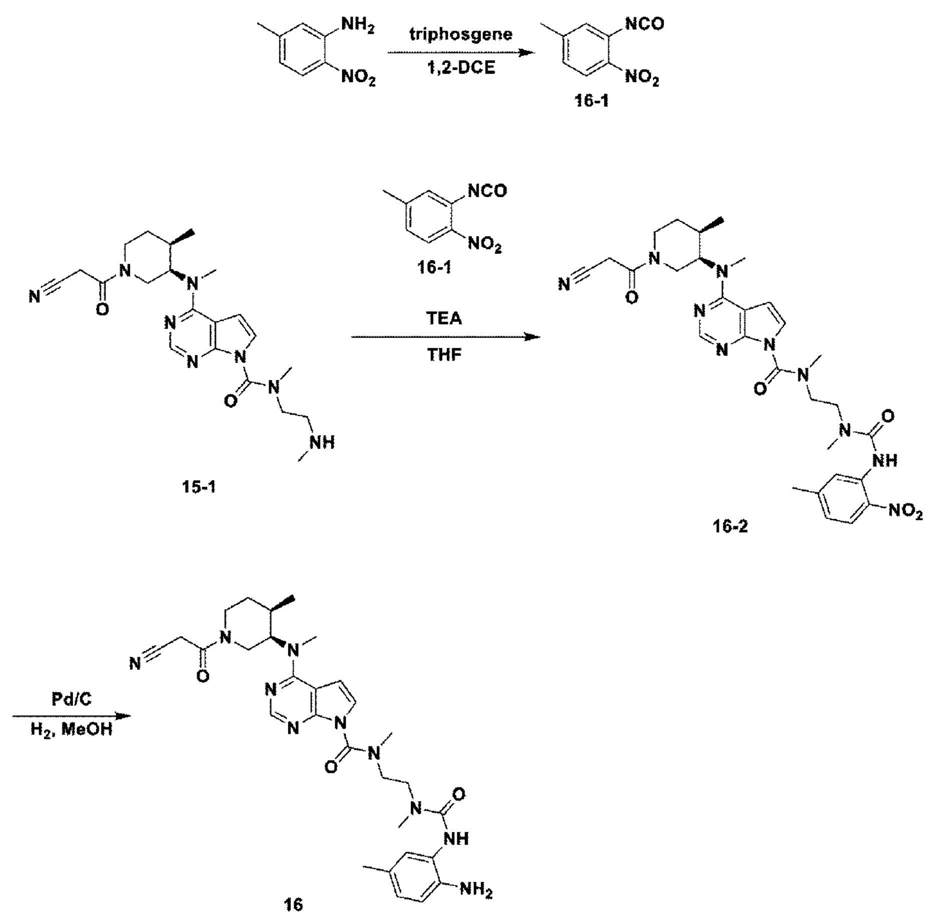


Step (1): Preparation of Compound (16-1)

After dissolving 152 mg of 5-methyl-2-nitroaniline (1.0 mmol, 1 equiv) in 10 mL of 1,2-dichloroethane, the mixture solution was cooled to 0°C to 5°C. 148 mg of triphosgene (0.5 mmol, 0.5 equiv) dissolved in 20 mL of 1,2-dichloroethane was introduced thereto, and the temperature was raised to room temperature. After that, the mixture solution was stirred under reflux for 16 hours. After the reaction was finished, the mixture solution was concentrated under reduced pressure to obtain Compound (16-1). (Yield 96%)

Step (2): Preparation of Compound (16-2)

Compound (16-2), a target compound, was obtained by repeating the same procedure as in Step (2) of Example 15 using Compound (16-1) instead of Compound (12-1). (Yield 58%)

Step (3): Preparation of Compound (16)

Compound (16), a target compound, was obtained by repeating the same procedure as in Step (3) of Example 15 using Compound (16-2) instead of Compound (15-2). (Yield 58%)
$^1$H-NMR (400 MHz, DMSO-$d_6$) $\delta$ 8.22-8.21 (m, 1H), 7.60 (s, 1H), 7.34-7.29 (m, 1H), 6.82-6.76 (m, 2H), 6.71-6.69 (d, 1H, J=8 Hz), 6.63-6.61 (d, 1H, J=8 Hz), 4.85 (s, 1H), 4.53 (s, 2H), 4.16-3.91 (m, 3H), 3.86-3.63 (m, 4H), 3.55-3.40 (m, 3H), 3.27

(s, 4H), 3.22-2.97 (m, 3H), 2.89-2.67 (m, 1H), 2.63-2.50 (m, 1H), 2.39-2.36 (m, 1H), 2.13 (s, 3H), 1.86-1.71 (m, 1H), 1.61-1.57 (m, 1H), 1.02-1.00 (d, 3H, J=7.2 Hz).

**Example 17: Preparation of Compound (17) (N-(2-(3-(2-amino-4-methoxyphenyl)-1-methylureido) ethyl)-4-(((3R,4R)-1-(2-cyanoacetyl)-4-methylpiperidin-3-yl) (methyl)amino)-N-methyl-7H-pyrrolo[2,3-d]pyrimidine-7-carboxamide)**

[Reaction Formula 17]

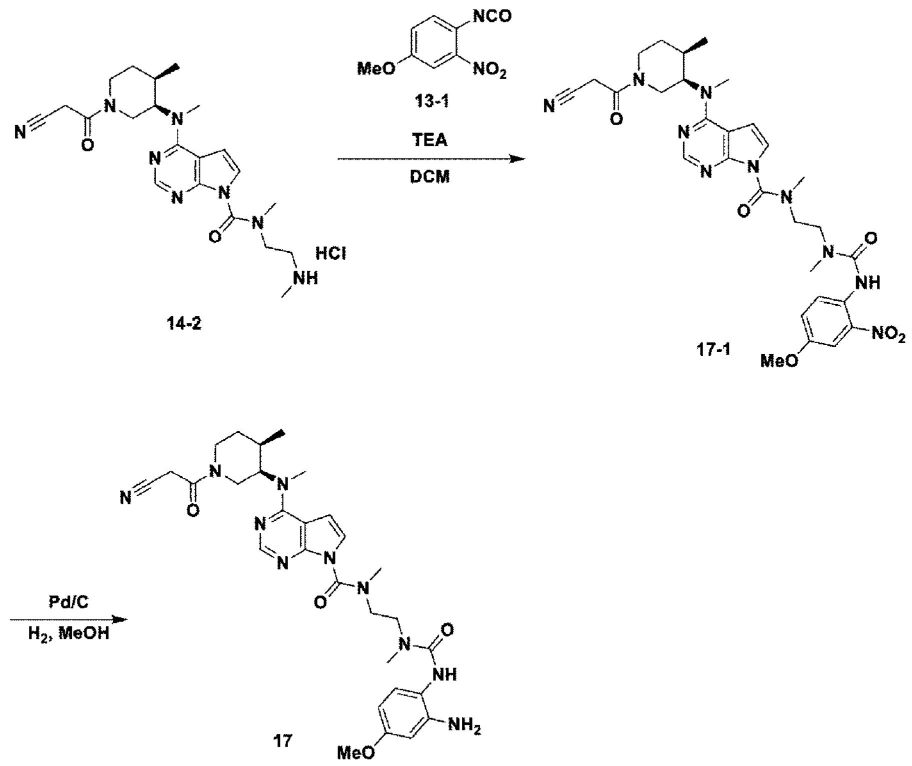


Step (1): Preparation of Compound (17-1)

After dissolving 1.2 g of Compound (14-2) (2.67 mmol, 1 equiv) and 540 mg of TEA (5.34 mmol, 2 equiv) in 10 mL of anhydrous THF, the mixture solution was cooled to 0°C to 5°C. Compound (13-1) (2.67 mmol, 1 equiv) was introduced thereto, and then the mixture solution was stirred for 16 hours at room temperature. After the reaction was finished, the reaction solution was concentrated under reduced pressure, and the concentrated solution was purified by silica gel column chromatography (dichloromethane:methanol=25:1) to obtain Compound (17-1), a target compound. (Yield 24%)

Step (2): Preparation of Compound (17)

400 mg of Compound (17-1) (0.64 mmol, 1 equiv) was dissolved in 20 mL of methanol. 80 mg of 10% Pd/C was introduced to the mixture solution at room temperature, and the mixture solution was stirred for 2 hours under hydrogen gas at atmospheric pressure. After the reaction was finished, the mixture solution was filtered using a Celite filter, and then the filtrate was concentrated under reduced pressure. The concentrated solution was purified by reverse-phase prep-HPLC to obtain Compound (17), a target compound. (Yield 29%)
$^1$H-NMR (400 MHz, DMSO-$d_6$) δ 8.21 (d, 1H, J=5.6 Hz), 7.51 (s, 1H), 7.30 (s, 1H), 6.90-6.66 (m, 2H), 6.29 (d, 1H, J=2.7 Hz), 6.10 (dd, 1H, J=8.4, 2.1 Hz), 4.94-4.66 (m, 3H), 4.20-3.53 (m, 10H), 3.48-3.35 (m, 3H), 3.27 (s, 3H), 3.17-2.98 (m, 3H), 2.88 (s, 2H), 2.64 (s, 1H), 2.45-2.31 (m, 1H), 1.88-1.66 (m, 1H), 1.64-1.51 (m, 1H), 1.02 (d, 3H, J=7.2 Hz).

**Example 18: Preparation of Compound (18) (N-(2-(3-(2-amino-5-methoxyphenyl)-1-methylureido) ethyl)-4-(((3R,4R)-1-(2-cyanoacetyl)-4-methylpiperidin-3-yl) (methyl)amino)-N-methyl-7H-pyrrolo[2,3-d]pyrimidine-7-carboxamide)**

[Reaction Formula 18]

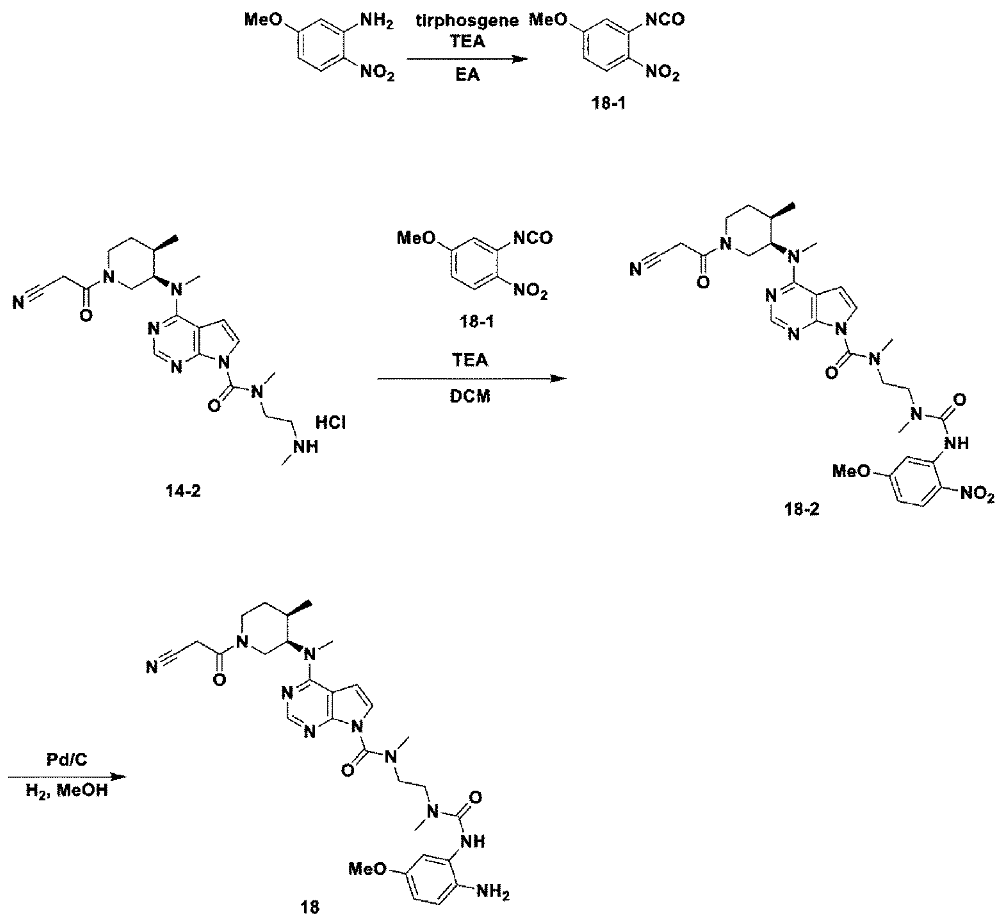


Step (1): Preparation of Compound (18-1)

After dissolving 500 mg of 5-methoxyl-2-nitroaniline (2.97 mmol, 1 equiv) in 10 mL of ethyl acetate, the mixture solution was cooled to 0°C to 5°C. 360 mg of TEA (3.57 mmol, 10.2 equiv) and 291 mg of triphosgene (0.98 mmol, 0.33 equiv) were introduced thereto, and the mixture solution was stirred for 1 hour at room temperature. After the reaction was finished, the reaction solution was used in the next reaction without purification.

Step (2): Preparation of Compound (18-2)

Compound (18-2), a target compound, was obtained by repeating the same procedure as in Step (1) of Example 17 using Compound (18-1) instead of Compound (13-1). (Yield 14%)

Step (3): Preparation of Compound (18)

Compound (18), a target compound, was obtained by repeating the same procedure as in Step (2) of Example 17 using Compound (18-2) instead of Compound (17-1). (Yield 49%)
$^1$H-NMR (400 MHz, DMSO-$d_6$) $\delta$ 8.26-8.16 (m, 1H), 7.69 (s, 1H), 7.45-7.19 (m, 1H), 6.85-6.60 (m, 3H), 6.56-6.48 (m, 1H), 4.96-4.76 (m, 1H), 4.57 (s, 2H), 4.20-3.50 (m, 10H), 3.50-3.35 (m, 3H), 3.30-3.20 (m, 3H), 3.18-3.00 (m, 3H), 2.96-2.78 (m, 2H), 2.74-2.56 (m, 1H), 2.44-2.31 (m, 1H), 1.90-1.65 (m, 1H), 1.64-1.50 (m, 1H), 1.01 (d, 3H, J=8 Hz).

**Example 19: Preparation of Compound (19) (N-(2-(3-(2-amino-4-hydroxyphenyl)-1-methylureido) ethyl)-4-(((3R,4R)-1-(2-cyanoacetyl)-4-methylpiperidin-3-yl) (methyl)amino)-N-methyl-7H-pyrrolo[2,3-d]pyrimidine-7-carboxamide)**

[Reaction Formula 19]

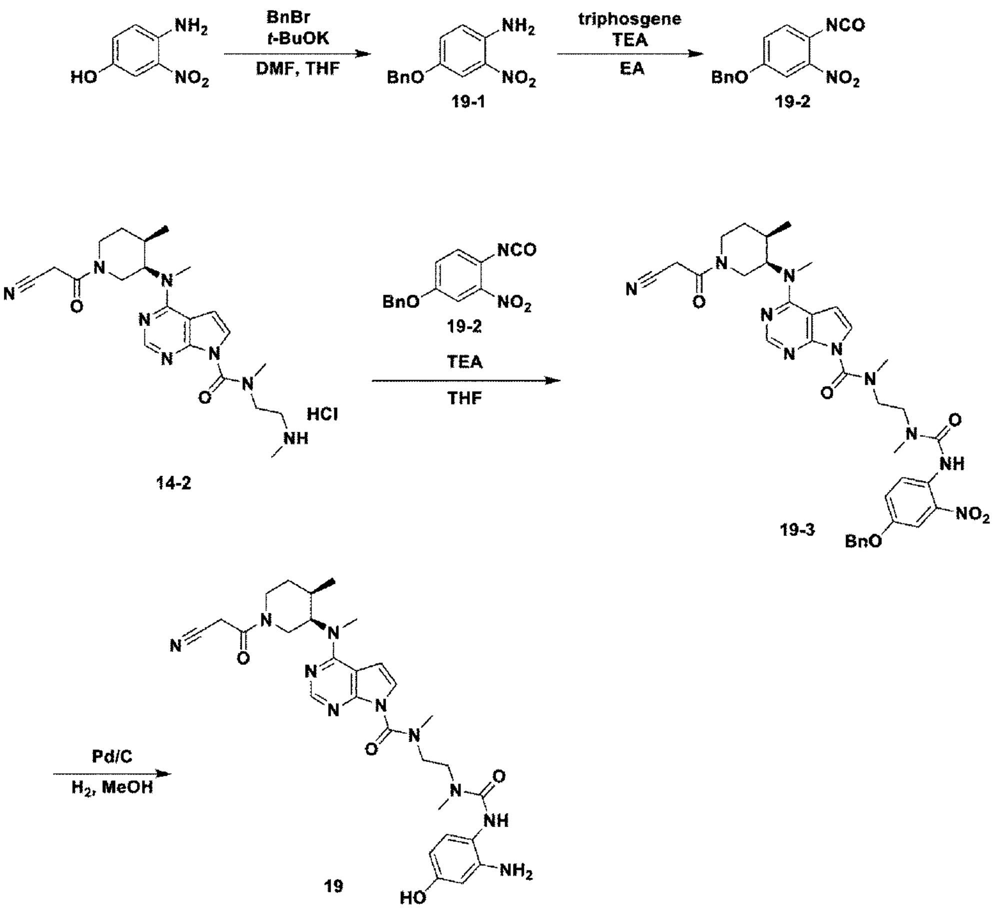


Step (1): Preparation of Compound (19-1)

After dissolving 1 g of 4-amino-3-nitrophenol (6.49 mmol, 1 equiv) in 10 mL of DMF, the mixture solution was cooled to 0°C to 5°C. 801 mg of t-BuOK (7.14 mmol, 1.1 equiv) dissolved in 5 mL of THF was introduced thereto, and the mixture solution was stirred for 20 minutes at 0°C. After that, 1.2 g of benzyl bromide (7.14 mmol, 1.1 equiv) dissolved in 5 mL of DMF was added dropwise thereto, and the mixture solution was stirred for 20 minutes at 0°C. After the reaction was finished, 20 mL of an aqueous $NH_4Cl$ solution was added dropwise thereto, and then ethyl acetate was introduced thereto for extraction. The organic layer was separated, then dried with magnesium sulfate, and filtered. The remaining filtrate was concentrated under reduced pressure. The concentrated solution was purified by silica gel column chromatography (dichloromethane:methanol=20:1) to obtain Compound (19-1), a target compound. (Yield 76%)

Step (2): Preparation of Compound (19-2)

Compound (19-2), a target compound, was obtained by repeating the same procedure as in Step (1) of Example 18 using Compound (19-1) instead of 5-methoxyl-2-nitroaniline.

Step (3): Preparation of Compound (19-3)

Compound (19-3), a target compound, was obtained by repeating the same procedure as in Step (1) of Example

17 using Compound (19-2) instead of Compound (13-1). (Yield 11%)

Step (4): Preparation of Compound (19)

Compound (19), a target compound, was obtained by repeating the same procedure as in Step (2) of Example 17 using Compound (19-3) instead of Compound (17-1). (Yield 57%)
$^1$H-NMR (400 MHz, DMSO-$d_6$) δ 8.82-8.75 (m, 1H), 8.25-8.16 (m, 1H), 7.49-7.21 (m, 2H), 6.85-6.50 (m, 2H), 6.17-6.09 (m, 1H), 5.98-5.88 (m, 1H), 4.96-4.76 (m, 1H), 4.63 (s, 2H), 4.20-3.50 (m, 7H), 3.49-3.34 (m, 3H), 3.30-3.18 (m, 3H), 3.16-2.95 (m, 3H), 2.94-2.77 (m, 2H), 2.71-2.54 (m, 1H), 2.45-2.31 (m, 1H), 1.91-1.65 (m, 1H), 1.64-1.50 (m, 1H), 1.02 (d, 3H, J=8.0 Hz).

**Example 20: Preparation of Compound (20) (N-(2-(3-(2-amino-5-hydroxyphenyl)-1-methylureido) ethyl)-4-(((1S,2R)-5-(2-cyanoacetyl)-2-methylcyclohexyl) (methyl)amino)-N-methyl-7H-pyrrolo[2,3-d]pyrimidine-7-carboxamide)**

[Reaction Formula 20]

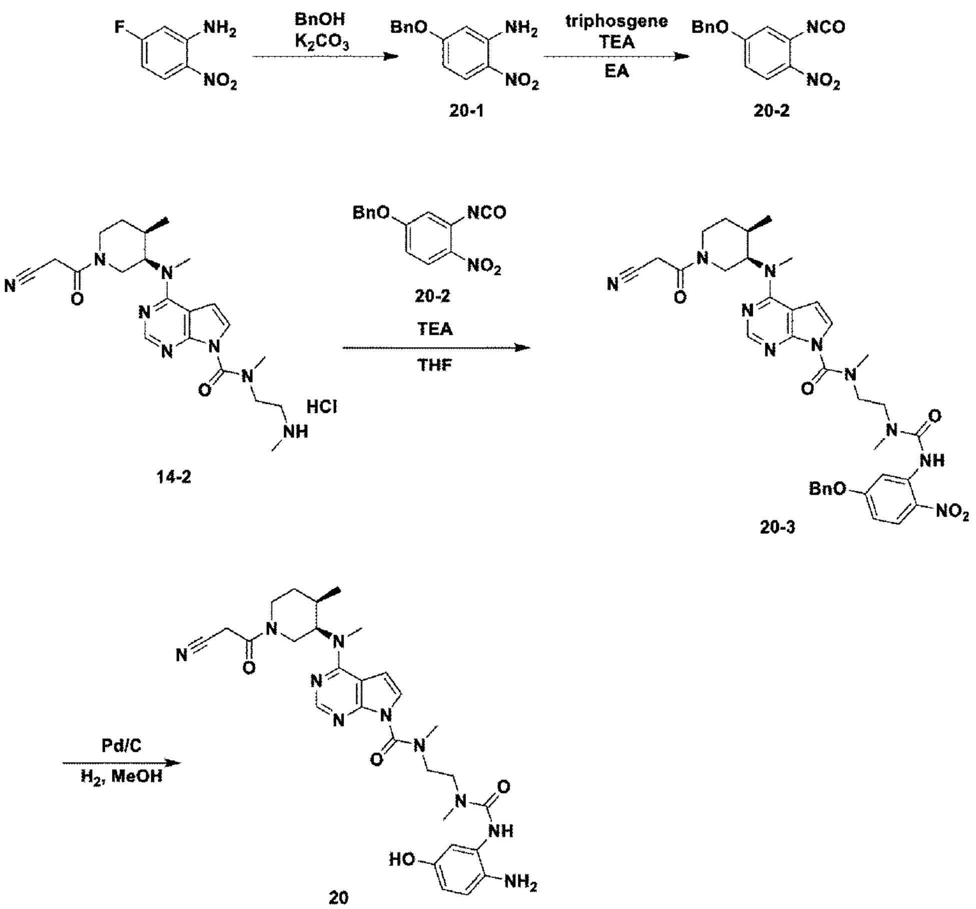


20-1 20-2 14-2 20-2 20-3 20

Step (1): Preparation of Compound (20-1)

After dissolving 1.5 g of 5-fluoro-2-nitroaniline (9.6 mmol, 1 equiv) and 2.6 g of $K_2CO_3$ in 40 mL of n-butanol, the mixture solution was stirred under reflux for 16 hours at 100°C. After the reaction was finished, the mixture solution was cooled to room temperature, then filtered, and concentrated under reduced pressure. The concentrated solution was purified by silica gel column chromatography (PE:ethyl acetate=3:1) to obtain Compound (20-1), a target compound. (Yield 23%)

Step (2): Preparation of Compound (20-2)

Compound (20-2), a target compound, was obtained by repeating the same procedure as in Step (1) of Example 18 using Compound (20-1) instead of 5-methoxyl-2-nitroaniline.

Step (3): Preparation of Compound (20-3)

Compound (20-3), a target compound, was obtained by repeating the same procedure as in Step (1) of Example 17 using Compound (20-2) instead of Compound (13-1). (Yield 13%)

Step (4): Preparation of Compound (20)

Compound (20), a target compound, was obtained by repeating the same procedure as in Step (2) of Example 17 using Compound (20-3) instead of Compound (17-1). (Yield 45%)
$^{1}$H-NMR (400 MHz, DMSO-$d_6$) δ 8.48 (s, 1H), 8.21 (d, 1H, J=5.6 Hz), 7.61 (s, 1H), 7.42-7.22 (m, 1H), 6.81-6.52 (m, 3H), 6.33 (dd, 1H, J=8.4, 2.4 Hz), 4.84 (s, 1H), 4.56 (s, 2H), 4.20-3.51 (m, 7H), 3.40 (s, 3H), 3.27 (s, 3H), 3.09 (d, 3H, J=21.2 Hz), 2.88 (s, 2H), 2.73-2.56 (m, 1H), 2.37 (s, 1H), 1.90-1.66 (m, 1H), 1.57 (s, 1H), 1.01 (d, 3H, J=7.2 Hz).

**Example 21: Preparation of Compound (21) (N-(2-(3-(2-amino-5-(dimethylamino)phenyl)-1-methylureido) ethyl)-4-(((3R,4R)-1-(2-cyanoacetyl)-4-methylpiperidin-3-yl) (methyl)amino)-N-methyl-7H-pyrrolo[2,3-d]pyrimidine-7-carboxamide)**

[Reaction Formula 21]

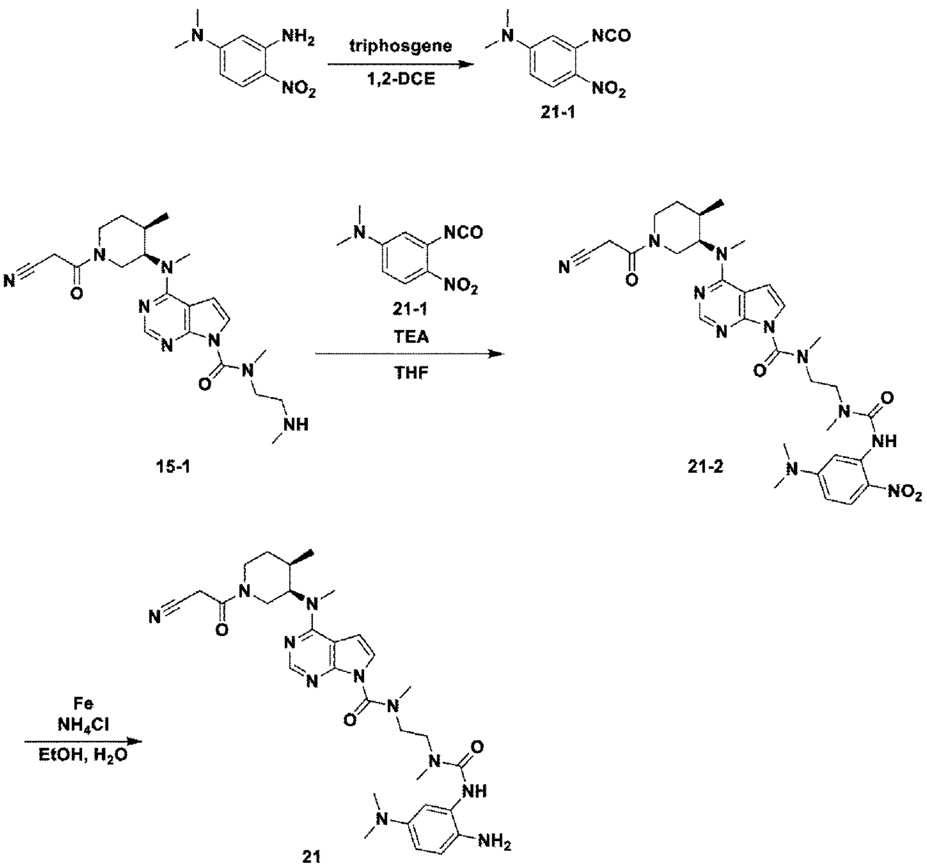


Step (1): Preparation of Compound (21-1)

After dissolving 181 mg of N1,N1-dimethyl-4-nitrobenzene-1,3-diamine (1.0 mmol, 1 equiv) in 10 mL of 1,2-dichloroethane, the mixture solution was cooled to 0°C to 5°C. 148 mg of triphosgene (0.5 mmol, 0.5 equiv) dissolved in 20 mL of 1,2-dichloroethane was introduced thereto, and the temperature was raised to room temperature. After that, the mixture solution was stirred under reflux for 16 hours. After the reaction was finished, the mixture solution was concentrated under reduced pressure to obtain Compound (21-1). (Yield 97%)

Step (2): Preparation of Compound (21-2)

Compound (21-2), a target compound, was obtained by repeating the same procedure as in Step (2) of Example 15 using Compound (21-1) instead of Compound (12-1). (Yield 76%) .

Step (3): Preparation of Compound (21)

110 mg of Compound (21-2) (0.17 mmol, 1 equiv) was dissolved in 10 mL of ethanol and 3 mL of water. 95 mg of Fe (1.7 mmol, 10 equiv) and 91 mg of $NH_4Cl$ (1.7 mmol, 10 equiv) were introduced to the mixture solution at room temperature, and the mixture solution was stirred under reflux for 1 hour. After the reaction was finished, the mixture solution was filtered using a Celite filter, and then the filtrate was concentrated under reduced pressure. The concentrated solution was purified by reverse-phase prep-HPLC to obtain Compound (21), a target compound. (Yield 34%)
$^1$H-NMR (400 MHz, DMSO-$d_6$) δ 8.22-8.19 (m, 1H), 7.67 (s, 1H), 7.39-7.28 (m, 1H), 6.74-6.74 (m, 1H), 6.65-6.63 (m, 2H), 6.45-6.42 (dd, 1H, J=8.8 Hz, 2.8 Hz), 5.59-5.21 (m, 2H), 4.85 (s, 1H), 4.17-3.91 (m, 3H), 3.86-3.63 (m, 4H), 3.54-3.31 (m, 3H), 3.26 (s, 3H), 3.12-3.07 (m, 3H), 2.87-2.75 (m, 2H), 2.71 (s, 6H), 2.67-2.63 (m, 1H), 2.49-2.33 (m, 1H), 1.86-1.57 (m, 2H), 1.02-1.00 (d, 3H, J=6.8 Hz).

**Example 22: Preparation of Compound (22) (N-(2-(3-(6-amino-1,3-dimethyl-2-oxo-2,3-dihydro-1H-benzo[d]imidazol-5-yl)-1-methylureido)ethyl)-4-(((3R,4R)-1-(2-cyanoacetyl)-4-methylpiperidin-3-yl) (methyl)amino)-N-methyl-7H-pyrrolo[2,3-d]pyrimidine-7-carboxamide)**

[Reaction Formula 22]

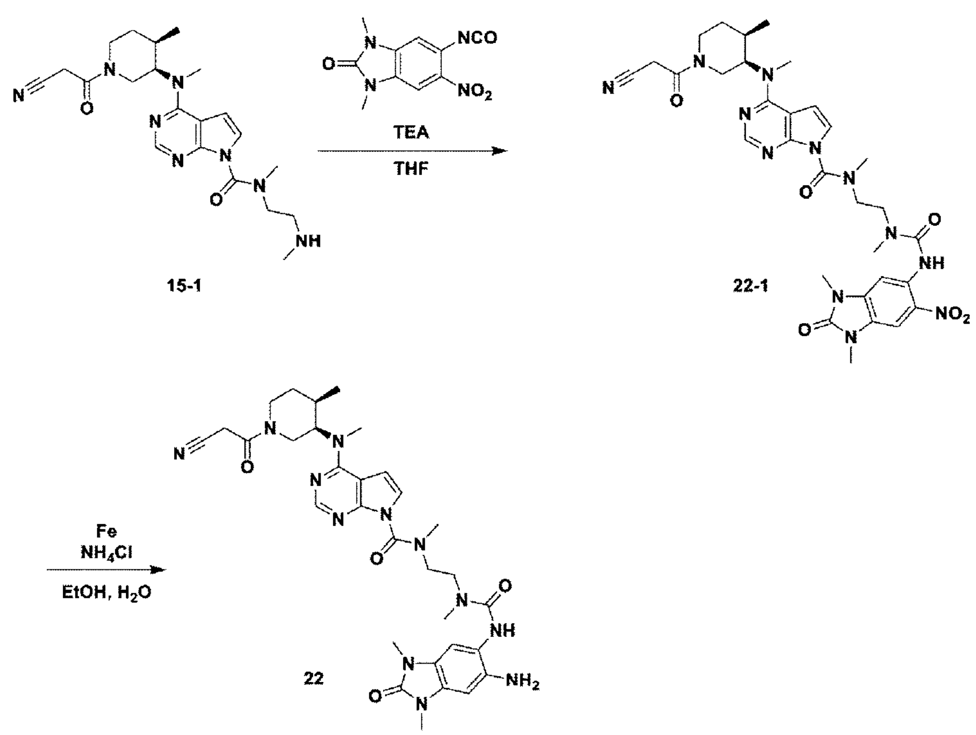

15-1
22-1
22

Step (1): Preparation of Compound (22-1)

Compound (22-1), a target compound, was obtained by repeating the same procedure as in Step (2) of Example

15 using 5-isocyanato-1,3-dimethyl-6-nitro-1H-benzo[d]imidazol-2(3H)-one instead of Compound (12-1). (Yield 70%)

Step (2): Preparation of Compound (22)

Compound (22), a target compound, was obtained by repeating the same procedure as in Step (3) of Example 21 using Compound (22-1) instead of Compound (21-2). (Yield 13%)
$^1$H-NMR (400 MHz, DMSO-$d_6$) δ 8.21-8.21 (m, 1H), 7.66 (s, 1H), 7.38-7.28 (m, 1H), 6.77 (s, 2H), 6.51 (s, 1H), 4.88-4.84 (m, 1H), 4.55-4.50 (m, 2H), 4. 18-3. 62 (m, 7H), 3.45-3.40 (m, 3H), 3.26-3.05 (m, 12H), 2. 89 (s, 2H), 2.66-2.61 (m, 1H), 2.41-2.35 (m, 1H), 1.85-1.54 (m, 2H), 1.02-1.00 (d, 3H, J=7.2 Hz).

**Example 23: Preparation of Compound (23) ((4-((((2-(3-(2-(4-(((3R,4R)-1-(2-cyanoacetyl)-4-methylpiperidin-3-yl) (methyl)amino)-N-methyl-7H-pyrrolo[2,3-d]pyrimidine-7-carboxamido)ethyl)-3-methylureido)-4-(dimethylamino)phenyl)carbamoyl)oxy)methyl)phenyl)boronic acid)**

[Reaction Formula 23]

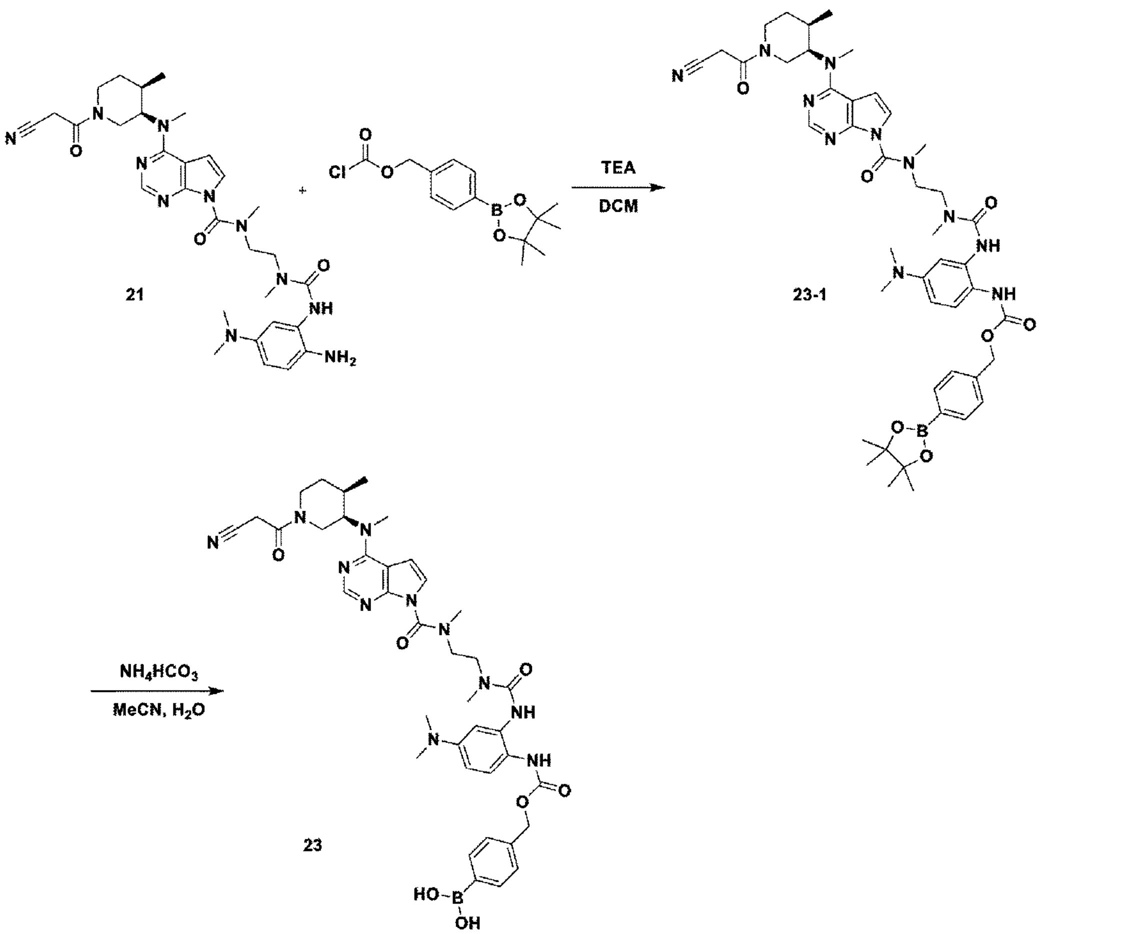


Step (1): Preparation of Compound (23-1)

603 mg of Compound (21) (1 mmol, 1 equiv) and 296 mg of 4-(4,4,5,5-tetramethyl-1,3,2-dioxaborolan-2-yl) benzyl carbonochloridate (1 mmol, 1 equiv) were dissolved in 40 mL of dichloromethane. 303 mg of TEA (3 mmol, 3 equiv) was introduced thereto, and the mixture solution was stirred for 16 hours at room temperature. After the reaction was finished, water and ethyl acetate were introduced thereto for extraction. The organic layer was separated, then dried with magnesium sulfate, and filtered. The remaining filtrate was concentrated under reduced pressure. The concentrated solution was purified by silica gel column chromatography (dichloromethane:methanol=20:1) to obtain Compound (23-1), a target compound. (Yield 46%)

Step (2): Preparation of Compound (23)

400 mg of Compound (23-1) (0.46 mmol, 1 equiv) was dissolved in 20 mL of MeCN. 10 mg of $NH_4HCO_3$ (0.79 mmol, 1.7 equiv) dissolved in 10 mL of water was introduced to the mixture solution, and the mixture solution was stirred for 16 hours. After the reaction was finished, water and ethyl acetate were introduced thereto for extraction. The organic layer was separated, then dried with magnesium sulfate, and filtered. The remaining filtrate was concentrated under reduced pressure. The concentrated solution was purified by reverse-phase prep-HPLC to obtain Compound (23), a target compound. (Yield 30%)

$^1$H-NMR (400 MHz, DMSO-$d_6$) δ 8.72-8.72 (m, 1H), 8.20-8.19 (m, 1H), 8.06 (s, 2H), 7.89-7.74 (m, 3H), 7.32-7.13 (m, 4H), 6.84-6.84 (m, 1H), 6.70-6.69 (m, 1H), 6.50-6.48 (d, 1H, J=9.2 Hz), 5.11 5.02 (m, 2H), 4.86-4.81 (m, 1H), 4.17-3.86 (m, 3H), 3.76-3.55 (m, 4H), 3.42-3.39 (m, 2H), 3.31-2.87 (m, 7H), 2.84 (s, 8H), 2.80-2.59 (m, 1H), 2.39-2.32 (m, 1H), 1.83-1.55 (m, 2H), 1.02-1.00 (d, 3H, J=7.2 Hz).

**Example 24: Preparation of Compound (24) ((4-((((6-(3-(2-(4-(((3R,4R)-1-(2-cyanoacetyl)-4-methylpiperidin-3-yl)(methyl)amino)-N-methyl-7H-pyrrolo[2,3-d]pyrimidine-7-carboxamido)ethyl)-3-methylureido)-1,3-dimethyl-2-oxo-2,3-dihydro-1H-benzo[d]imidazol-5-yl)carbamoyl)oxy)methyl)phenyl)boronic acid)**

[Reaction Formula 24]

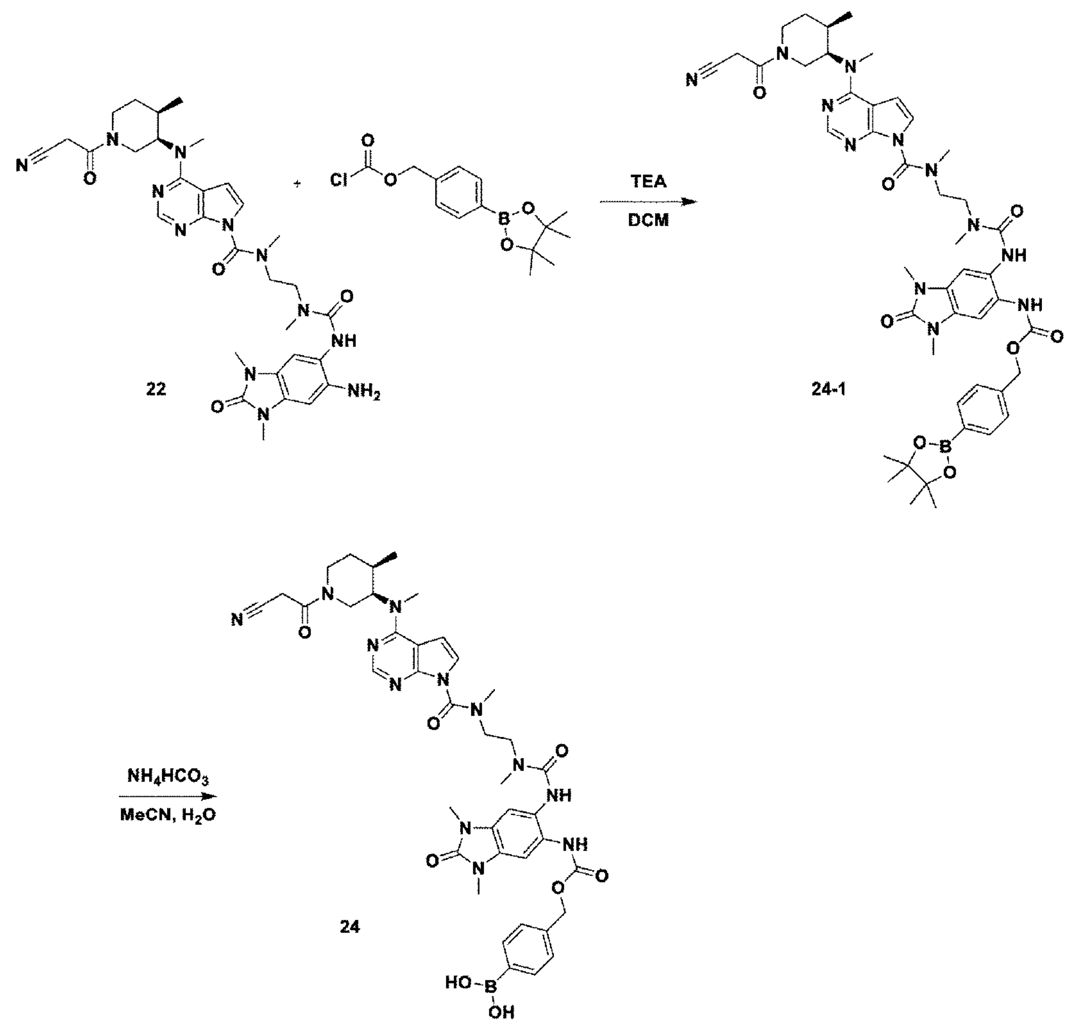


Step (1): Preparation of Compound (24-1)

Compound (24-1), a target compound, was obtained by repeating the same procedure as in Step (1) of Example

23 using Compound (22) instead of Compound (21). (Yield 29%)

Step (2): Preparation of Compound (24)

Compound (24), a target compound, was obtained by repeating the same procedure as in Step (2) of Example 23 using Compound (24-1) instead of Compound (23-1). (Yield 30%)
$^1$H-NMR (400 MHz, DMSO-$d_6$) δ 8.92-8.92 (m, 1H), 8.20 -8.19 (m, 1H), 8.07 (s, 2H), 8.00-7.63 (m, 3H), 7.38-7.22 (m, 4H), 7.10 (s, 1H), 5.16-5.05 (m, 2H), 4.87-4.76 (m, 1H), 4.17-3.90 (m, 3H), 3.76-3.57 (m, 4H), 3.44-3.38 (m, 2H), 3.29-3.20 (m, 10H), 3.09-2.99 (m, 3H), 2.86 -2.82 (m, 2H), 2.67-2.61 (m, 1H), 2.39-2.33 (m, 3H), 1.85-1.51 (m, 2H), 0.99-0.98 (m, 3H).

**Example 25: Preparation of Compound (25) (4-(4,4,5,5-tetramethyl-1,3,2-dioxaborolan-2-yl)benzyl (2-(4-(((3R,4R)-1-(2-cyanoacetyl)-4-methylpiperidin-3-yl) (methyl)amino)-7H-pyrrolo[2,3-d]pyrimidine-7-carboxamido) phenyl) carbamate)**

[Reaction Formula 25]

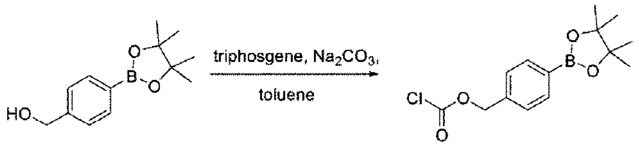


25-1 25-2

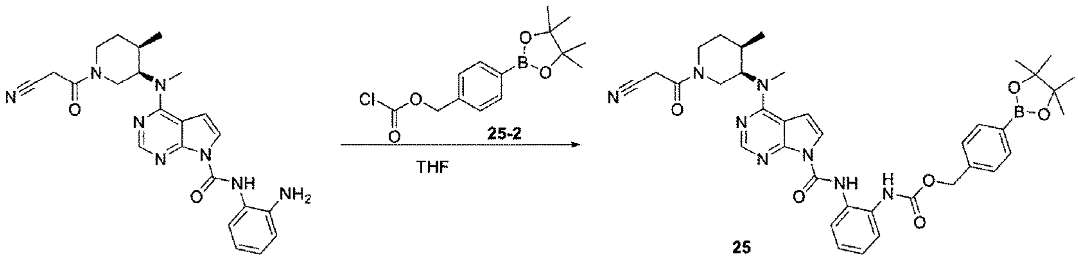


1 25

Step (1): Preparation of Compound (25-2)

After dissolving 4.6 g of $Na_2CO_3$ (43 mmol, 10 equiv) in 5 mL of toluene, the mixture solution was cooled to 0°C. 2.5 g of triphosgene (8.4 mmol, 1.95 equiv) was introduced thereto, and then the mixture solution was stirred for 30 minutes. 1 g of Compound (25-1) (4.3 mmol, 1 equiv) dissolved in 5 mL of toluene was added dropwise to the reaction material, and then the mixture solution was warmed to room temperature and stirred for 16 hours. After the reaction was finished, the reaction solution was filtered, and the filtrate was concentrated under reduced pressure. The concentrated solution was purified by silica gel column chromatography (ethyl acetate:petroleum ether=1:20) to obtain Compound (25-2), a target compound. (Yield 28%)

Step (2): Preparation of Compound (25)

To a solution in which 200 mg of Compound (1) (0.45 mmol, 1 equiv) was dissolved in 5 mL of THF, Compound (25-2) (160 mg, 0.54 mmol, 1.2 equiv) was introduced. The mixture solution was stirred for 2 hours at room temperature. After the reaction was finished, the reaction solution was concentrated under reduced pressure. The concentrated solution was purified by silica gel column chromatography to obtain Compound (25), a target compound. (Yield 22%)
$^1$H-NMR (400 MHz, $CDCl_3$) δ 11.94-11.75 (m, 1H), 8.24-8.23 (m, 1H), 7.71-7.67(m, 5H), 7.30-7.19 (m, 3H), 7.17-7.15 (m, 2H), 6.58-6.55 (m, 1H), 5.14 (s, 2H), 5.07 (br, 1H), 4.03-3.72 (m, 2H), 3.56-3.42 (m, 4H), 3.33-3.31 (m, 3H), 2.48-2.36 (m, 1H), 1.94-1.52 (m, 2H), 1.27 (s, 12H), 1.01 (d, 3H, J=6.8 Hz).

**Example 26: Preparation of Compound (26) (4-(4,4,5,5-tetramethyl-1,3,2-dioxaborolan-2-yl)benzyl (2-(4-(((3R,4R)-1-(2-cyanoacetyl)-4-methylpiperidin-3-yl) (methyl)amino)-7H-pyrrolo[2,3-d]pyrimidine-7-carboxamido)-4-methylphenyl)carbamate)**

[Reaction Formula 26]

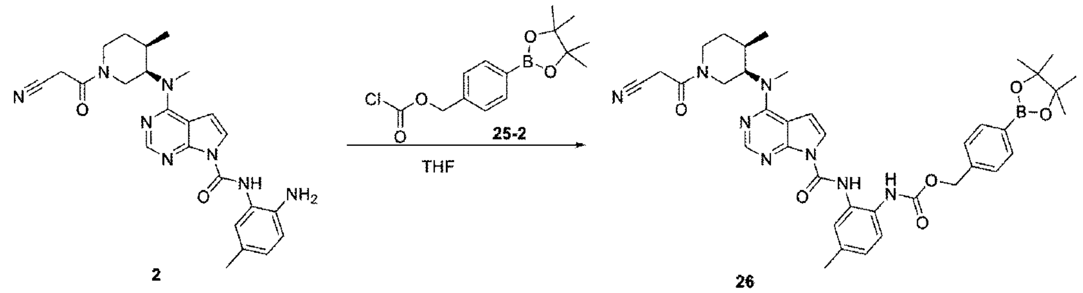


2

26

Step (1): Preparation of Compound (26)

Compound (26), a target compound, was obtained by repeating the same procedure as in Step (2) of Example 25 using Compound (2) instead of Compound (1). (Yield 18%)

$^1$H-NMR (400 MHz, $CDCl_3$) δ 11.94-11.81 (m, 1H), 8.30-8.29 (m, 1H), 7.7-7.36 (m, 7H), 7.03-7.02 (m, 1H), 6.63-6.59 (m, 1H), 5.19 (s, 2H), 5.16-5.14 (m, 1H), 4.09-3.78 (m, 2H), 3.62-3.46 (m, 4H), 3.38-3.37 (m, 3H), 2.53-2.51 (m, 1H), 2.37-2.36 (m, 3H), 2.00-1.79 (m, 2H), 1.33 (s, 12H), 1.08-1.05 (m, 3H).

**Example 27: Preparation of Compound (27) (4-(4,4,5,5-tetramethyl-1,3,2-dioxaborolan-2-yl)benzyl (2-(4-(((3R,4R)-1-(2-cyanoacetyl)-4-methylpiperidin-3-yl) (methyl)amino)-7H-pyrrolo[2,3-d]pyrimidine-7-carboxamido)-4-methoxyphenyl)carbamate)**

[Reaction Formula 27]

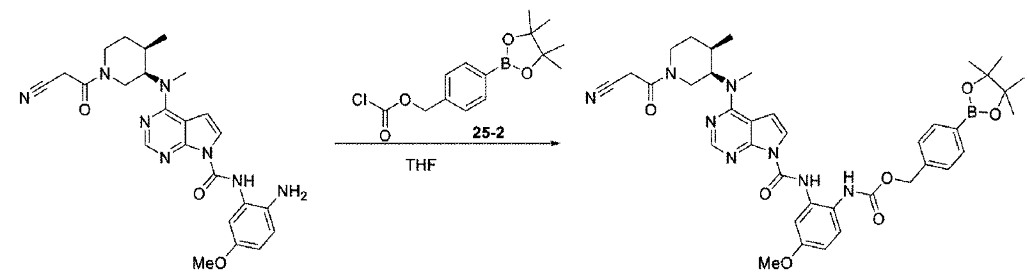


7

27

Step (1): Preparation of Compound (27)

Compound (27), a target compound, was obtained by repeating the same procedure as in Step (2) of Example 25 using Compound (7) instead of Compound (1). (Yield 16%)

$^1$H-NMR (400 MHz, $CDCl_3$) δ 12.30-11.96 (m, 1H), 8.16 (s, 1H), 7.73-7.57 (m, 6H), 7.19-7.05 (m, 1H), 6.82-6.52 (m, 2H), 5.13-5.03 (m, 3H), 4.01-3.97 (m, 2H), 3.76 (s, 3H), 3.53-3.42 (m, 4H), 2.29 (s, 3H), 2.43-2.34 (m, 1H), 1.94-1.57 (m, 2H), 1.26 (s, 12H), 1.01-0.99 (m, 3H).

**Example 28: Preparation of Compound (28) (4-(4,4,5,5-tetramethyl-1,3,2-dioxaborolan-2-yl)benzyl (6-(4-(((3R,4R)-1-(2-cyanoacetyl)-4-methylpiperidin-3-yl) (methyl)amino)-7H-pyrrolo[2,3-d]pyrimidine-7-carboxamido)-1,3-dimethyl-2-oxo-2,3-dihydro-1H-benzo[d]imidazol-5-yl)carbamate)**

[Reaction Formula 28]

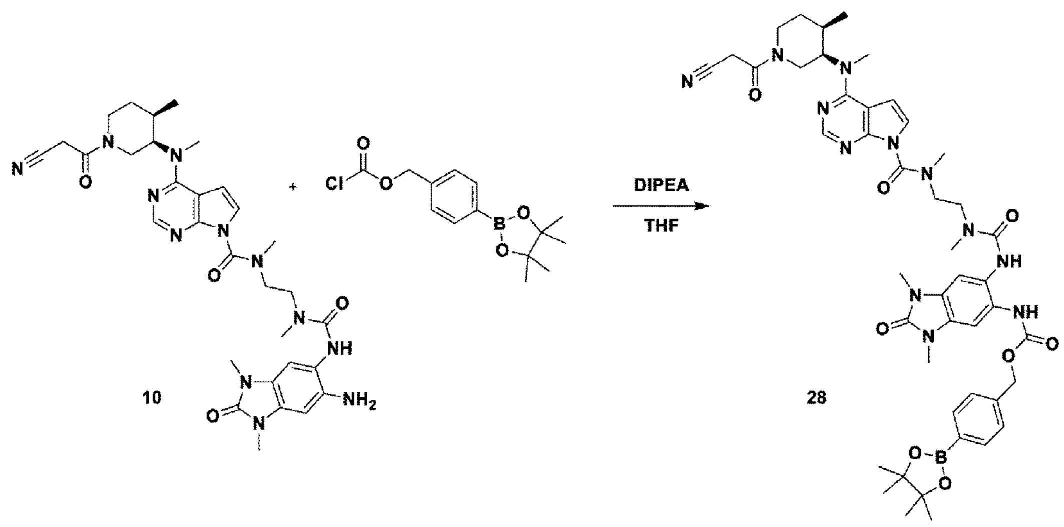

10 28

Step (1): Preparation of Compound (28)

133 mg of Compound (10) (0.25 mmol, 1 equiv) and 149 mg of 4-(4,4,5,5-tetramethyl-1,3,2-dioxaborolan-2-yl) benzyl carbonochloridate (0.5 mmol, 2 equiv) were dissolved in 4 mL of THF. 85 μL of DIPEA (0.5 mmol, 2 equiv) was introduced thereto, and the mixture solution was stirred for 16 hours at 40°C. After the reaction was finished, the mixture solution was cooled to room temperature, and then concentrated under reduced pressure. The concentrated solution was recrystallized using dichloromethane and ether. Solid obtained by filtering the result was purified by silica gel column chromatography (dichloromethane:methanol=20:1) to obtain Compound (28), a target compound. (Yield 54%)
$^1$H-NMR (300 MHz, DMSO-$d_6$) δ 12.02-12.02 (m, 1H), 9.37-9.36 (m, 1H), 8.23 (s, 1H), 7.88-7.38 (m, 5H), 7.16 (s, 1H), 6.91-6.84 (m, 1H), 5.13 (s, 1H), 4.91-4.78 (m, 2H), 4.55-4.54 (m, 1H), 4.20-3.72 (m, 5H), 2.73-2.69 (m, 2H), 2.20-2.09 (m, 3H), 1.97-1.85 (m, 3H), 1.74-1.47 (m, 3H), 1.29-1.23 (m, 10H), 1.16-1.14 (m, 1H), 1.07-1.01 (m, 3H), 0.85-0.81 (t, 1H, J=6 Hz).

**Example 29: Preparation of Compound (29) (N-(2-aminophenyl)-8-((3R,4S)-4-ethyl-1-((2,2,2-trifluoroethyl)carbamoyl)pyrrolidin-3-yl)-3H-imidazo[1,2-a]pyrrolo[2,3-e]pyrazine-3-carboxamide)**

[Reaction Formula 29]

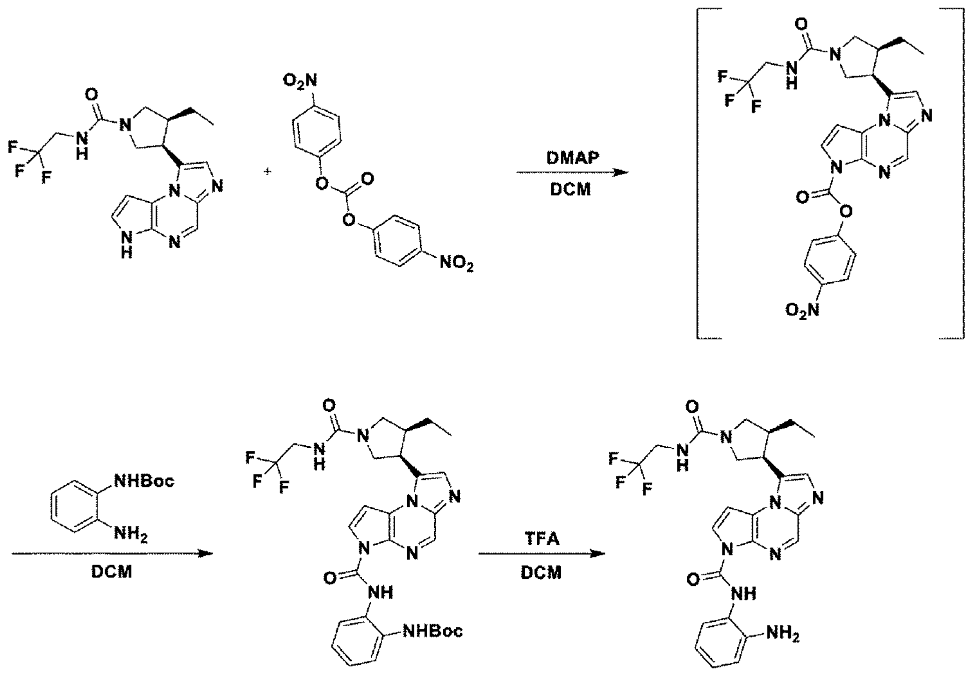


29-1 29

Step (1): Preparation of Compound (29-1)

200 mg of upadacitinib (0.53 mmol, 1 equiv), 216 mg of bis (4-nitrophenyl) carbonate (0.71 mmol, 1.35 equiv) and DMAP (0.74 mmol, 1.4 equiv) were introduced to 5 mL of dichloromethane, and then the mixture solution was stirred for 1 day at room temperature. To the mixture solution, 131 mg of tert-butyl (2-aminophenyl)carbamate (0.63 mmol, 1.2 equiv) dissolved in 3 mL of dichloromethane was added dropwise, and the mixture solution was stirred for 16 hours at room temperature. The concentrated solution was purified by silica gel column chromatography (dichloromethane:methanol=10:1) to obtain Compound (29-1), a target compound. (Yield 63%)

Step (2): Preparation of Compound (29)

After dissolving 203 mg of Compound (29-1) (0.33 mmol, 1 equiv) in 16 mL of dichloromethane, 4.1 mL of trifluoroacetic acid (20 V) was introduced to the mixture solution, and then the mixture solution was stirred for 16 hours at room temperature. After the reaction was finished, the reaction solution was cooled to 0°C to 5°C, and neutralized by adding an aqueous $NaHCO_3$ solution dropwise thereto. Dichloromethane was introduced thereto to separate the organic layer, and then the organic layer was dried with magnesium sulfate and filtered. The remaining filtrate was concentrated under reduced pressure. The concentrated solution was purified by silica gel column chromatography (dichloromethane:methanol=10:1) to obtain Compound (29), a target compound. (Yield 81%)
$^1$H-NMR (300 MHz, DMSO-$d_6$) $\delta$ 10.76 (s, 1H), 8.88 (s, 1H), 8.09 -8.08 (d, 1H, J=3 Hz), 7.67 (s, 1H), 7.46-7.40 (m, 2H), 7.02-6.98 (m, 2H), 6.85-6.83 (dd, 1H, J=6 Hz, 0.9 Hz), 6.67-6.64 (td, 1H, J=6 Hz, 0.9 Hz), 5.14 (s, 2H), 4.42-4.41 (m, 1H), 3.89-3.80 (m, 4H), 3.72-3.69 (m, 1H), 3.27-3.25 (m, 1H), 2.61-2.59 (m, 1H), 1.09-1.05 (m, 1H), 0.82-0.77 (m, 1H), 0.65-0.63 (m, 3H).

**Example 30: Preparation of Compound (30) (N-(2-amino-4-(dimethylamino)phenyl)-8-((3R,4S)-4-ethyl-1-((2,2,2-trifluoroethyl)carbamoyl)pyrrolidin-3-yl)-3H-imidazo[1,2-a]pyrrolo[2,3-e]pyrazine-3-carboxamide)**

[Reaction Formula 30]

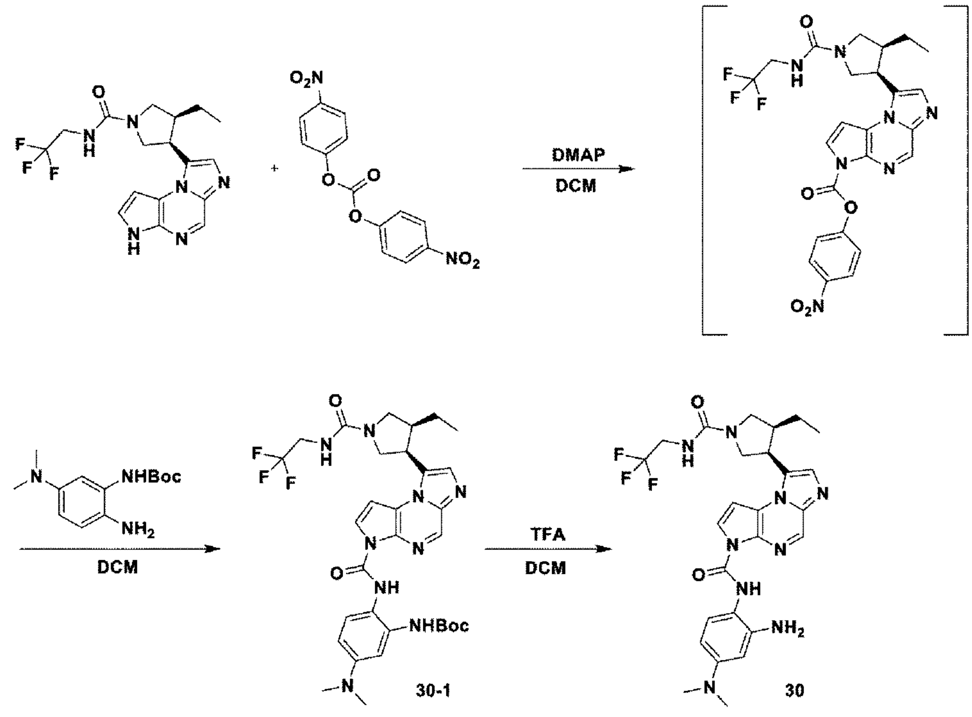


Step (1): Preparation of Compound (30-1)

Compound (30-1), a target compound, was obtained by repeating the same procedure as in Step (1) of Example 29 using tert-butyl (2-amino-5-(dimethylamino)phenyl)carbamate instead of tert-butyl (2-aminophenyl)carbamate. (Yield 52%)

Step (2): Preparation of Compound (30)

Compound (30), a target compound, was obtained by repeating the same procedure as in Step (2) of Example 29 using Compound (30-1) instead of Compound (29-1). (Yield 91%)
$^1$H-NMR (300 MHz, DMSO-$d_6$) $\delta$ 10.46 (s, 1H), 8.87 (s, 1H), 8.06-8.06 (d, 1H, J=1.8 Hz), 7.66 (s, 1H), 7.38-7.37 (d, 1H, J=2.1 Hz), 7.11-7.10 (d, 1H, J=4.2 Hz), 7.00-6.98 (t, 1H, J=3 Hz), 6.19-6 .19 (d, 1H, J=1.2 Hz), 6.08-6.07 (dd, 1H, J=3 Hz, 1.5 Hz), 4.96 (s, 2H), 4.42-4.41 (m, 1H), 3.91-3.79 (m, 4H), 3.72-3.69 (m, 1H), 3.27-3.24 (m, 1H), 2.85 (s, 6H). 2.62-2.61 (m, 1H), 1.09-1.03 (m, 1H), 0.82-0.77 (m, 1H), 0.65-0.63 (t, 3H, J=6 Hz).

**Example 31: Preparation of Compound (31) (N-(6-amino-1,3-dimethyl-2-oxo-2,3-dihydro-1H-benzo[d]imidazol-5-yl)-8-((3R,4S)-4-ethyl-1-((2,2,2-trifluoroethyl)carbamoyl)pyrrolidin-3-yl)-3H-imidazo[1,2-a]pyrrolo[2,3-e]pyrazine-3-carboxamide)**

[Reaction Formula 31]

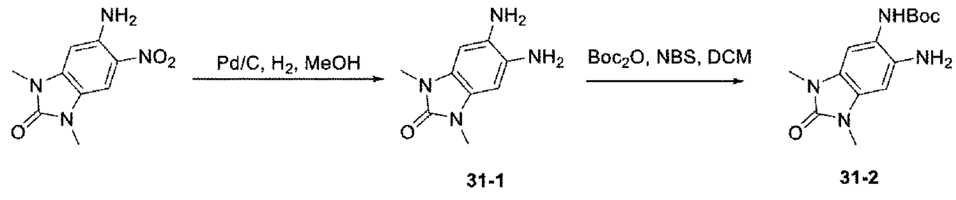


31-1

31-2

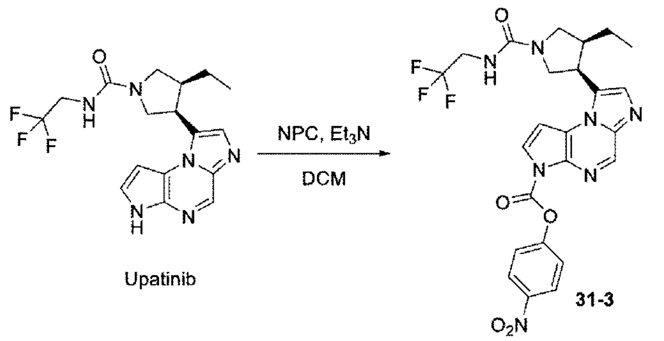


Upatinib

31-3

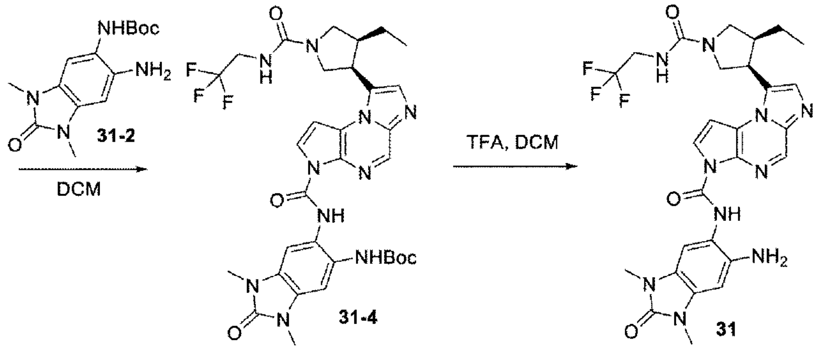


31-2

31-4

31

Step (1): Preparation of Compound (31-1)

To a solution in which 1.5 g of 5-amino-1,3-dimethyl-6-nitro-1H-benzo[d]imidazol-2(3H)-one (6.75 mmol, 1 equiv) was dissolved in 150 mL of methanol, 750 mg of 10% Pd/C (10 wt.) was introduced, and then the mixture solution was stirred for 18 hours under hydrogen gas at atmospheric pressure. The mixture solution was warmed to room temperature, and stirred for 18 hours. After the reaction was finished, the reaction solution was filtered through Celite, and washed with 50 mL of methanol. The filtrate was concentrated under reduced pressure to obtain Compound (31-1), a target compound.

Step (2): Preparation of Compound (31-2)

After dissolving 1.3 g of Compound (31-1) (6.75 mmol, 1 equiv) and $(Boc)_2$ (1.77 g, 8.1 mmol, 1.2 equiv) in 100 mL of dichloromethane, the mixture solution was cooled to 0°C. 120 mg of NBS (0.675 mmol, 0.1 equiv) was introduced thereto, and then the mixture solution was warmed to room temperature and stirred for 18 hours. After the reaction was finished, the reaction solution was filtered, and the filtrate was concentrated under reduced pressure. The concentrated solution was purified by silica gel column chromatography (100% ethyl acetate) to obtain Compound (31-2), a target compound. (2 steps yield 25%)

Step (3): Preparation of Compound (31-3)

To a solution in which 570 mg of upadacitinib (1.5 mmol, 1 equiv) and 500 mg of NPC (1.65 mmol, 1.1 equiv) were dissolved in 40 mL of dichloromethane, 0.52 mL of triethylamine (3.75 mmol, 2.5 equiv) was introduced, and then the mixture solution was stirred under reflux for 18 hours. After the reaction was finished, the reaction solution was cooled to room temperature, and Compound (31-3), a target compound, was obtained without a purification process.

Step (4): Preparation of Compound (31-4)

After introducing Compound (31-2) (450 mg, 1.5 mmol, 1 equiv) to the reaction solution of Step (3), the mixture solution was stirred for 18 hours. After the reaction was finished, the reaction solution was concentrated under reduced pressure. The concentrated solution was purified by silica gel column chromatography to obtain Compound (31-4), a target compound. (Yield 37%)

Step (5): Preparation of Compound (31)

To a solution in which 735 mg of Compound (31-4) (1.05 mmol, 1 equiv) was dissolved in 30 mL of dichloromethane, 6 mL of TFA was introduced, and the mixture solution was stirred for 2 hours at room temperature. After the reaction was finished, the reaction solution was concentrated under reduced pressure, and basified by introducing an aqueous $Na_2CO_3$ solution thereto. Dichloromethane was introduced thereto for extraction. The organic layer was separated, then dried with magnesium sulfate, and filtered. The remaining filtrate was concentrated under reduced pressure. The concentrated solution was purified by silica gel column chromatography to obtain Compound (31), a target compound. (Yield 85%)
$^1$H-NMR (400 MHz, DMSO-$d_6$) δ 10.75 (s, 1H), 8.89 (s, 1H), 8.09 (d, 1H, J=4.0 Hz), 7.68 (s, 1H), 7.41 (d, 1H, J=4.0 Hz), 7.24 (s, 1H), 7.01 (t, 1H, J=6.0 Hz), 4.93 (s, 2H), 4.44-4.40 (m, 1H), 3.90-3.79 (m, 4H), 3.73-3.69 (m, 1H), 3.29-3.25 (m, 7H), 2.60-2.57 (m, 1H), 1.11-1.05 (m, 1H), 0.85-0.79 (m, 1H), 0.64 (t, 3H, J=7.2 Hz).

**Example 32: Preparation of Compound (32) ((4-((((6-(8-((3R,4S)-4-ethyl-1-((2,2,2-trifluoroethyl)carbamoyl)pyrrolidin-3-yl)-3H-imidazo[1,2-a]pyrrolo[2,3-e]pyrazine-3-carboxamido)-1,3-dimethyl-2-oxo-2,3-dihydro-1H-benzo[d]imidazol-5-yl)carbamoyl)oxy)methyl)phenyl)boronic acid)**

[Reaction Formula 32]

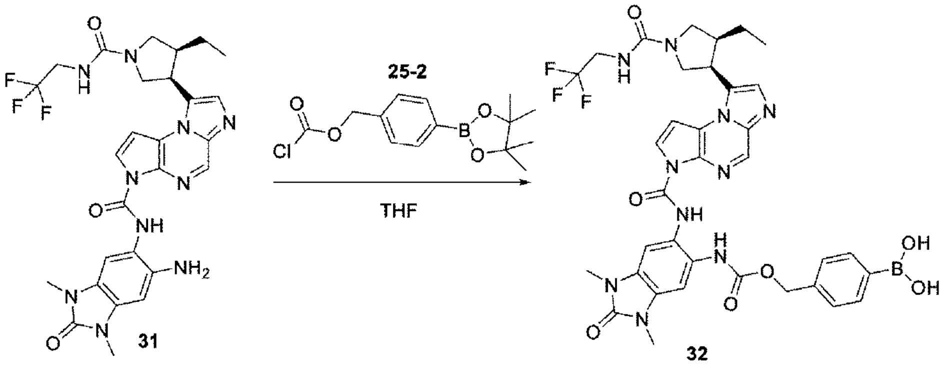


31

32

Step (1): Preparation of Compound (32)

Compound (32), a target compound, was obtained by repeating the same procedure as in Step (2) of Example 25 using Compound (31) instead of Compound (1). (Yield 28%)
$^1$H-NMR (400 MHz, DMSO-$d_6$) δ 11.57 (s, 1H), 9.44 (s, 1H), 8.59-8.57 (m, 1H), 8.10-8.00 (m, 2H), 7.90-7.69 (m, 3H), 7.51-6.84 (m, 6H), 5.45 (s, 1H), 4.41-4.34 (m, 1H), 3.88-3.66 (m, 5H), 3.36-3.25 (m, 9H), 2.60-2.54 (m, 1H), 1.13-1.05 (m, 1H), 0.84-0.77 (m, 1H), 0.64 (t, 1H, J=7.2 Hz).

**Example 33: Preparation of Compound (33) (2-(9-fluoro-11,17-dihydroxy-10,13,16-trimethyl-3-oxo-6,7,8,9,10,11,12,13,14,15,16,17-dodecahydro-3H-cyclopenta[a]phenanthren-17-yl)-2-oxoethyl (2-aminophenyl) carbamate)**

[Reaction Formula 33]

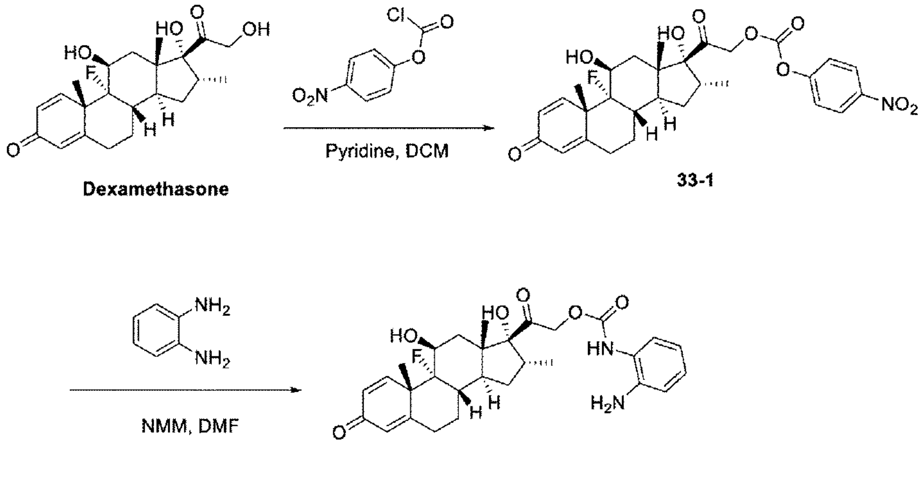


Dexamethasone

33-1

33

Step (1): Preparation of Compound (33-1)

After dissolving 5.0 g of dexamethasone (12.76 mmol, 1 equiv) and pyridine (3.09 mL, 38.27 mmol, 3 equiv) in 40 mL of dichloromethane, the mixture solution was cooled to 0°C. A solution in which 3.33 g of 4-nitrophenyl carbonochloridate (16.58 mmol, 1.3 equiv) was dissolved in 10 mL of dichloromethane was added dropwise thereto, and the mixture solution was warmed to room temperature and stirred for 1 hour. The mixture solution was warmed to room temperature, and stirred for 18 hours. After the reaction was finished, the reaction solution was stirred after introducing water thereto, and then the organic layer was separated. The separated organic layer was washed with 80 mL of 0.1 M HCl and a saturated aqueous $NaHCO_3$ solution in this order, then dried with magnesium sulfate, and filtered. The remaining filtrate was concentrated under reduced pressure. The concentrated solution was purified by silica gel column chromatography (ethyl acetate:petroleum ether=1:1) to obtain Compound (33-1), a target compound. (Yield 87%)

Step (2): Preparation of Compound (33)

After introducing 500 mg of Compound (33-1) (0.9 mmol, 1 equiv), 0.59 mL of NMM (5.39 mmol, 6 equiv) and 291 mg of o-phenylenediamine (2.69 mmol, 3 equiv) to 8 mL of DMF, the mixture solution was stirred for 3 days at room temperature. After the reaction was finished, water and ethyl acetate were introduced to the reaction solution for extraction. The organic layer was separated, then dried with magnesium sulfate, and filtered. The remaining filtrate was concentrated under reduced pressure. The concentrated solution was purified by silica gel column chromatography (ethyl acetate:petroleum ether=3:1) to obtain Compound (33), a target compound. (Yield 54%)
$^1$H-NMR (400 MHz, DMSO-$d_6$) δ 8.83 (s, 1H), 7.29 (d, 1H, J=10.4 Hz), 7.16 (d, 1H, J=6.0 Hz), 6.88 (t, 1H, J=7.2 Hz), 6.70 (dd, 1H, J=7.6, 1.2 Hz), 6.53 (t, 1H, J=7.2 Hz), 6.23 (d, 1H, J=8.0 Hz), 6.01 (s, 1H), 5.42 (d, 1H, J=4.0 Hz), 5.14 (s, 1H), 5.04-4.79 (m, 4H), 4.16 (br, 1H), 2.91 (br, 1H), 2.65-2.63 (m, 1H), 2.41-2.31 (m, 2H), 2.20-2.15 (m, 2H), 1.91-1.77 (m, 1H), 1.69-1.58 (m, 2H), 1.49 (s, 3H), 1.41-1.36 (m, 1H), 1.11-1.02 (m, 1H), 0.91 (s, 3H), 0.80 (d, 3H, J=7.2 Hz).

**Example 34: Preparation of Compound (34) (2-(9-fluoro-11,17-dihydroxy-10,13,16-trimethyl-3-oxo-6,7,8,9,10,11,12,13,14,15,16,17-dodecahydro-3H-cyclopenta[a]phenanthren-17-yl)-2-oxoethyl (2-amino-4-methylphenyl) carbamate)**

[Reaction Formula 34]

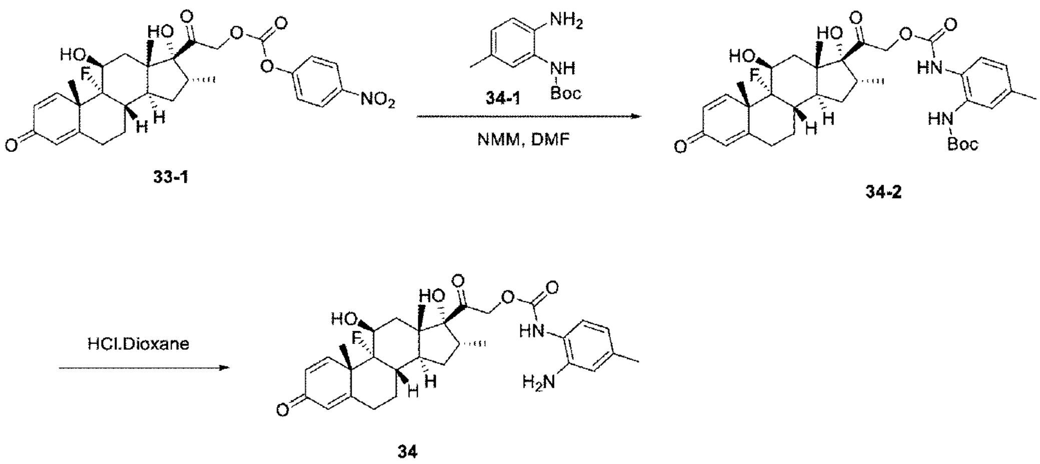


33-1 34-2 34

Step (1): Preparation of Compound (34-2)

Compound (34-2), a target compound, was obtained by repeating the same procedure as in Step (2) of Example 33 using Compound (34-1) instead of o-phenylenediamine. (Yield 30%)

Step (2): Preparation of Compound (34)

Compound (34), a target compound, was obtained by repeating the same procedure as in Step (2) of Example 1 using Compound (34-2) instead of Compound (1-1). (Yield 14%)
$^1$H-NMR (400 MHz, DMSO-$d_6$) δ 8.75 (s, 1H), 7.29 (d, 1H, J=10.0 Hz), 7.02 (s, 1H,), 6.51 (s, 1H), 6.35 (d, 1H, J=7.6 Hz), 6.23 (d, 1H, J=10.0 Hz), 6.01 (s, 1H), 5.42 (d, 1H, J=4.4 Hz), 5.15 (s, 1H), 5.03-4.78 (m, 4H), 4.16 (br, 1H), 2.91 (br, 1H), 2.67-2.58 (m, 1H), 2.34-2.31 (m, 2H), 2.19-2.12 (m, 5H), 1.91-1.79 (m, 1H), 1.68-1.58 (m, 2H), 1.49 (s, 3H), 1.37-1.34 (m, 1H), 1.10-1.07 (m, 1H), 0.90 (s, 3H), 0.81-0.79 (m, 3H).

**Example 35: Preparation of Compound (35) ((1R,2R,E)-1-((2S,5S,11S,14S,17S,20S,23R,26S,29S,32S)-5-ethyl-11,17,26,29-tetraisobutyl-14,32-diisopropyl-1,7,10,16,20,23,25,28,31-nonamethyl-3,6,9,12,15,18,21,24,27,30,33-undecaoxo-1,4,7,10,13,16,19,22,25,28,31-undecaazacyclotritriacontan-2-yl)-2-methylhex-4-en-1-yl (2-aminophenyl)carbamate)**

[Reaction Formula 35]

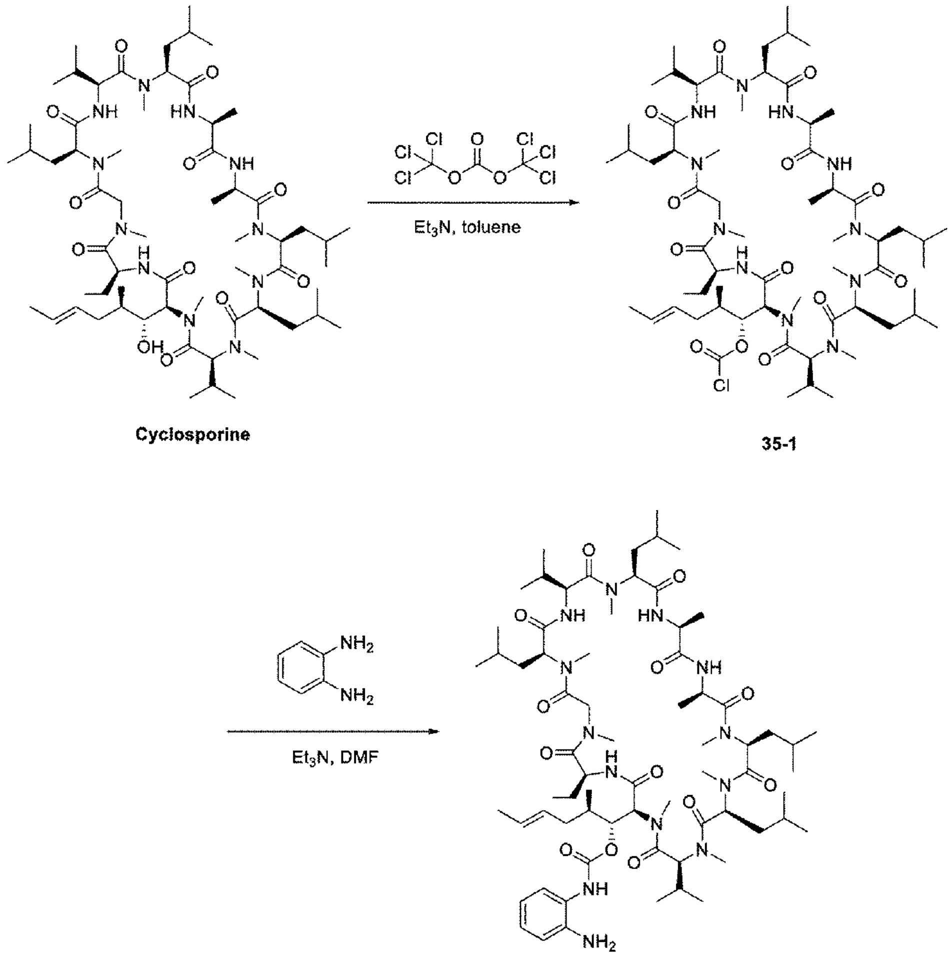


Cyclosporine

35-1

35

Step (1): Preparation of Compound (35-1)

To a solution in which 120 mg of cyclosporine (0.1 mmol, 1 equiv) was dissolved in 15 mL of toluene, triphosgene (2.97 g, 10 mmol, 100 equiv) and triethylamine (10 mg, 0.1 mmol, 1 equiv) were introduced, and then the mixture solution was stirred for 16 hours at 40°C. After the reaction was finished, the reaction solution was concentrated under reduced pressure to obtain Compound (35-1), a target compound.

Step (2): Preparation of Compound (35)

After introducing Compound 35-1 (120 mg, 0.1 mmol, 1 equiv), o-phenylenediamine (108 mg, 1 mmol, 10 equiv) and triethylamine (100 mg, 1.0 mmol, 10 equiv) to 5 mL of DMF, the mixture solution was stirred for 16 hours at 20°C. After the reaction was finished, the reaction solution was concentrated under reduced pressure. The concentrated solution was purified by silica gel column chromatography to obtain Compound (35), a target compound. (Yield 10%) FIG. 3 shows NMR data of Compound (35) prepared in Example 35.

MS (m/z): 1337.7 (M+H).

**Comparative Example (Negative Control Group): Preparation of Compound (36)**

[Reaction Formula 36]

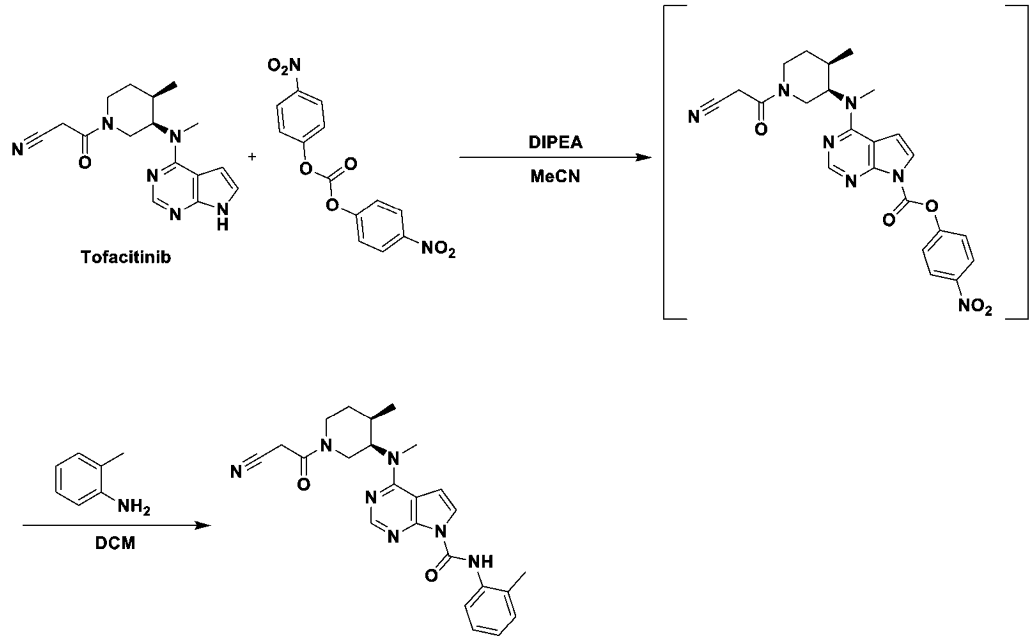


Tofacitinib

36

Step (1): Preparation of Compound 36

4-(((3R,4R)-1-(2-cyanoacetyl)-4-methylpiperidin-3-yl)(methyl)amino)-N-(o-tolyl)-7H-pyrrolo[2,3-d]pyrimidine-7-carboxamide, a target compound, was obtained by repeating the same procedure as in Step (1) of Example 1 using o-toluidine instead of tert-butyl (2-aminophenyl)carbamate. (Yield 46%)
$^1$H-NMR (300 MHz, DMSO-$d_6$) $\delta$ 11.85-11.83 (m, 1H), 8.43-8.41 (d, 1H, J=6 Hz), 8.14-8.05 (m, 2H), 7.78-7.77 (m, 1H), 7.33-7.25 (m, 2H), 7.13-7.08 (t, 1H, J=6Hz), 6.96-6.92 (m, 1H), 4.92 (s, 1H), 4.20-3.67 (m, 5H), 3.57-3.37 (m, 2H), 2.44-2.39 (m, 4H), 1.91-1.58 (m, 3H), 1.04-1.01 (d, 3H, J=9 Hz).

**Experimental Example 1: Verification of Nitrogen Monoxide Scavenging Activity of Pharmaceutical Composition Sensitive to Nitrogen Monoxide (DAF-2 Assay)**

NO scavenging activity of the pharmaceutical composition sensitive to nitrogen monoxide including the compound according to each of Examples 1 to 22, 29 to 31, and 33 to 35 was evaluated through a DAF-2 assay. An NO donor SNAP (S-nitroso-N-acetyl-D,L-penicillamine) capable of releasing NO under a hydrophilic condition was used to treat the composition, and then the composition was reacted with DAF-2 that exhibits fluorescence upon binding to NO to verify the amount of NO.
**[0195]** Specifically, a reagent stock solution was made using DMSO (dimethyl sulfoxide) in order to prepare 5 mM of DAF-2, 100 mM of SNAP and 5 mM of the compound synthesized in each of the Examples, and for the reaction, the solution was added to a PBS (phosphate buffered saline) solution so that DAF-2, SNAP and the compound had final concentrations of 10 $\mu$M, 100 $\mu$M and the concentration of Table 1, respectively, followed by a reaction for 3 hours in a 37°C incubator. In order to relatively compare the amount of remaining NO, NO scavenging activity was calculated by the following Equation 1, and the results are shown in the following Table 10 (N/T: Not Tested).

NO scavenging activity (%) = 100 - [(fluorescence value when adding compound of Example - background fluorescence value)/(fluorescence value in the absence of any added substance-background fluorescence value)] $\times$ 100     [Equation 1]

[Table 10]

| Exa mpl e | Concen tratio n (μM) | NO Scav engi ng Acti vity (%) (Mea n ±SD ) | Exa mpl e | Concen tratio n (μM) | NO Scav engi ng Acti vity (%) (Mea n ±SD ) | Exam ple | Concen tratio n (μM) | NO Scave nging Activ ity (%) (Mean ±SD) |
|---|---|---|---|---|---|---|---|---|
| 1 | 1 | 40.5 0±2. 57 | 11 | 1 | 87.2 4±0. 22 | 21 | 1 | 2.57± 1.66 |
| | 5 | 69.0 2±0. 12 | | 5 | 86.3 7±0. 05 | | 5 | 38.13 ±1.01 |
| | 10 | 80.8 ±5.6 9 | | 10 | 88.5 7±0. 97 | | 10 | 66.27 ±0.14 |
| 2 | 1 | 87.5 3±0. 12 | 12 | 1 | 83.1 4±2. 29 | 22 | 1 | 14.83 ±6.51 |
| | 5 | 87.7 6±0. 06 | | 5 | 86.4 4±1. 93 | | 5 | 36.86 ±2.09 |
| | 10 | 89.2 4±0. 04 | | 10 | 88.1 9±1. 75 | | 10 | 48.96 ±3.12 |
| 3 | 1 | 71.4 8±0. 99 | 13 | 1 | 79.0 2±1. 76 | 29 | 1 | 3.68± 7.63 |
| | 5 | 81.6 7±0. 40 | | 5 | 83.5 7±3. 24 | | 5 | 1.71± 4.16 |
| | 10 | 84.2 8±0. 25 | | 10 | 87.8 0±1. 25 | | 10 | 25.18 ±3.14 |
| 4 | 1 | N/T | 14 | 1 | - 3.19 | 30 | 1 | 83.14 ±2.29 |
| | | | | | ±0.9 1 | | | |
| | 5 | 85.5 2±0. 96 | | 5 | - 3.82 ±0.3 5 | | 5 | 86.44 ±1.93 |
| | 10 | 97.8 6±0. 2 | | 10 | 2.82 ±1.5 5 | | 10 | 88.19 ±1.75 |
| 5 | 1 | N/T | 15 | 1 | N/T | 31 | 1 | 79.61 ±3.60 |
| | 5 | 46.0 6±0. 74 | | 5 | 1.48 ±1.8 9 | | 5 | 90.34 ±0.98 |
| | 10 | 97.5 3±0. 14 | | 10 | 4.47 ±0.4 9 | | 10 | 92.21 ±0.98 |
| 6 | 1 | N/T | 16 | 1 | 5.29 ±0.3 7 | 33 | 1 | 2.22± 4.76 |
| | 5 | 47.9 ±0.5 9 | | 5 | N/T | | 5 | 1.72± 4.28 |
| | 10 | 49.1 5±1. 49 | | 10 | 14.4 7±1. 01 | | 10 | 1.52± 10.22 |
| 7 | 1 | N/T | 17 | 1 | 1.04 ±0.6 0 | 34 | 1 | - 0.67± 6.17 |
| | 5 | 87.8 7±0. 37 | | 5 | 0.28 ±0.8 4 | | 5 | - 0.15± 16.19 |
| | 10 | 90.4 9±0. 54 | | 10 | 2.73 ±2.1 1 | | 10 | 0.76± 5.81 |
| 8 | 1 | N/T | 18 | 1 | 32.9 1±0. 89 | 35 | 1 | - 4.87± 8.61 |
| | 5 | 76.8 2±0. 31 | | 5 | 62.3 6±0. 41 | | 5 | - 18.88 ±14.9 3 |
| | 10 | 87.8 2±0. 27 | | 10 | 71.1 9±0. 06 | | 10 | - 32.35 ±12.0 2 |
| 9 | 1 | N/T | 19 | 1 | 9.38 ±1.9 9 | Vita min B12 (Pos itiv e Cont rol Grou p) | 1 | 3.18± 1.03 |
| | 5 | 67.0 1±0. 16 | | 5 | 29.8 3±1. 29 | | 5 | 8.98± 2.46 |
| | 10 | 81.3 2±2. 94 | | 10 | 51.5 ±0.5 2 | | 10 | 12.37 ±3.73 |
| 10 | 1 | N/T | 20 | 1 | 51.7 7±2. 96 | | 100 | 41.09 ±1.57 |
| | 5 | 96.1 9±0. 07 | | 5 | 96.9 9±0. 40 | | 1,000 | 99.48 ±0.95 |
| | 10 | 97.5 8±0. 02 | | 10 | 98.6 6±0. 06 | | | |

As a positive control group, vitamin B12 (cyanocobalamin) known to have NO scavenging activity was used.

Referring to Table 10, the compounds synthesized in the Examples were verified to have higher nitrogen monoxide scavenging activity than vitamin B12, a positive control group.

**Experimental Example 2: Verification of Nitrogen Monoxide Scavenging Activity of Pharmaceutical Composition Sensitive to Nitrogen Monoxide Capable of Removing Reactive Oxygen (DAF-2 Assay)**

The pharmaceutical composition sensitive to nitrogen monoxide including the compound according to each of Examples 23 to 28 and 32 may have scavenging activity for reactive oxygen species (ROS). NO scavenging activity of the pharmaceutical composition was evaluated through a DAF-2 assay. An NO donor SNAP capable of releasing NO and $H_2O_2$ capable of releasing ROS under a hydrophilic condition were used to treat the composition, and then composition was reacted with DAF-2 that exhibits fluorescence upon binding to NO to verify the amount of NO.

**[0199]** Specifically, to a 100 μM solution of SNAP, the compound synthesized in each of the Examples was added at a concentration of the following Table 2, and reacted. Then 10 μM of DAF-2 was added thereto, followed by a reaction for 3 hours in a 37°C incubator. In order to relatively compare the amount of remaining NO, NO scavenging activity was calculated by Equation 1, and the results are shown in the following Table 11.

Additionally, a stock solution of $H_2O_2$ (10 M) was added to the reaction solution so that SNAP and $H_2O_2$ had final concentrations of 100 μM and 1 mM, respectively. Then, the compound synthesized in each of the Examples was added thereto at a concentration of the following Table 11, followed by a reaction in the same manner, and NO scavenging activity was calculated by Equation 1. The results are shown in the following Table 11 (N/T: Not Tested).

[Table 11]

| Exam ple | Concent ration (μM) | NO Scavenging Activity (%) (Mean±SD) | | Exam ple | Concent ration (μM) | NO Scavenging Activity (%) (Mean±SD) | |
|---|---|---|---|---|---|---|---|
| | | Witho ut $H_2O_2$ | With $H_2O_2$ | | | Witho ut $H_2O_2$ | With $H_2O_2$ |
| 23 | 1 | 21.08 ±0.77 | 25.76 ±6.11 | 27 | 1 | N/T | N/T |
| | 5 | 39.33 ±8.29 | 64.84 ±1.94 | | 5 | N/T | N/T |
| | 10 | 52.96 ±6.23 | 83.96 ±0.29 | | 10 | 59.31 ±0.55 | 97.09 ±0.01 |
| 24 | 1 | 16.43 ±4.86 | 34.66 ±3.80 | 28 | 1 | 3.73± 0.57 | 76.23 ±0.94 |
| | 5 | 27.84 ±7.68 | 43.58 ±6.00 | | 5 | 13.25 ±0.69 | 98.01 ±0.04 |
| | 10 | 40.54 ±3.96 | 53.52 ±3.09 | | 10 | 14.18 ±0.62 | 98.34 ±0.05 |
| 25 | 1 | N/T | N/T | 32 | 1 | 6.42± 10.86 | 4.05± 3.57 |
| | 5 | N/T | N/T | | 5 | 6.84± 5.21 | 29.92 ±7.09 |
| | 10 | 42.36 ±1.91 | 89.96 ±0.19 | | 10 | 9.26± 5.51 | 32.06 ±3.99 |
| 26 | 1 | N/T | N/T | | | | |
| | 5 | N/T | N/T | | | | |
| | 10 | 42.98 ±2.51 | 92.96 ±0.04 | | | | |

Referring to Table 11, the compounds of the Examples are compounds including a reactive oxygen scavenger, and it was verified that nitrogen monoxide scavenging activity was improved when $H_2O_2$ was present compared to when $H_2O_2$ was not present.

**Experimental Example** 3: **Analysis of Nitrogen Monoxide Sensitivity of Pharmaceutical Composition Sensitive to Nitrogen Monoxide (HPLC)**

It was checked whether the pharmaceutical composition sensitive to nitrogen monoxide including the compound according to each of the Examples is capable of releasing a low-molecular-weight compound having pharmaceutical activity by separating a nitrogen monoxide scavenger in an environment where nitrogen monoxide is present.

A sample for HPLC analysis was prepared as follows. 28 mg of $NaNO_2$ (0.41 mmol, 16.4 equiv) was dissolved in 0.3 mL of water, and then the mixture solution was cooled to 0°C to 5°C. After introducing 0.2 mL of sulfuric acid (0.1 mmol, 4 equiv) to the mixture solution, 12.2 mg of Compound (4) (0.025 mmol, 1 equiv), which was prepared in Example 4, dissolved in 0.2 mL of acetone was introduced thereto. The mixture solution was stirred for 1 hour at room temperature. After the reaction was finished, water and dichloromethane were introduced to the reaction solution for extraction. The organic layer was separated, then dried with magnesium sulfate, and filtered. The remaining filtrate was concentrated under reduced pressure.

The sample for analysis was subjected to HPLC (Agilent 1200, USA) under the condition shown in the following

Table 12, and the results are shown in FIG. 2.

[Table 12]

| Detector | Ultraviolet Spectrophotometer | | |
|---|---|---|---|
| Detection Wavelength | UV 280 nm | | |
| Column | YMC ODS-A (250 mm×4.6 mm, S-5 μm, 12 nm) | | |
| Column Temperature | 35°C | | |
| Amount of Injection | 10 μL | | |
| Flow Rate | 1 mL/min | | |
| Mobile Phase | Time (Minute) | Water | Acetonitril e |
| | 0 | 80 | 20 |
| | 20 | 0 | 100 |
| | 30 | 0 | 100 |
| | 31 | 80 | 20 |
| | 50 | 80 | 20 |

As a result of the HPLC test, it was verified that the pharmaceutical composition sensitive to nitrogen monoxide was capable of separating a nitrogen monoxide scavenger in an environment where excess nitrogen monoxide is present, and capable of releasing a low-molecular-weight compound having pharmaceutical activity.

Specifically, referring to FIG. 2A, the peak of Compound (4) of Example 4, which is the pharmaceutical composition sensitive to nitrogen monoxide, was detected at 14.2 minutes. On the other hand, referring to FIG. 2B, the peak of Compound (4) disappeared in an environment where excess nitrogen monoxide is present, and a peak detected at 7.7 minutes was generated. FIG. 2C shows the result of HPLC test on tofacitinib, a low-molecular-weight compound having pharmaceutical activity, and a peak was detected at 7.7 minutes. Accordingly, FIGS. 2A to 2C are results showing that Compound (4) of Example 4 reacted with excess nitrogen monoxide to release tofacitinib.

**Experimental Example 4: Verification of Nitrogen Monoxide Scavenging Activity of Pharmaceutical Composition Sensitive to Nitrogen Monoxide in Immune Cells**

Raw 264.7 cell line was seeded in a 48-well plate at a density of $8\times10^4$ cells/well, and cultured at 37°C in a DMEM medium including 10% FBS, 100 U/mL of penicillin and 100 μg/mL of streptomycin.

The medium was cultured overnight, and then replaced with a fresh medium including 10 μM of the pharmaceutical composition sensitive to nitrogen monoxide including the compound of each of Examples 1 to 30 and 500 ng/mL of LPS, and the fresh medium was cultured again for another 24 hours.

Lastly, in order to measure the amount of NO produced, the amount of $NO_2^-$ present in the cell culture medium was measured using a Griess reagent (sulfanilamide, N-1-naphthylethylenediamine dihydrochloride). Specifically, 100 μL of a Griess reagent was mixed with a mixture solution of 50 μL of the cell culture medium and 50 μL of PBS. The mixture solution was reacted for 10 minutes in a well plate, and absorbance thereof at 540 nm was measured. NO scavenging activity was calculated by the following Equation 2, and the results are shown in the following Table 13 (Examples 12 and 13 represent SD values each derived from at least three independent experiments, and the remaining Examples represent representative values each from at least three independent experiments).

NO scavenging activity (%) = 100 - [(NO value of group treated with compound of Example - NO value of group with no treatment)/(NO value of LPS-stimulated group-NO value of group with no treatment)] × 100 [Equation 2]

[Table 13]

| Example (10 μM) | NO Scavenging Activity (%) (Mean ±SD) | Example (10 μM) | NO Scavenging Activity (%) (Mean ±SD) |
|---|---|---|---|
| 1 | 77.4±0.52 | 16 | 49.3±0.23 |

(continued)

| Example (10 μM) | NO Scavenging Activity (%) (Mean ±SD) | Example (10 μM) | NO Scavenging Activity (%) (Mean ±SD) |
|---|---|---|---|
| 2 | 78.1±0.21 | 17 | 29.6±2.95 |
| 3 | 95.4±0.11 | 18 | 55. 8±1.17 |
| 4 | 68.7±0.74 | 19 | 54.1±2.51 |
| 5 | 84.1±0.01 | 20 | 59.5±0.11 |
| 6 | 68.5±0.11 | 21 | 61.2±0.15 |
| 7 | 88.2±0.06 | 22 | 31.3±0.88 |
| 8 | 72.6±0.31 | 23 | 16.8±0.70 |
| 9 | 78.9±0.21 | 24 | 9.8±0.64 |
| 10 | 93.7±0.11 | 25 | 63.2±0.56 |
| 11 | 48.7±0.01 | 26 | 73.7±0.33 |
| 12 | 31.7±0.15 | 27 | 59.3±0.06 |
| 13 | 34.7±0.28 | 28 | 49.8±0.43 |
| 14 | 46.0±2.52 | 29 | 90.1±0.74 |
| 15 | 54.0±0.59 | 30 | 91.6±0.68 |

Referring to Table 13, it was verified that the concentration of nitrogen monoxide was reduced in immune cells as well when treated with the compounds of the Examples.

**Experimental Example** 5: **Verification of Inhibitory Effect of Pharmaceutical Composition Sensitive to Nitrogen Monoxide on Inflammatory Cytokine Secretion in Immune Cells**

Raw 264.7 cell line was seeded in a 48-well plate at a density of $8\times10^4$ cells/well, and cultured at 37°C in a DMEM medium including 10% FBS, 100 U/mL of penicillin and 100 μg/mL of streptomycin.

The medium was cultured overnight, and then replaced with a fresh medium including 10 μM of the pharmaceutical composition sensitive to nitrogen monoxide including the compound of each of Examples 1 and 11 to 13 and 500 ng/mL of LPS, and the fresh medium was cultured again for another 24 hours.

Lastly, the supernatant was collected, and then the level of IL-6 (Interleukin-6) expression was analyzed using a Sandwich enzyme-linked immunosorbent assay (ELISA). Specifically, a standard solution and 50 μL of the diluted supernatant were introduced to each well of a well plate coated with an antibody specific to IL-6, and after binding IL-6 to the immobilized antibody, the well was washed with a washing buffer, followed by a reaction with HRP as a secondary antibody for 30 minutes at room temperature. The well was washed three times with a washing buffer, then color was developed using TMB (3,3', 5,5'-tetramethyl benzidine) as a substrate, and the reaction was stopped using 1 M sulfuric acid to measure the degree of color development at an absorbance of 450 nm. Based on the degree of color development, the degree of reduction in the IL-6 expression level was calculated by the following Equation 3, and the results are shown in the following Table 14.

IL-6 reduction activity (%) = 100 - [(IL-6 quantification value of group treated with compound of Example-IL-6 quantification value of group with no treatment)/(IL-6 quantification value of LPS-stimulated group-IL-6 quantification value of group with no treatment)] × 100 [Equation 3]

[Table 14]

| Example (10 μM) | IL-6 Reduction Activity (%) (Mean ±SD) | Example (10 μM) | IL-6 Reduction Activity (%) (Mean ±SD) |
|---|---|---|---|
| 1 | 93.6±0.60 | 12 | 61.68±1.85 |
| 11 | 43. 6±1. 87 | 13 | 63.10±1.23 |

Referring to Table 14, it was verified that inflammatory cytokine secretion was suppressed in immune cells when treated with the compounds of the Examples.

**Experimental Example 6: Verification of Nitrogen Monoxide Scavenging Activity, Inhibitory Effect on Immune Cell Proliferation and Inhibitory Effect on Inflammatory Cytokine Secretion of Pharmaceutical Composition Sensitive to Nitrogen Monoxide**

Experimental Example 6-1: Verification of Nitrogen Monoxide Scavenging Activity in Splenocytes

A spleen was isolated from an 8-week-old C57BL/6 mouse, and splenocytes were isolated using a physical method.

The separated splenocytes were diluted to a concentration of $5\times10^6$ cells/mL in a RPMI 1640 medium including 10% FBS, 100 U/mL of penicillin and 100 μg/mL of streptomycin, and the diluted splenocytes were dispensed at 500 μL/well into a 48-well plate and 100 μL/well into a 96-well plate, and cultured for 72 hours in an incubator. The splenocytes were treated with Concanavalin A (5 μg/mL) when dispensed to induce an immune response, and herein, 10 Mm of the pharmaceutical composition including the compound according to each of the Examples was also added thereto. After 72 hours, the supernatant was collected, and the level of remaining NO was checked through a Griess assay, and calculated by the following Equation 4. The results are shown in the following Table 15.

NO scavenging activity (%) = 100 - [(No value of group treated with compound of Example-NO value of group with no treatment)/(NO value of Concanavalin A-stimulated group- NO value of group with no treatment)] × 100 [Equation 4]

Experimental Example 6-2: Verification of IFN-γ Reduction Activity in Splenocytes

NO is one of important inflammatory mediators, and may increase the level of inflammatory cytokine. Concanavalin A (5 μg/mL), which induces an immune response in splenocytes, and the compound of each of Examples were treated in a concentration-dependent manner to check whether the level of IFN-γ, a representative inflammatory cytokine, was reduced. The supernatant was collected 72 hours after the treatment, and analyzed using a quantitative enzyme-linked immunosorbent assay (Quantitative-ELISA). The results are shown in the following Table 15.

Experimental Example 6-3: Verification of Inhibitory Activity on Cell Proliferation in Splenocyte

In Experimental Example 6-1, the level of cell proliferation in splenocyte was verified through a CCK-8 analysis instead of the Griess assay, and the results are shown in the following Table 15.

[Table 15]

| Example (10 μM) | NO Scavenging Activity (%) (Mean±SD) | IFN-γ Reduction Activity (%) (Mean±SD) | Inhibitory Activity on Cell Proliferation (%) (Mean±SD) |
|---|---|---|---|
| 1 | 98.1±0.09 | 91.5±1.37 | 55.01±8.48 |
| 2 | 94.3±0.27 | 95.2±0.39 | 90.43±4.69 |
| 3 | 98.1±0.34 | 99.1±0.61 | 76.17±2.45 |
| 4 | 91.0±0.01 | 98.9±0.03 | 79.51±6.67 |
| 5 | 94.1±0.52 | 96.1±0.45 | 60.47±5.48 |
| 10 | 77.0±0.87 | 93.0±2.99 | 76.35±2.45 |
| 11 | 67.7±0.84 | 57.6±15.53 | 49.71±3.78 |
| 12 | 64.6±3.65 | 97.0±0.05 | 64.00±6.17 |
| 13 | 66.9±5.06 | 89.7±0.57 | 16.15±2.70 |
| 15 | 31.6±0.16 | 86.5±0.29 | 22.62±6.36 |
| 16 | 27.1±1.20 | 81.4±0.50 | 24.96±5.67 |
| 21 | 52.4±0.67 | 98.1±0.03 | 48.34±3.72 |
| 22 | 45.9±0.51 | 41. 8±3.14 | 19.40±0.21 |

(continued)

| Example (10 μM) | NO Scavenging Activity (%) (Mean±SD) | IFN-γ Reduction Activity (%) (Mean±SD) | Inhibitory Activity on Cell Proliferation (%) (Mean±SD) |
|---|---|---|---|
| 23 | 19.5±0.65 | 68.0±0.45 | 11.80±2.08 |
| 24 | 78.3±0.50 | 95.5±0.15 | 67.17±4.67 |
| 25 | 100.0±0.00 | 98.7±0.04 | 81.36±7.94 |
| 26 | 100.0±0.00 | 97.4±0.10 | 75.58±0.96 |
| 27 | 77.6±1.24 | 74.6±2.41 | 81.14±9.03 |
| 28 | 92.0±0.56 | 100.0±0.00 | 80.69±7.12 |
| 29 | 88.5±0.47 | 100.0±0.00 | 78.03±6.90 |
| 30 | 98.1±0.09 | 91.5±1.37 | 55.01±8.48 |

Referring to Table 15, it was verified that, when treated with the compounds of the Examples, nitrogen monoxide was removed, inflammatory cytokine secretion was suppressed, and cell proliferation was suppressed in splenocytes.

**Experimental Example** 7: **JAK 1, 2 and 3 Activity Assay of Pharmaceutical Composition Sensitive to Nitrogen Monoxide**

For the compounds of the Examples, JAK activity of each of the compounds was verified using the JAK Human TK Kinase Enzymatic Radiometric Kinase Profiler Assay service provided by Eurofins Scientific.

The activity was verified after inducing a reaction at the JAK 1, 2 and 3 active sites using radioactively labeled ATP. JAK 1,2 and 3 activities depending on each concentration are shown in the following Tables 16 and 17. The JAK activity close to 100 indicates low JAK activity of the compound according to the Example.

[Table 16]

| | Example | Concentration (nM) | | | |
|---|---|---|---|---|---|
| | | 0.1 | 1 | 10 | 100 |
| JAK 1 Activity | 1 | 101 | 102 | 45 | 7 |
| | 11 | 108 | 103 | 105 | 101 |
| | 12 | 109 | 110 | 103 | 81 |
| | 13 | 110 | 113 | 106 | 89 |
| | 15 | 102 | 90 | 99 | 66 |
| | 16 | 101 | 104 | 98 | 59 |
| | 21 | 97 | 105 | 102 | 85 |
| | 22 | 91 | 97 | 84 | 77 |
| | 23 | 94 | 90 | 100 | 83 |
| | Positive Control Group (Tofacitinib) | 107 | 69 | 17 | 3 |

[Table 17]

| | Example | Concentration (nM) | | | |
|---|---|---|---|---|---|
| | | 0.1 | 1 | 10 | 100 |
| JAK 2 Activity | 1 | 100 | 92 | 69 | 16 |
| | 11 | 91 | 99 | 89 | 98 |
| | 12 | 108 | 111 | 102 | 92 |

(continued)

| | Example | Concentration (nM) | | | |
|---|---|---|---|---|---|
| | | 0.1 | 1 | 10 | 100 |
| | 13 | 120 | 107 | 102 | 104 |
| | Positive Control Group (Tofacitinib) | 109 | 87 | 35 | 4 |
| JAK 3 Activity | 1 | 94 | 95 | 55 | 16 |
| | 11 | 103 | 103 | 98 | 94 |
| | 12 | 114 | 107 | 111 | 88 |
| | 13 | 113 | 109 | 112 | 92 |
| | Positive Control Group (Tofacitinib) | 96 | 80 | 27 | 5 |

Referring to Table 16, activity of the pharmaceutical composition sensitive to nitrogen monoxide for JAK 1 was very low compared to the activity of tofacitinib, a positive control group. Particularly, as shown in Table 17, it was verified that tofacitinib, a positive control group, exhibited high activity for JAK 2 and 3 as well as JAK 1 at 100 nM, whereas Compounds (11) to (13) exhibited very low activity.

**Experimental Example** 8: **Dose Escalation Experiment of Pharmaceutical Composition Sensitive to Nitrogen Monoxide**

Compound (1) and tofacitinib, a positive control group, were administered orally once daily with increasing doses daily to experimental animals, and then maximum tolerated dose in mice was compared.

Specifically, the experimental animals were twelve six-week-old C57BL/6 male mice. The experimental animals were housed in polycarbonate cages in groups of four, and had free access to food and water. The animal housing room was maintained at a temperature of 20±1°C and humidity of about 50±5%, with a constant 12-hour light/dark cycle. The experimental animals were acclimated to the animal room environment for one week, and then randomly divided into three groups of four animals each based on body weight. The experimental groups and the compounds administered to each of the experimental groups were set as shown in the following Table 18.

[Table 18]

| Experimental Group | Administered Compound | Dose (mg/kg) |
|---|---|---|
| Normal | | |
| Example | Compound (1) | 200 |
| | | 400 |
| | | 800 |
| | | 1,600 |
| | Tofacitinib | 200 |
| Positive Control Group | | 400 |
| | | 800 |
| | | 1, 600 |

Mortality was monitored while administering the administered compounds of Table 18 as a single oral dose once daily, starting at a low dose of 200 mg/kg on day 1 and escalating daily with a geometric ratio of 2 to 400 mg/kg on day 2, 800 mg/kg on day 3 and 1,600 mg/kg on day 4.

In the group administered with escalating doses of Compound (1), no mortality was observed within 24 hours after escalating the dose up to 1,600 mg/kg. On the other hand, in the group administered with tofacitinib, a positive control group, two mice died after administering 800 mg/kg on day 3 and before administering on day 4. Accordingly, the maximum tolerated dose of tofacitinib, a positive control group, was verified to be 400 mg/kg, and the maximum tolerated dose of Compound (1) of Example was verified to be 1,600 mg/kg.

**Experimental Example 9: Hematological Toxicity Evaluation of Pharmaceutical Composition Sensitive to Nitrogen Monoxide after Single Oral Administration**

Compounds (1) and (4) were administered orally as a single dose to experimental animals, and hematological toxicity responses were compared with the responses of tofacitinib.

Preparation of the experimental animals was conducted in the same manner as in the previous Experimental Example, and the experimental groups and the compounds administered to each of the experimental groups were set as shown in the following Table 19.

[Table 19]

| Experimental Group | Administered Compound | Dose (mg/kg) |
|---|---|---|
| Normal | | |
| Example | Compound (1) | 200 |
| | | 800 |
| | Compound (4) | 200 |
| | | 800 |
| Positive Control Group | Tofacitinib | 200 |
| | | 800 |

The administered compounds of Table 19 were administered orally once daily as a single dose, and two days after the administration, the mice were necropsied, and whole blood was collected from the heart. Herein, to prevent blood coagulation, the samples were placed in EDTA (ethylene diamine tetra acetate) tubes, and then a routine blood test (XN-1000, SYSMEX, Japan) was performed to assess hematological toxicity. The results are shown in the following Table 20.

[Table 20]

| Experimental Group | Dose (mg/kg) | WBC (% of Normal) | WBC Differential Counting (NEU) (% of Normal) | WBC Differential Counting (LYM) (% of Normal) |
|---|---|---|---|---|
| Normal | | 100±5.6 | 100±16.4 | 100±7.0 |
| Example (Compound (1)) | 200 | 94.6±7.7 | 105. 9±18.1 | 91.9±5.2 |
| | 800 | 89.6±16. 6 | 64.4±4.5 | 92.6±19.7 |
| Example (Compound (4)) | 200 | 104.0±2. 6 | 108.9±22.7 | 103.5±4.4 |
| | 800 | 94.8±18. 7 | 65.3±18.6 | 99. 0±18.4 |
| Positive Control Group | 200 | 80.7±28. 6 | 68.3±15.7 | 81.1±31.3 |
| | 800 | 62.1±4.7 * | 56.4±7.9* | 61.2±4.6* |
| (Tofacitinib) | | | | |

p<0.05, ** p<0.01, *** p<0.001, compared with the normal group, as determined by Student t-test.

Toxicity of the test substances was compared by evaluating hematological toxicity after single dose administration at a low dose of 200 mg/kg and a high dose of 800 mg/kg. No hematological toxicity responses were observed in all the groups administered with Compounds (1) and (4), as there were no statistically significant differences compared with the normal control group.

Only in the group administered with tofacitinib, a positive control group, at a high dose of 800 mg/kg, hematological toxicity was observed with statistically significant decreases in total white blood cell (WBC), neutrophils (NEU) and lymphocytes (LYM) counts (p<0.05).

Accordingly, comparing the hematological toxicity results when the compounds are administered orally as a single dose at the same high dose of 800 mg/kg to mice, hematological toxicity of the compound of the Example was verified to be low compared to the hematological toxicity of tofacitinib.

**Experimental Example 10: Efficacy Evaluation of Pharmaceutical Composition Sensitive to Nitrogen Monoxide on DSS Induced Ulcerative Colitis**

Experimental Example 10-1: Efficacy Evaluation of Compound (1)

Compound (1) of the Example was administered orally once daily to experimental animals having ulcerative colitis, and disease-alleviating efficacy of Compound (1) was compared with that of tofacitinib, a positive control group.
The experimental animals were prepared in the same manner as in the previous Experimental Example except that the animals were randomly divided into four groups of 5 animals each.
In order to establish an ulcerative colitis model, 2.5% DSS powder was mixed with drinking water and provided ad libitum. The DSS powder-containing drinking water was supplied for 5 to 6 days of disease induction period, and after that, normal drinking water was supplied. The experimental groups and the compounds administered to each of the experimental groups were set as shown in the following Table 21.

[Table 21]

| Experimental Group | Administered Compound | Dose (mg/kg) | Duration of Drug Administration (Duration of Treatment) |
|---|---|---|---|
| Normal | - | - | From Day 3 after Disease Induction to End of Experiment |
| Example | Compound (1) | 25 | |
| Positive Control Group | Tofacitinib | 25 | |

The administered compounds of Table 21 were administered orally once daily. Hematochezia, diarrhea and body weight loss were scored according to the criteria shown in the following Table 22, and the scores were summed to calculate a disease activity index (DAI), which is shown in FIG. 4.

[Table 22]

| Score | Severity of Hematochezia | Severity of Diarrhea | Percentage of Body Weight Loss with Respect to Initial Weight |
|---|---|---|---|
| 0 | None | None | Less than 1% |
| 1 | Mild hematochezia and dry anus | Loose stool not adhering to anus | 1% or more and less than 5% |
| 2 | Severe hematochezia and blood observed around anus | Very loose stool adhering to anus | 5% or more and less than 10% |
| 3 | Dark red hematochezia and blood adhering around anus | Wet anus and elongated liquid-type stool | 10% or more and less than 15% |
| 4 | Blood or blood clots | Diarrhea | 15% or more |

Referring to FIG. 4, statistically significant efficacy was observed with tofacitinib ($p<0.01$) and Compound (1) ($p<0.01$) on day 7 of the test. The efficacy of Compound (1) in alleviating ulcerative colitis was verified to be comparable to that of tofacitinib on average.

Experimental Example 10-2: Efficacy Evaluation of Compound (4)

Efficacy of Compound (4) on ulcerative colitis was verified in the same manner as in Experimental Example 10-1, except that Compound (4) was used instead of Compound (1), and DSS was administered until day 5, and the drug was administered from day 6. The results are shown in FIG. 5.
Referring to FIG. 5, the efficacy of tofacitinib in alleviating ulcerative colitis was not observed on day 9 of the test, and statistically significant efficacy was observed only in the group administered with Compound (4) ($p<0.05$).
From the results of FIGS. 4 and 5, it was verified that Compounds (1) and (4) had comparable or more superior efficacy in alleviating ulcerative colitis compared to tofacitinib.

**Experimental Example 11: Efficacy Evaluation of Pharmaceutical Composition Sensitive to Nitrogen Monoxide on Rheumatoid Arthritis**

Experimental Example 11-1: Efficacy Evaluation of Compound (1)

Compound (1) of the Example and tofacitinib were administered orally once daily to experimental animals having rheumatoid arthritis, and their disease-alleviating efficacy was compared.

The experimental animals were 30 8-week-old DBA/1 male mice. The experimental animals were housed in polycarbonate cages in groups of six, and had free access to food and water. The animal housing room was maintained at a temperature of 20±1°C and humidity of about 50±5%, with a constant 12-hour light/dark cycle. The experimental animals were acclimated to the animal room environment for one week, and then randomly divided into five groups. The number of normal mice was set to five in order to minimize the number of experimental animals. The group administered with Compound (1) consisted of 5 to 6 mice, a minimal number required to obtain statistically meaningful data given the limited amount of the drug.

In order to establish a rheumatoid arthritis model, 100 μL of a mixture solution in which 2 mg/mL of chick type II collagen and 1 mg/mL of Complete Freund's Adjuvant (CFA) were mixed in a ratio of 1:1 was administered intradermally to each of the mouse tails. After 19 days, 100 μL of an emulsion in which 2 mg/mL of chick type II collagen and Incomplete Freund's Adjuvant (IFA) were mixed in a ratio of 1:1 was administered intradermally to each of the mouse tails. The experimental groups and the compounds administered to each of the experimental groups were set as shown in the following Table 23.

[Table 23]

| Experimental Group | Administered Compound | Dose (mg/kg) | Duration of Drug Administration |
|---|---|---|---|
| Normal | - | - | From Day 19 after Disease Induction to End of Experiment |
| Disease-Induced Group (RA) | - | - | |
| Example | Compound (1) | 30 | |
| Example | Compound (1) | 60 | |
| Positive Control Group | Tofacitinib | 40 | |

The test substances of Table 23 were administered orally every day according to the drug dosing period and dosing frequency. As for the rheumatoid arthritis score (arthritis score), the score of each of the four paws was quantified according to the criteria shown in the following Table 24, and the individual scores were summed to generate the final score. The results are shown in FIG. 6.

[Table 24]

| Rheumatoid Arthritis Score | Symptoms |
|---|---|
| 0 | Normal, no difference from normal animal, and no symptoms. |
| 1 | Inflammation in 1 or 2 toes, no swelling in dorsum of foot and ankle. |
| 2 | Inflammation in 3 or more toes, but foot not swollen or mildly swollen overall. |
| 3 | Entire foot swollen. |
| 4 | Entire foot and dorsum of foot severely swollen, or ankle and toes swollen enough to impair foot function. |

Referring to FIG. 6, the group administered with tofacitinib and the group administered with Compound (1) showed comparable efficacy in alleviating rheumatoid arthritis symptoms from day 28 after disease induction on average. From day 33 after disease induction ($p<0.01$) to the end of the experiment, statistically significant rheumatoid arthritis-alleviating efficacy was observed in the group administered with 40 mg/kg of tofacitinib. Alleviating efficacy was observed from day 35 after disease induction ($p<0.05$) in the group administered with 30 mg/kg of Compound (1), and although no statistically significant alleviating efficacy was observed from day 42 to day 47 after disease induction, statistically significant alleviating efficacy was observed again from day 49 to the end of the experiment ($p<0.05$). In the group administered with 30 mg/kg of Compound (1), the average rheumatoid arthritis score was observed to be comparable to that of the group administered with 40 mg/kg of tofacitinib, and the lack of statistically significant alleviating efficacy observed from day 42 to day 47 after disease induction was verified to be attributed to inter-individual differences.

In the group administered with 60 mg/kg of Compound (1), rheumatoid arthritis-alleviating efficacy comparable to or higher than that of the group administered with 40 mg/kg of tofacitinib, which corresponds to the same dose of tofacitinib

included in 60 mg/kg of Compound (1), was observed from day 33 after disease induction ($p<0.01$) to the end of the experiment.

Accordingly, when Compound (1) was administered orally once daily at concentrations of 30 mg/kg and 60 mg/kg throughout the entire period of the experiment, it was verified that Compound (1) alleviated rheumatoid arthritis at a level comparable to or higher than 40 mg/kg of tofacitinib.

Experimental Example 11-2: Efficacy Evaluation of Compound (11)

Compound (11) of the Example was administered orally once daily to experimental animals having rheumatoid arthritis, and its disease-alleviating efficacy was compared with that of tofacitinib, a positive control group.

The experimental animals were 31 8-week-old DBA/1 male mice. The experimental animals were housed in polycarbonate cages in groups of five animals each, and had free access to food and water. The animal housing room was maintained at a temperature of 20±1°C and humidity of about 50±5%, with a constant 12-hour light/dark cycle. The experimental animals were acclimated to the animal room environment for one week, and then randomly divided into five groups based on body weight. The number of normal mice was set to five in order to minimize the number of experimental animals. The group administered with Compound (11) consisted of 5 mice, a minimal number required to obtain statistically meaningful data given the limited amount of the drug. Each of the remaining groups consisted of 7 mice, with two extra mice added to account for experimental error.

In order to establish a rheumatoid arthritis model, 100 μL of a mixture solution in which 2 mg/mL of chick type II collagen and 1 mg/mL of Complete Freund's Adjuvant (CFA) were mixed in a ratio of 1:1 was administered intradermally to each of the mouse tails. After 19 days, 100 μL of an emulsion in which 2 mg/mL of chick type II collagen and Incomplete Freund's Adjuvant (IFA) were mixed in a ratio of 1:1 was administered intradermally to each of the mouse tails.

The experimental groups and the compounds administered to each of the experimental groups were set as shown in the following Table 25.

[Table 25]

| Experiment al Group | Administere d Compound | Dose (mg/kg ) | Administrati on Frequency | Duration of Drug Administrati on (Duration of Treatment) |
|---|---|---|---|---|
| Normal | - | - | - | From Day 19 after Disease Induction to End of Experiment |
| Disease-Induced Group (RA) | - | - | - | |
| Example | Compound (11) | 20 | Once Daily (QD) | |
| Positive Control Group | Tofacitinib | 10 | Twice Daily (BID) | |
| Positive Control Group | Dexamethaso ne | 1 | Once Daily (QD) | |

Oral administration was conducted daily according to the dose, dosing frequency and dosing period of Table 25. Based on the criteria shown in Table 24, rheumatoid arthritis score for the four paws were quantified and summed to generate the score. The results are shown in FIG. 7.

Referring to FIG. 7, the group administered with Compound (11) and the group administered with tofacitinib showed comparable efficacy in alleviating rheumatoid arthritis symptoms from day 28 after the test on average. Statistically significant rheumatoid arthritis-alleviating efficacy was observed in the group administered with tofacitinib on day 39 ($p<0.05$) and day 40 ($p<0.01$) after the test. Although no statistical differences were verified in the group administered with Compound (11) due to within-group variability, it exhibited alleviating efficacy comparable to that of tofacitinib over the entire 40-day period.

## Claims

1. A compound of the following Chemical Formula 1, or a solvate, a hydrate, a stereoisomer or a pharmaceutically acceptable salt thereof:

[Chemical Formula 1]

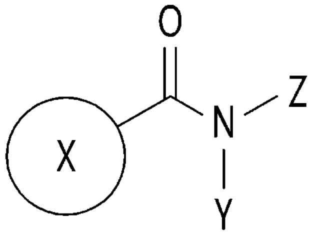

wherein, in Chemical Formula 1,

X is a low-molecular-weight compound including an amine group or a hydroxyl group in the molecular structure of the compound and having pharmaceutical activity;
Y is selected from the group consisting of hydrogen, a $C_1$-$C_6$ alkyl group, a $C_1$-$C_6$ alkoxy group, a hydroxyl group, a nitro group, an amino group, halogen, a thiol group, a cyano group and

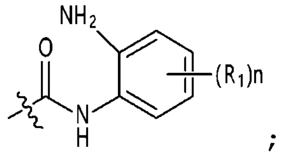

;

Z is selected from the group consisting of

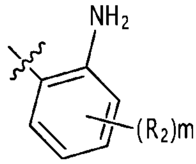

,

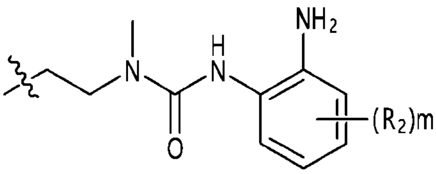

,

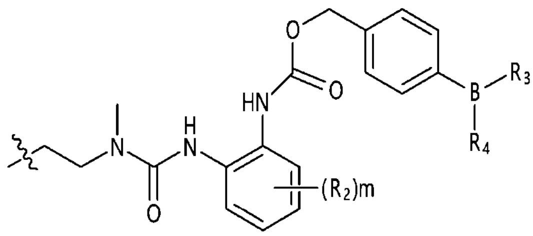

and

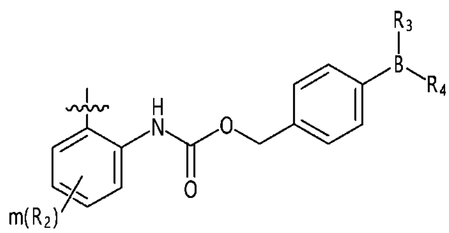

;

n and m are each independently an integer of 0 to 4;
$R_1$ and $R_2$ are each independently selected from the group consisting of a $C_1$-$C_6$ alkyl group, a $C_1$-$C_6$ alkoxy group, a hydroxyl group, a nitro group, an amino group, halogen, a thiol group, a cyano group, a $C_3$-$C_{14}$ aryl group, a $C_3$-$C_{14}$ heteroaryl group, $-NR_{11}R_{12}$, $-NR_{12}C(=O)R_{12}$, $-C(=O)R_{11}$ and - $C(=O)OR_{11}$;
$R_{11}$ and $R_{12}$ are each independently selected from the group consisting of hydrogen, halogen, $CF_3$, a $C_1$-$C_6$ alkyl group, a $C_3$-$C_{14}$ aryl group and a $C_3$-$C_{14}$ heteroaryl group;

the aryl group or the heteroaryl group is each independently unsubstituted or substituted with at least one substituent selected from the group consisting of halogen, a hydroxyl group, a thiol group, a cyano group, a $C_1$-$C_6$ alkyl group and a $C_1$-$C_6$ alkoxy group;
when there are two or more $R_1$s and $R_2$s, each independently, these are the same as or different from each other, and two adjacent $R_1$s or $R_2$s optionally bond to each other to form a fused ring or not form a fused ring; and
$R_3$ and $R_4$ are each independently selected from the group consisting of hydrogen, halogen, a hydroxyl group, a thiol group, a cyano group, a $C_1$-$C_6$ alkyl group and a $C_1$-$C_6$ alkoxy group, or $R_3$ and $R_4$ bond to form a fused ring.

2. The compound, or a solvate, a hydrate, a stereoisomer or a pharmaceutically acceptable salt thereof of claim 1, wherein X is a kinase inhibitor, an anticancer agent or an anti-inflammatory agent.

3. The compound, or a solvate, a hydrate, a stereoisomer or a pharmaceutically acceptable salt thereof of claim 2, wherein the kinase inhibitor is selected from the group consisting of netarsudil, fostamatinib, belumosudil, tofacitinib, upadacitinib, baricitinib, filgotinib, abrocitinib, delgocitinib, oclacitinib, peficitinib, and ruxolitinib.

4. The compound, or a solvate, a hydrate, a stereoisomer or a pharmaceutically acceptable salt thereof of claim 2, wherein the anticancer agent is selected from the group consisting of doxorubicin, cyclophosphamide, cisplatin, oxaliplatin, 5-fluorouracil (5-Fu), gemcitabine, paclitaxel, docetaxel, irinotecan, monomethyl auristatin E (MMAE), crizotinib, osimertinib, sorafenib, ibrutinib, ruxolitinib, vemurafenib, ceritinib, alectinib, brigatinib, lorlatinib, capmatinib, gefitinib, erlotinib, lapatinib, icotinib, afatinib, neratinib, dacomitinib, almonertinib, tucatinib, midostaurin, gilteritinib, quizartinib, pexidartinib, sunitinib, pazopanib, vandetanib, axitinib, cabozantinib, regorafenib, apatinib, lenvatinib, tivozanib, fruquintinib, nintedanib, anlotinib, erdafitinib, pemigatinib, avapritinib, ripretinib, pralsetinib, larotrectinib, entrectinib, imatinib, dasatinib, nilotinib, bosutinib, radotinib, ponatinib, acalabrutinib, zanubrutinib, fedratinib, dabrafenib, encorafenib, trametinib, cobimetinib, binimetinib, selumetinib, palbociclib, ribociclib, abemaciclib, idelalisib, copanlisib, duvelisib, alpelisib, tazemetostat, vorinostat belinostat, tucidinostat, panobinostat, enasidenib, ivosidenib, venetoclax, vismodegib, sonidegib, glasdegib, bortezomib, carfilzomib, ixazomib, olaparib, rucaparib, niraparib, talazoparib, umbralisib, trilaciclib, infigratinib, mobocertinib, asciminib, futibatinib, pacritinib, and everolimus.

5. The compound, or a solvate, a hydrate, a stereoisomer or a pharmaceutically acceptable salt thereof of claim 2, wherein the anti-inflammatory agent is selected from the group consisting of dexamethasone, methotrexate, cyclosporine, acetaminophen, etodolac, piroxicam, and aceclofenac.

6. The compound, or a solvate, a hydrate, a stereoisomer or a pharmaceutically acceptable salt thereof of claim 1, which is selected from the group consisting of the following Chemical Formulae 2 to 6:

[Chemical Formula 2]

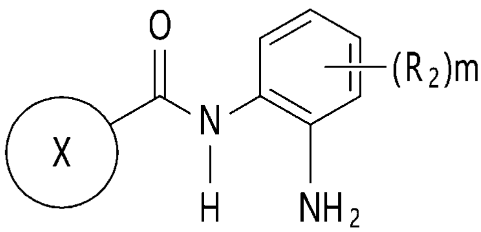

[Chemical Formula 3]

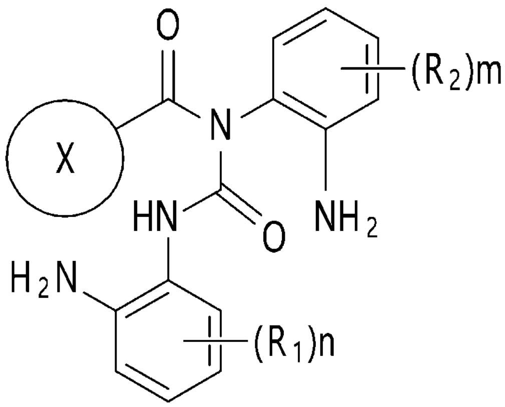

[Chemical Formula 4]

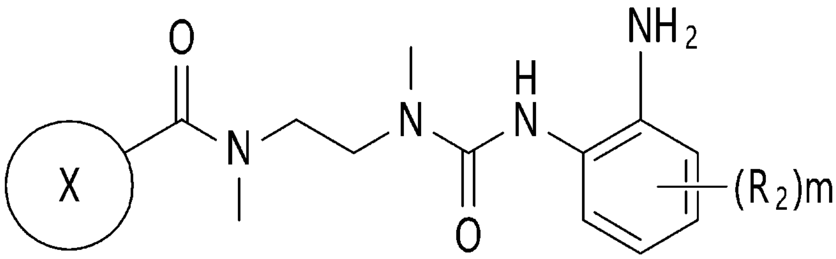

[Chemical Formula 5]

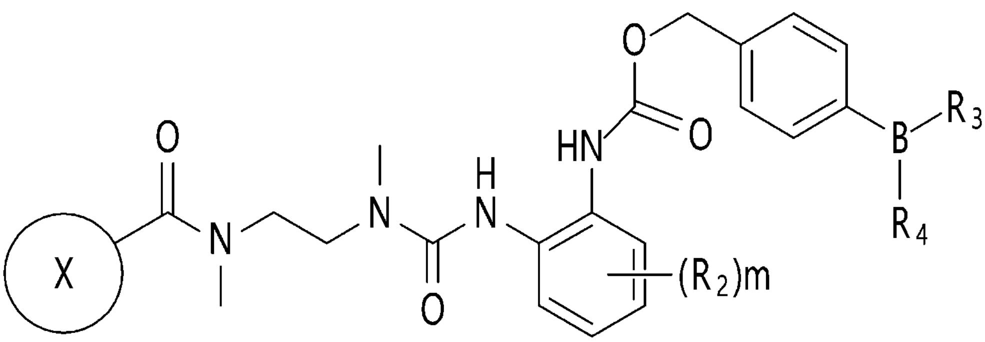

[Chemical Formula 6]

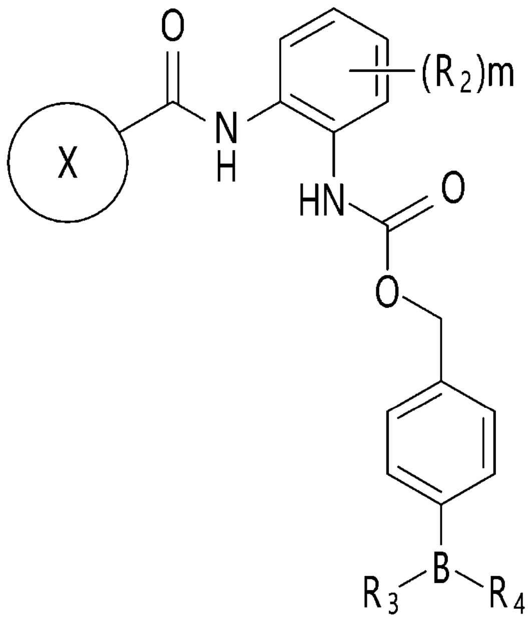

wherein, in Chemical Formulae 2 to 6, X, $R_1$, $R_2$, $R_3$, $R_4$, $R_{11}$, $R_{12}$, n and m have the same definitions as in Chemical Formula 1.

**7.** The compound, or a solvate, a hydrate, a stereoisomer or a pharmaceutically acceptable salt thereof of claim 1, which is selected from the group consisting of the following Compounds (1) to (35):

[Compound (1)]

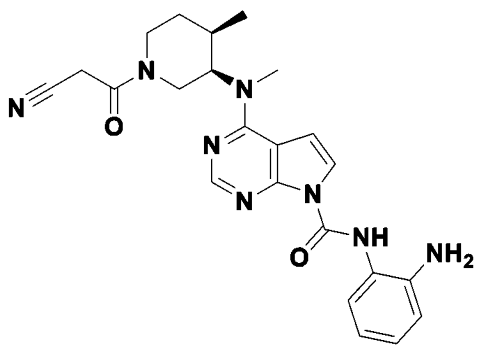

[Compound (2)]

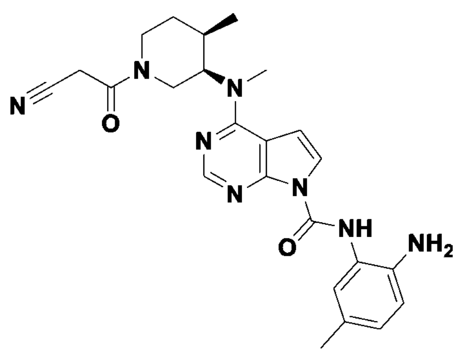

[Compound (3)]

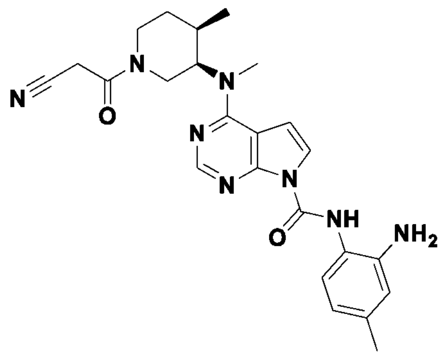

[Compound (4)]

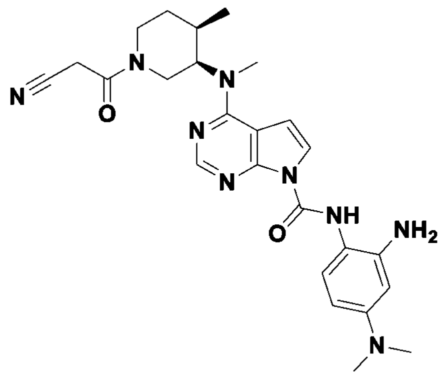

[Compound (5)]

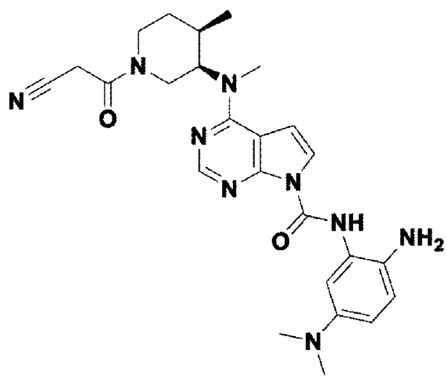

[Compound (6)]

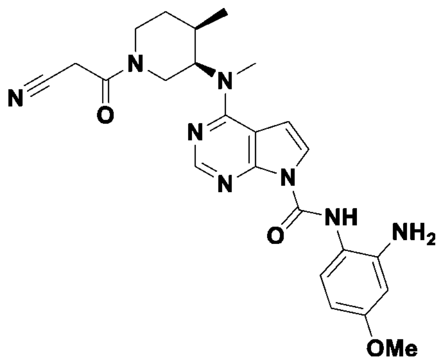

[Compound (7)]

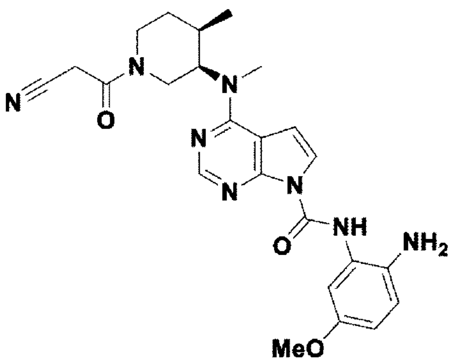

[Compound (8)]

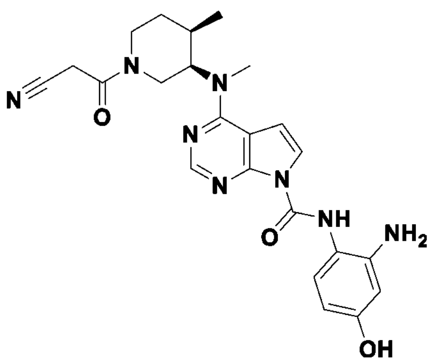

[Compound (9)]

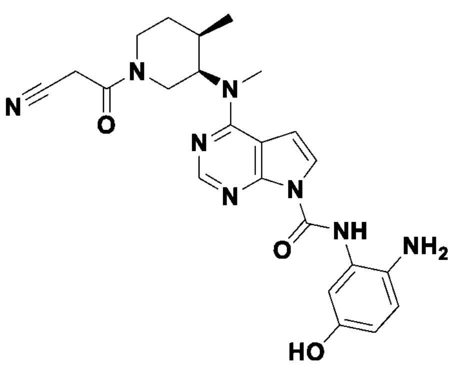

[Compound (10)]

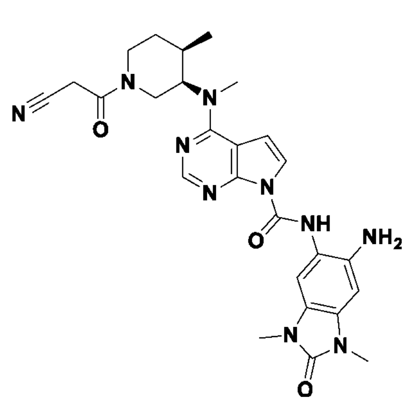

[Compound (11)]

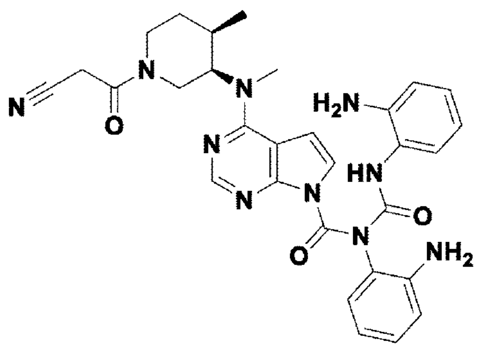

[Compound (12)]

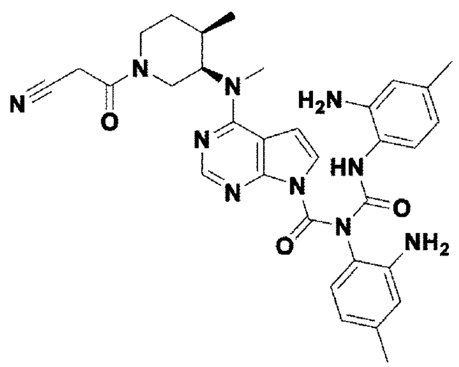

[Compound (13)]

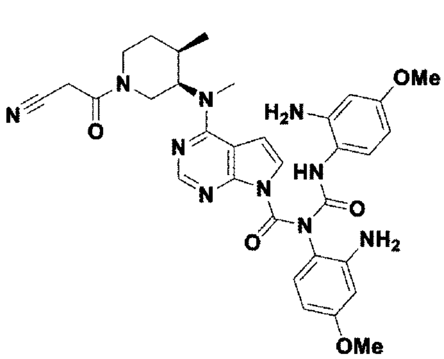

[Compound (14)]

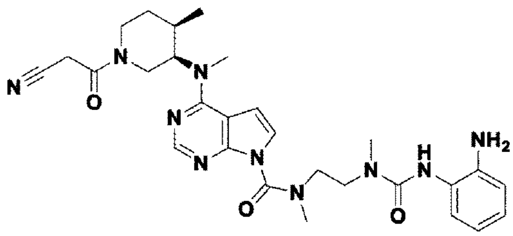

[Compound (15)]

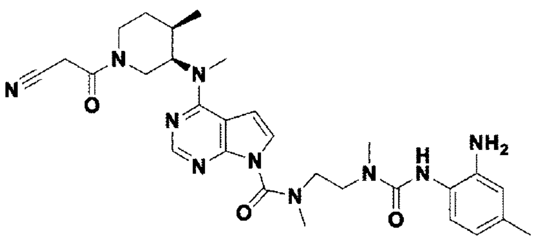

[Compound (16)]

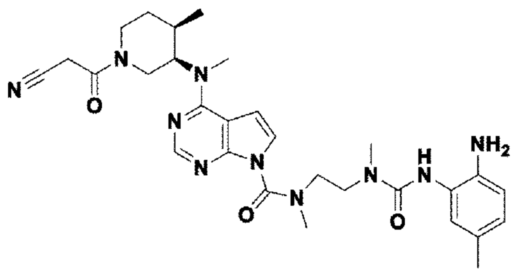

[Compound (17)]

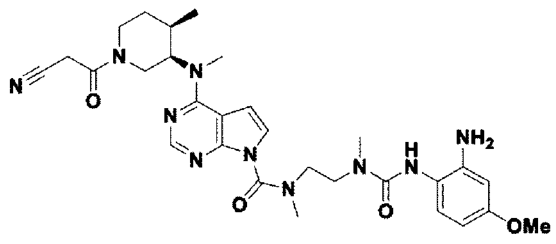

[Compound (18)]

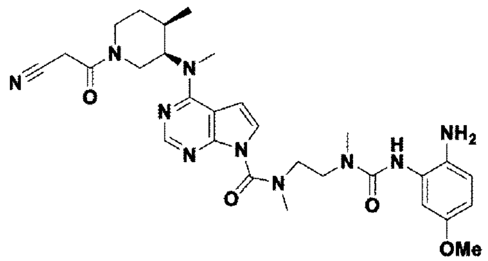

[Compound (19)]

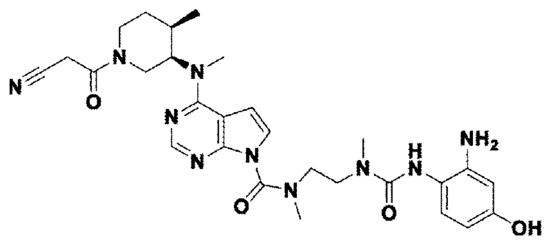

[Compound (20)]

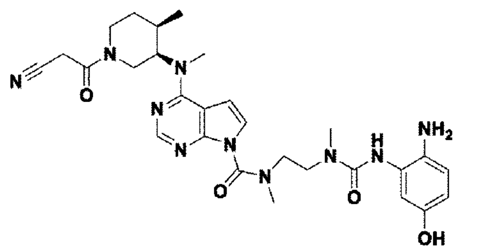

[Compound (21)]

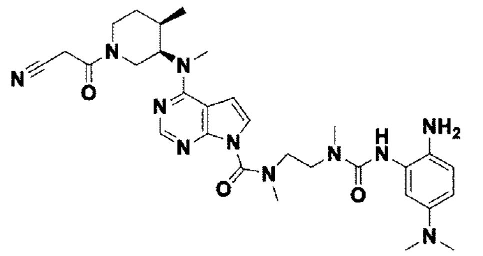

[Compound (22)]

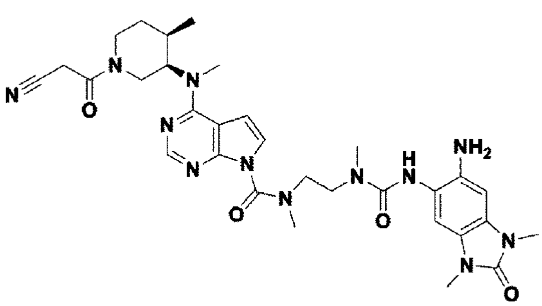

[Compound (23)]

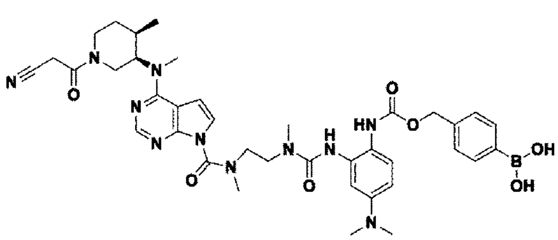

[Compound (24)]

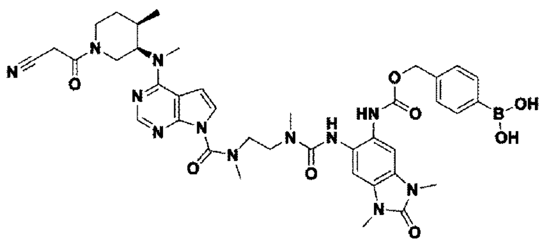

[Compound (25)]

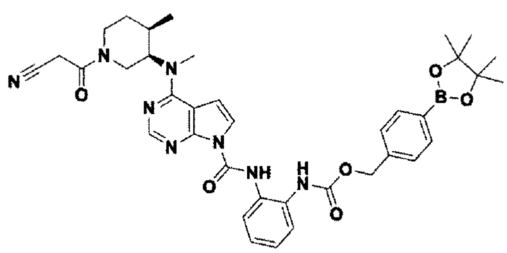

[Compound (26)]

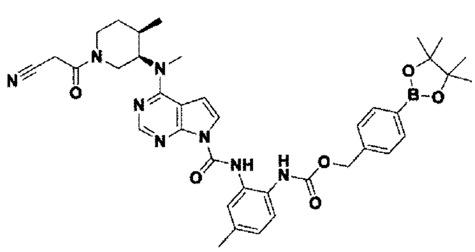

[Compound (27)]

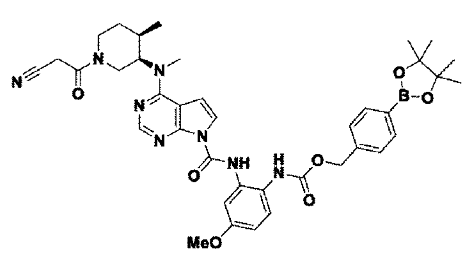

[Compound (28)]

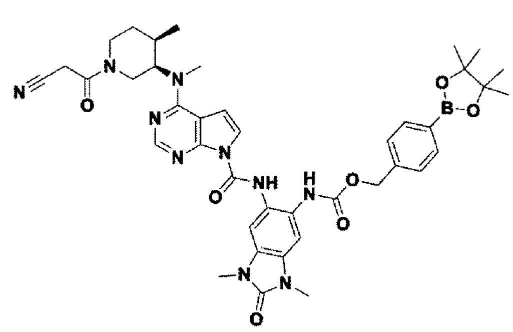

[Compound (29)]

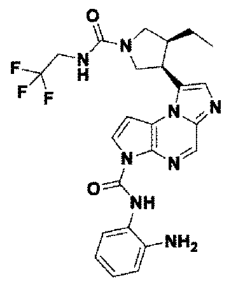

[Compound (30)]

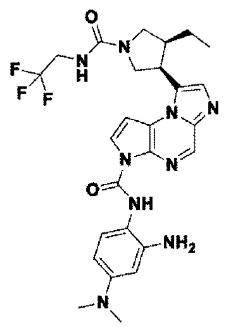

[Compound (31)]

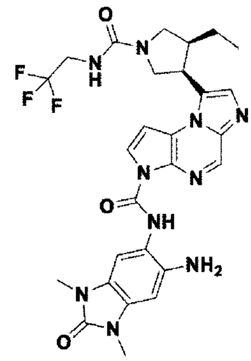

[Compound (32)]

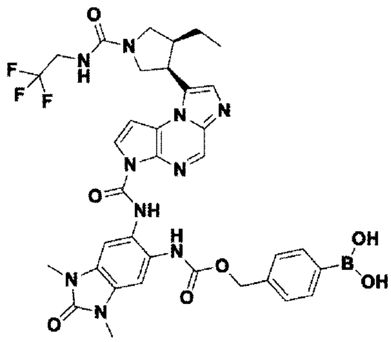

[Compound (33)]

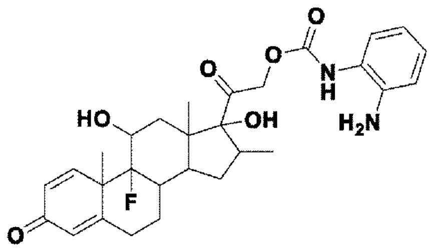

[Compound (34)]

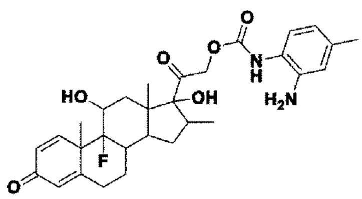

[Compound (35)]

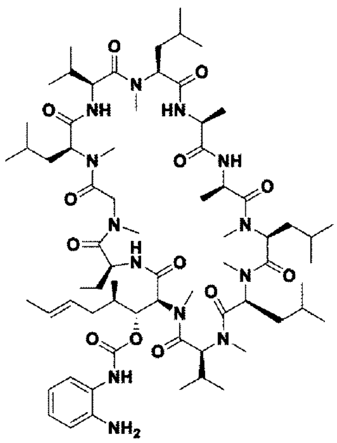

.

**8.** A pharmaceutical composition for preventing and treating a disease associated with nitrogen monoxide generation, the composition comprising the compound, or a solvate, a hydrate, a stereoisomer or a pharmaceutically acceptable salt thereof of claim 1.

**9.** The pharmaceutical composition of claim 8, wherein the disease associated with nitrogen monoxide generation includes:

an inflammatory disease selected from the group consisting of inflammatory diseases caused by viral infection, inflammatory diseases caused by bacterial infection, sepsis and degenerative inflammatory diseases;
a neurological disorder selected from the group consisting of Alzheimer's disease, amyotrophic lateral sclerosis, Huntington's disease, Parkinson's disease and stroke;
a cardiovascular disease, which is hypertension or heart failure;
an autoimmune disease selected from the group consisting of rheumatoid arthritis, ulcerative colitis, Crohn's disease, systemic lupus erythematosus, psoriasis, multiple sclerosis and juvenile idiopathic arthritis;
an allergic disease selected from the group consisting of asthma, rhinitis and atopic dermatitis;
cancer selected from the group consisting of lung cancer, breast cancer, colorectal cancer, gastric cancer, liver cancer, brain cancer, pancreatic cancer, thyroid cancer, skin cancer, bone marrow cancer, lymphoma, uterine cancer, cervical cancer, ovarian cancer, renal cancer and melanoma;
obesity; myelofibrosis; or hepatic encephalopathy.

**10.** The pharmaceutical composition of claim 8, which is administered orally, intraarticularly, intravenously, subcutaneously, intramuscularly, intraperitoneally, intranasally, intrapulmonarily or rectally.

[FIG. 1]

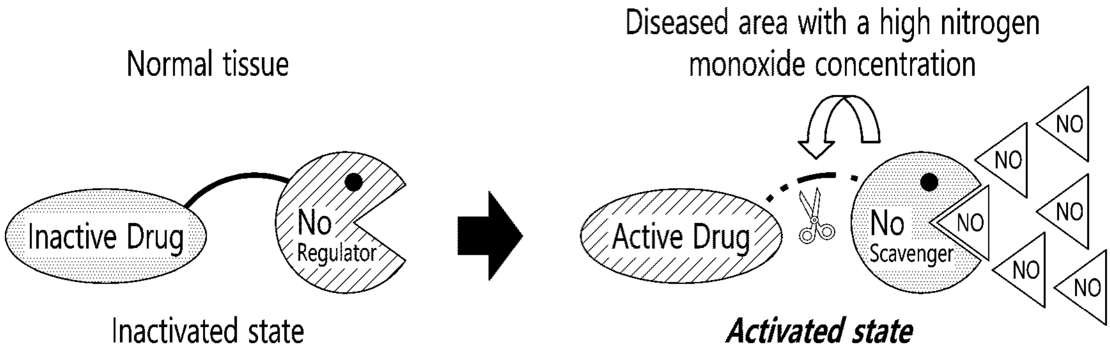


[FIG. 2A]

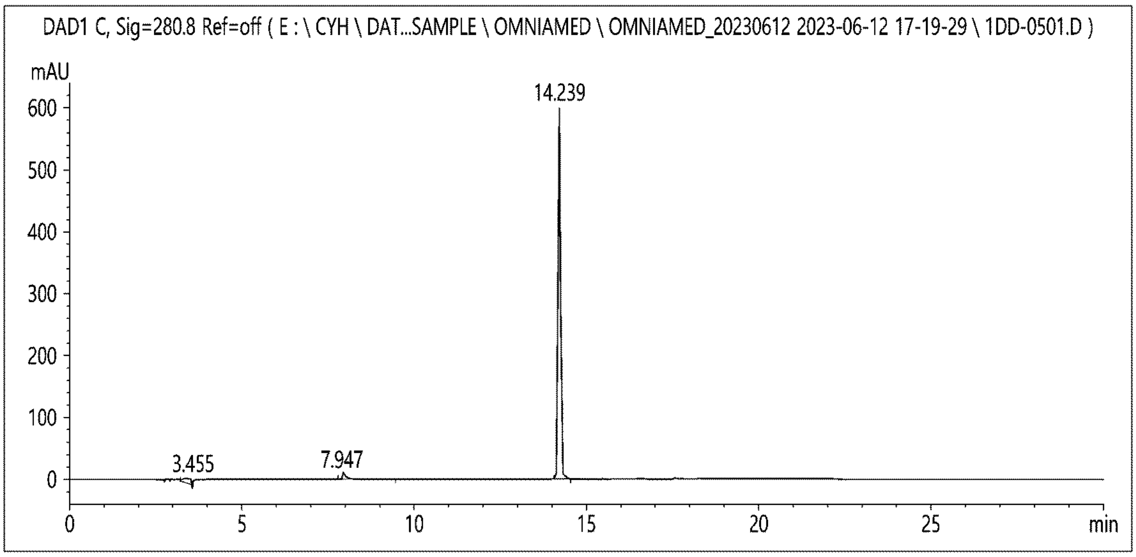


[FIG. 2B]

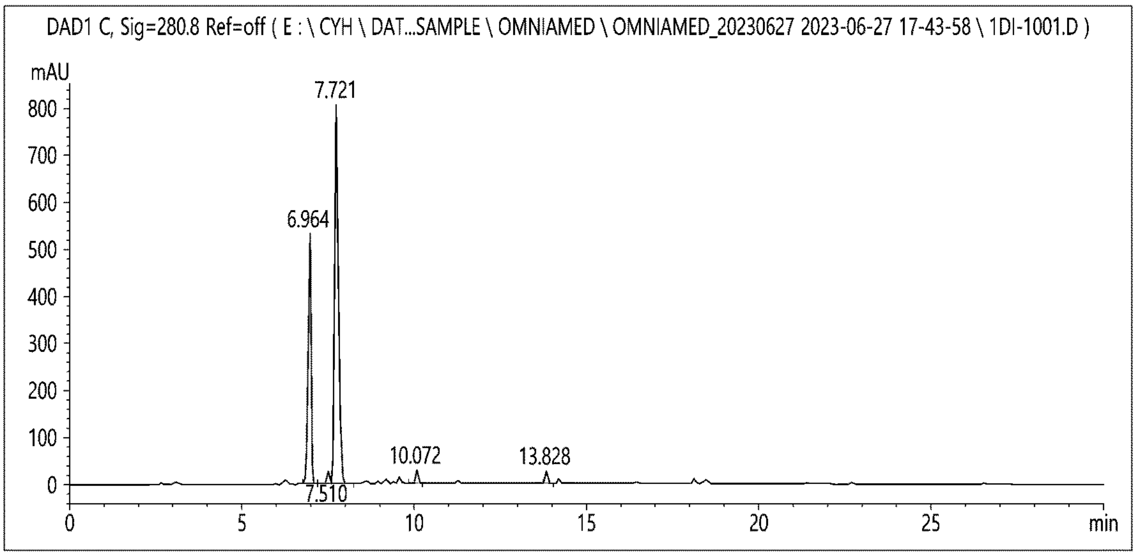

[FIG. 2C]

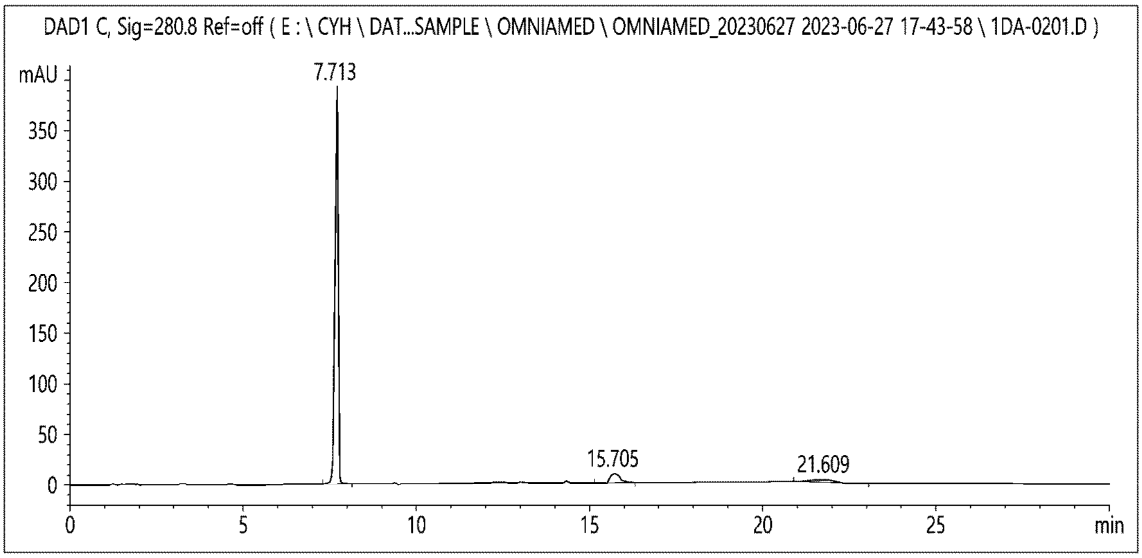
DAD1 C, Sig=280.8 Ref=off ( E : \ CYH \ DAT...SAMPLE \ OMNIAMED \ OMNIAMED_20230627 2023-06-27 17-43-58 \ 1DA-0201.D )
mAU
7.713
15.705
21.609
min

[FIG. 3]

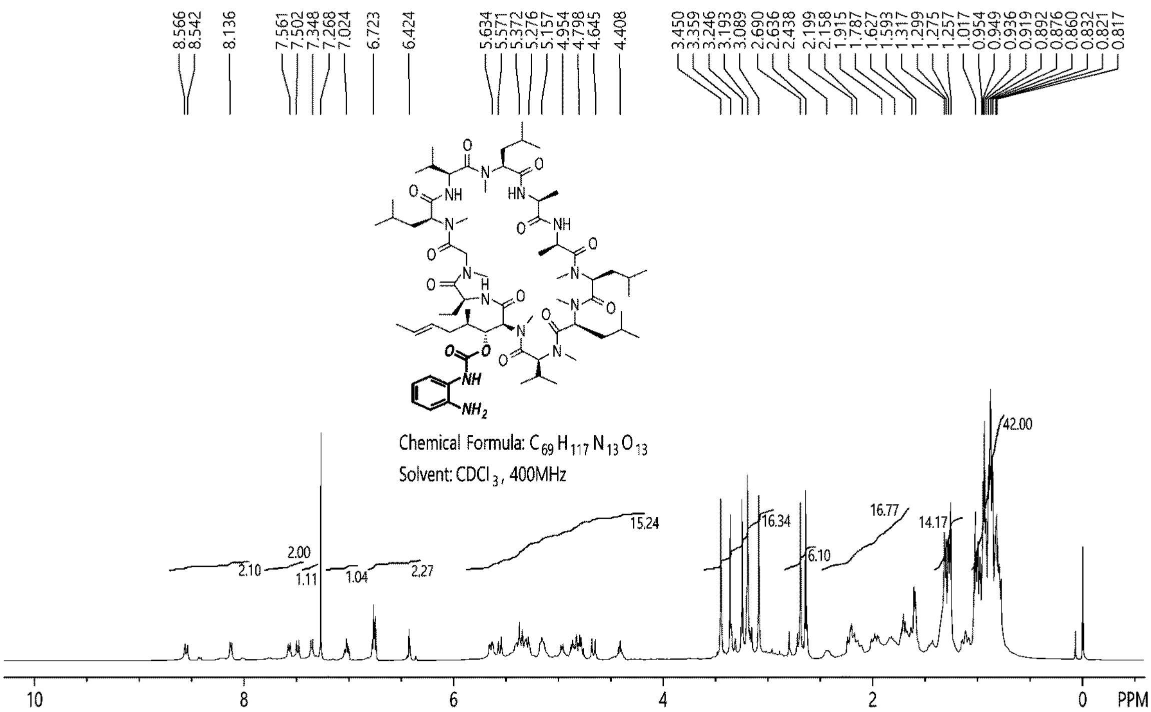
8.566 8.542 8.136 7.561 7.502 7.348 7.268 7.024 6.723 6.424 5.634 5.571 5.372 5.276 5.157 4.954 4.798 4.645 4.408 3.450 3.359 3.246 3.193 3.089 2.690 2.636 2.438 2.199 2.158 1.915 1.787 1.627 1.593 1.317 1.299 1.275 1.257 1.017 0.954 0.949 0.936 0.919 0.892 0.876 0.860 0.832 0.821 0.817
Chemical Formula: C69 H117 N13 O13
Solvent: CDCl3, 400MHz
2.10 2.00 1.11 1.04 2.27 15.24 16.34 6.10 16.77 14.17 42.00
PPM

[FIG. 4]

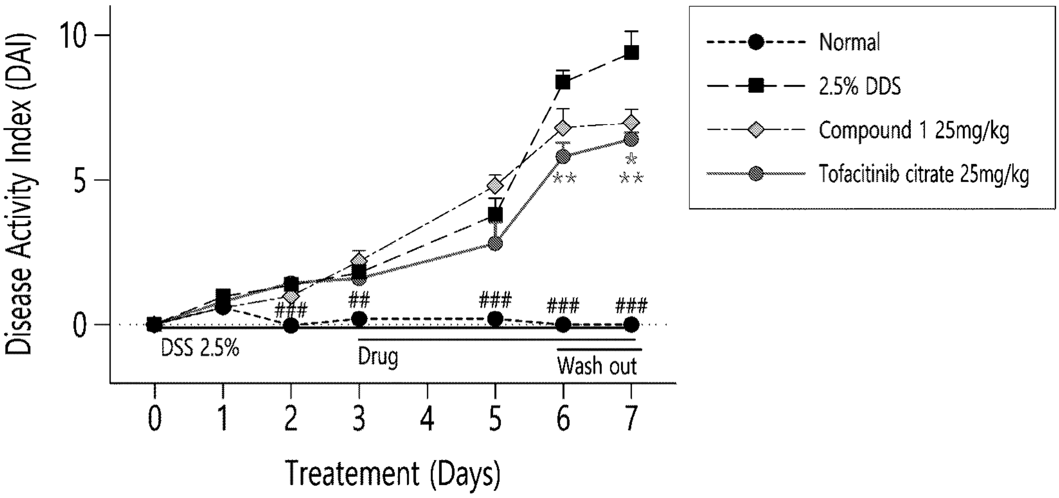


Data were presented as the mean ± SEM.

* p<0.05, ** p<0.01, *** p<0.001, compared with DSS group, were measured by student t-test.

# p<0.05, ## p<0.01, ### p<0.001, compared normal with DSS group, were measured by student t-test.

[FIG. 5]

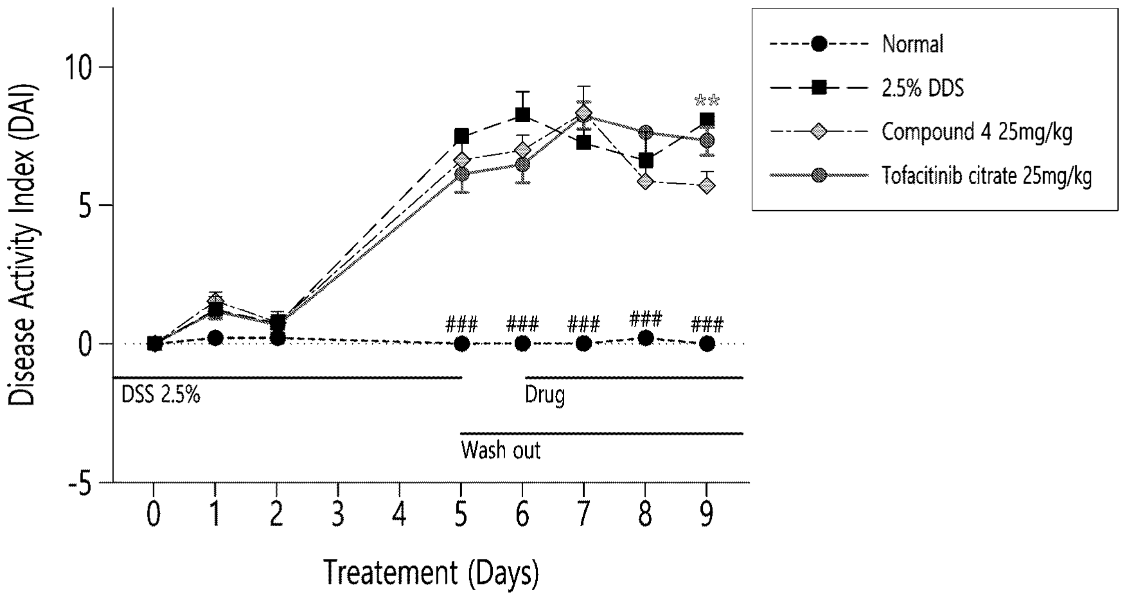


Data were presented as the mean ± SEM.

* p<0.05, ** p<0.01, *** p<0.001, compared with DSS group, were measured by student t-test.

# p<0.05, ## p<0.01, ### p<0.001, compared normal with DSS group, were measured by student t-test.

[FIG. 6]

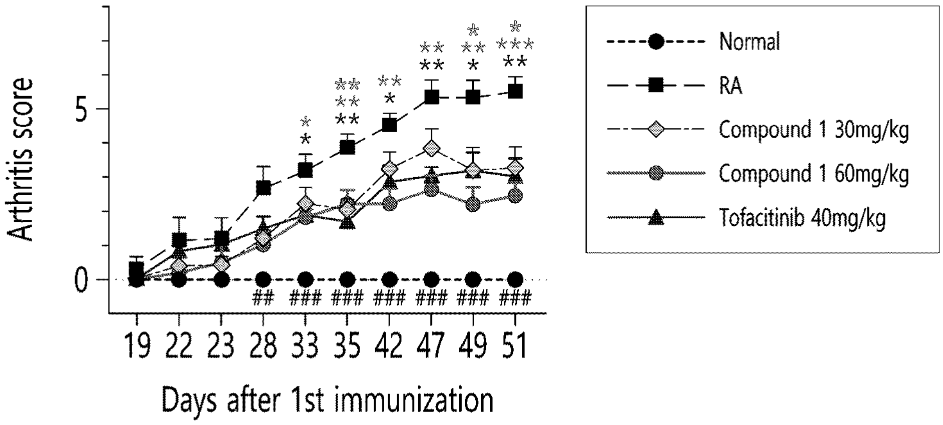


Data were presented as the mean ± SEM.
* p<0.05, ** p<0.01, *** p<0.001, compared with RA group, were measured by student t-test.
# p<0.05, ## p<0.01, ### p<0.001, compared normal with RA group, were measured by student t-test.

[FIG. 7]

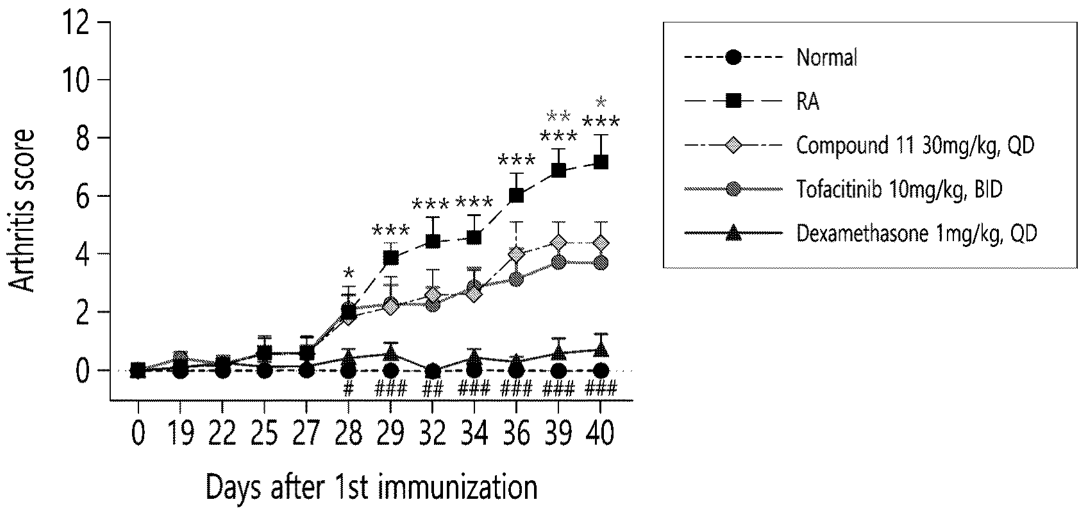


Data were presented as the mean ± SEM.
* p<0.05, ** p<0.01, *** p<0.001, compared with RA group, were measured by student t-test.
# p<0.05, ## p<0.01, ### p<0.001, compared normal with RA group, were measured by student t-test.

**INTERNATIONAL SEARCH REPORT**

International application No.
**PCT/KR2024/009549**

**A. CLASSIFICATION OF SUBJECT MATTER**

**C07D 487/04**(2006.01)i; **A61K 31/519**(2006.01)i; **A61K 47/55**(2017.01)i; **A61P 29/00**(2006.01)i; **A61P 25/00**(2006.01)i; **A61P 9/00**(2006.01)i; **A61P 35/00**(2006.01)i; **A61P 3/04**(2006.01)i; **A61P 39/06**(2006.01)i

According to International Patent Classification (IPC) or to both national classification and IPC

**B. FIELDS SEARCHED**

Minimum documentation searched (classification system followed by classification symbols)

C07D 487/04(2006.01); A61K 31/473(2006.01); A61K 31/497(2006.01); A61K 31/69(2006.01); C07D 221/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 저분자 화합물 (low molecular compound), 일산화질소 (nitric oxide), 키나아제 억제제 (kinase inhibitor), 항암제 (anti-cancer agent), 항염증제 (anti-inflammatory agent)

**C. DOCUMENTS CONSIDERED TO BE RELEVANT**

| Category* | Citation of document, with indication, where appropriate, of the relevant passages | Relevant to claim No. |
|---|---|---|
| | ZHANG, J. et al. Dual-acting antitumor agents targeting the A2A adenosine receptor and histone deacetylases: Design and synthesis of 4-(furan-2-yl)-1H-pyrazolo[3,4-d]pyrimidin-6-amine derivatives. European Journal of Medicinal Chemistry. 2022, vol. 236, article no. 114326, pp. 1-24. | |
| X | See abstract; pages 2 and 11; table 2; figure 1; and formula 2. | 1,2,6 |
| Y | | 8-10 |
| A | | 3-5,7 |
| | 메디컬투데이. MIT 연구팀...`일산화질소` 대장암 유발. 21 January 2009, non-official translation (MEDICAL TODAY. According to MIT Research Team...`Nitrogen Monoxide` Causes Colon Cancer). [Retrieved from the Internet on 25 September 2024]. Retrieved from the Internet <URL:https://mdtoday.co.kr/news/view/179571840922685?dt=m>. | |
| Y | See the entire main text. | 8-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 October 2024** | **14 October 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/009549**

**C. DOCUMENTS CONSIDERED TO BE RELEVANT**

| Category* | Citation of document, with indication, where appropriate, of the relevant passages | Relevant to claim No. |
|---|---|---|
| X<br>Y | WANG, Z. et al. Discovery of novel polysubstituted N-alkyl acridone analogues as histone deacetylase isoform-selective inhibitors for cancer therapy. Journal of Enzyme Inhibition and Medicinal Chemistry. [electronic publication] 05 May 2023, vol. 38, no. 1, article no. 2206581, pp. 1-15.<br>See abstract; figure 2; and scheme 2. | 1,2,6<br>8-10 |
| X<br>Y | CN 106279021 A (SECOND MILITARY MEDICAL UNIVERSITY, PLA) 04 January 2017 (2017-01-04)<br>See claims 1, 8 and 9. | 1,2,6<br>8-10 |
| A | KR 10-2019-0016511 A (KARUS THERAPEUTICS LTD.) 18 February 2019 (2019-02-18)<br>See claims 1 and 8. | 1-10 |

Form PCT/ISA/210 (second sheet) (July 2022)

**INTERNATIONAL SEARCH REPORT**
**Information on patent family members**

International application No.
**PCT/KR2024/009549**

| Patent document cited in search report | Publication date (day/month/year) | Patent family member(s) | Publication date (day/month/year) |
|---|---|---|---|
| CN 106279021 A | 04 January 2017 | None | |
| KR 10-2019-0016511 A | 18 February 2019 | AU 2017273189 A1 | 20 December 2018 |
| | | BR 112018074632 A2 | 06 March 2019 |
| | | CA 3025722 A1 | 07 December 2017 |
| | | CN 109310679 A | 05 February 2019 |
| | | EP 3463348 A1 | 10 April 2019 |
| | | IL 263360 A | 31 December 2018 |
| | | JP 2019-517509 A | 24 June 2019 |
| | | MX 2018014934 A | 24 April 2019 |
| | | SG 11201810516 UA | 28 December 2018 |
| | | US 2020-0316062 A1 | 08 October 2020 |
| | | WO 2017-208032 A1 | 07 December 2017 |
| | | ZA 201807855 B | 31 July 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230086949